# EUROPEAN PATENT APPLICATION

(11) **EP 4 311 829 A1**
(43) Date of publication of application: **31.01.2024**
(21) Application number: 22187495.1
(22) Date of filing: 28.07.2022
(51) Int. Cl.: C07D 403/06, C07D 403/14, C07D 417/06, C07D 417/14, A61K 31/498, A61K 31/517

(54) **SUBSTITUTED BICYCLIC HETEROARYL SULFONAMIDE DERIVATIVES FOR THE TREATMENT OF CANCER**

(71) Applicant: Nodus Oncology Limited, Macclesfield SK10 4TG (GB)
(72) Inventor: WEILER, Sven, 4123 Allschwil (CH); GAUCHER, Bérangère, 4123 Allschwil (CH); RADIMERSKI, Thomas, 4123 Allschwil (CH); KELLENBERGER, Laurenz, 4123 Allschwil (CH)
(74) Representative: Haseltine Lake Kempner LLP

(57) **Abstract**

The invention provides compounds of formula (I) 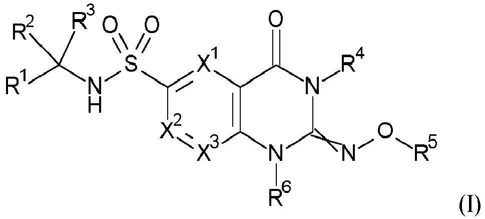 and pharmaceutically acceptable salts thereof; wherein X¹ is CR⁷ or N, X² is CR⁸ or N, X³ is CR⁹ or N and wherein R¹, R², R³, R⁴, R⁵, R⁶, R⁷, R⁸ and R⁹ are as defined in the claims, as well as methods of using the compounds for the treatment of neoplastic diseases such as cancer.

## Description

The present invention relates to compounds which inhibit poly ADP-ribose glycohydrolase (PARG) and their use in the treatment of neoplastic diseases such as cancer.

Genomic instability and replicative immortality are key hallmarks of cancer (Hanahan D, Cancer Discovery 2022). However, sustained, uncontrolled proliferation driven by oncogenes and loss of tumor suppressor genes also puts a strain on cancer cell anabolism. For instance, the constitutive activation of oncogenic pathways forces cells through the cell cycle, which can lead to DNA replication stress (Gaillard H et al, Nature Reviews Cancer 2015). In order to cope with the latter and avoid detrimental DNA damage, cells trigger a variety of complementary DNA damage response and repair (DDR) pathways to resolve stalled replication forks and to repair single and/or double strand DNA breaks (Brown JS et al, Cancer Discovery 2017).

Amongst the tumor suppressor gene functions lost in cancer, numerous components of the different DDR pathways can be compromised, including in hereditary cancer syndromes. On one hand, defects in certain DDR mechanisms can be compensated and buffered to some extent by alternative repair pathways (Curtin NJ, Nature Reviews Cancer 2012). On the other hand, these alternative pathways will often not repair DNA lesions with the same fidelity as the compromised DDR pathway. Thus, gaps in repair mechanisms can create vulnerabilities that can be exploited for cancer therapy. This concept is often also referred to as synthetic lethality, where a dysfunction in one biological process is still compatible with cell viability, whereas genetic loss, or pharmacological inhibition, of a parallel and/or compensatory process will be cell lethal (Hartwell and Friend, Science 1997).

The discovery and subsequent clinical translation of synthetic lethality in cancer settings with defective homologous recombination repair (HR) upon inhibition of poly(ADP-ribose polymerases 1 and 2 (PARP1/2) has been a scientific and therapeutic breakthrough (Farmer H et al, Nature 2005; Bryant HE et al, Nature 2005). HR is the most accurate DNA repair pathway, but loss of *BRCA1* (BReast CAncer gene 1) or *BRCA2* (BReast CAncer gene 2) tumor suppressor genes in the pathway blunts its function, and affected cells instead rely on alternative, less-faithful DDR mechanisms for the repair of damaged DNA. PARP1 and 2 are DNA damage sensors involved in the alternative non-homologous end-joining (alt-NHEJ) and DNA single-strand break (SSB) repair/base excision repair (BER) pathways. Upon pharmacological inhibition of PARP1/2 enzymes in cancers with loss of either *BRCA1* or *BRCA2,* or exhibiting so-called "BRCAness" (Turner N et al, Nature Reviews Cancer 2004), stalled DNA replication forks collapse and DNA lesions persist, leading to cancer cell death.

PARP1/2 enzymes belong to a family of 17 members (Hottiger MO et al, Trends Biochem. Sci. 2010), and upon sensing of DNA damage they rapidly post-translationally modify themselves and other target proteins with ADP ribose (ADPr) using the co-enzyme nicotinamide adenine dinucleotide (NAD+) as a substrate. On the acceptor proteins, PARP1/2 typically form polymers of ADPr, referred to as poly ADP-ribose (PAR) or PARylation, having varying lengths and extent of branching, and these are thought to facilitate the recruitment of additional DNA repair enzymes at the site of DNA damage (Leung AKL, Trends in Cell Biology 2020). However, to allow for completion of DNA damage repair, PAR chains need to be removed again from acceptor proteins. Poly(ADP-ribose) glycohydrolase (PARG) counterbalances PARP1/2 by degrading PAR chains. PARG primarily exhibits exo-glycohydrolase activity, degrading PAR chains to monomeric ADPr (Barkauskaite E at al, Nature Communications 2013), however, endo-glycohydrolase activity leading to release of protein-free PAR chains has also been described (Pourfarjam Y et al, BBRC 2020).

Preclinical evidence suggests that increased removal of PAR chains facilitates cancer growth. Genetically enforced overexpression of PARG was shown to promote transformation and tumor growth of normal human mammary epithelial cells in mice (Marques M et al, Oncogene 2019). Conversely, shRNA-mediated depletion of PARG led to decreased tumor initiation, outgrowth and metastasis (Marques M et al, Oncogene 2019). Pharmacological inhibition of PARG has been achieved using cell permeable tool compounds. Importantly, these studies in cell-based models of cancer have shown the potential for PARG inhibitors to act complementary to PARP inhibitors (Pillay N et al, Cancer Cell 2019), as well as in settings of acquired PARP inhibitor resistance (Coulson-Gilmer C et al, Journal of Experimental & Clinical Cancer Research 2021). Sensitivity to PARG inhibition was reported to correlate with DNA replication vulnerabilities and markers of DNA replication stress, and PARG inhibition resulted in a profound loss of mitotic potential in sensitive cancer cell lines (Pillay N et al, Cancer Cell 2019; Coulson-Gilmer C et al, Journal of Experimental & Clinical Cancer Research 2021). Furthermore, persistent PARylation is highly toxic to cells (Prokhorova E et al, Molecular Cell, 2021), and PARG inhibition prolongs PARylation at DNA lesions, which impedes DNA repair factors and suppresses DNA repair (Chen S-H & Yu X, Science Advances 2019). Consequently, PARG inhibition sensitizes to a range of DNA-damaging agents (Slade D, Genes & Development 2020), to drugs targeting other DDR enzymes such as e.g. Checkpoint kinase 1 (CHK1) (Pillay N et al, Cancer Cell 2019), and to ionizing radiation-induced DNA-damage (Houl JH et al, Nature Communications 2019). In conclusion, PARG inhibition holds promise both as monotherapy, and in combination with other therapies for the treatment of cancers.

WO 2021/055744, WO 2016/097749 and WO 2016/092326 describe PARG inhibitors.

In a first aspect the invention provides compounds of formula (I) 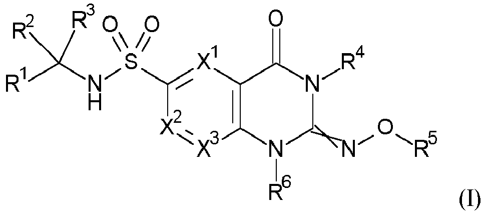 and pharmaceutically acceptable salts thereof,
wherein
R¹ is hydrogen, cyano, formyl, -CONH₂, -CH₂OH, -CH₂OC₁₋₂ alkyl, C₁₋₂ alkyl, C₁₋₂ haloalkyl or ethynyl;
R² and R³ are independently C₁₋₂ alkyl;
or R² and R³ together with the carbon atom to which they are attached form cyclopropyl or oxetanyl;
X¹ is CR⁷ or N;
R⁷ is hydrogen or fluoro;
X² is CR⁸ or N;
R⁸ is hydrogen or fluoro;
X³ is CR⁹ or N;
R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, heteroaryl, heterocyclyl, fused heterocyclyl, spiro heterocyclyl or bridged heterocyclyl, wherein the cycloalkyl, phenyl, heteroaryl, heterocyclyl, fused heterocyclyl, spiro heterocyclyl and bridged heterocyclyl are optionally substituted with R^{a}, R^{b} and/or R^{c};
R^{a} is hydrogen, -N(R^{m})Rⁿ, C₁₋₆ alkyl, hydroxy, C₁₋₆ alkoxy, halogen, cyano, oxo, C₁₋₆ haloalkyl, C₁₋₆ haloalkoxy, hydroxyC₁₋₆ alkyl, heteroaryl, heterocyclyl, -C(O)R^{d}, -C(O)OR^{e}, -C(O)N(R^{f})R^{g}, - S(O)₂N(R^{h})Rⁱ or C₁₋₆ alkyl-N(R^{j})R^{k};
R^{b} and R^{c} are independently hydrogen, -N(R^{m})Rⁿ, C₁₋₆ alkyl, C₁₋₄ alkyl-N(R^{m})Rⁿ, hydroxy, C₁₋₆ alkoxy, halogen, cyano, C₁₋₆ haloalkyl or C₁₋₆ haloalkoxy;
R^{d} is hydrogen, C₁₋₆ alkyl, C₁₋₆ haloalkyl, C₃₋₆ cycloalkyl, phenyl, heteroaryl or heterocyclyl;
R^{e} is hydrogen or C₁₋₆ alkyl;
R^{f}, R^{g}, R^{h}, Rⁱ, R^{j}, R^{k}, R^{m} and Rⁿ are each independently hydrogen, C₁₋₆ alkyl, C₁₋₆ haloalkyl, aminoC₁₋₆ alkyl or hydroxyC₁₋₆ alkyl; or
R^{f} and R^{g}, R^{h} and Rⁱ, or R^{j} and R^{k} together with the nitrogen atom to which they are attached form heterocyclyl;
wherein
   (i) the heteroaryl and heterocyclyl of R^{a};
   (ii) the cycloalkyl, phenyl, heteroaryl and heterocyclyl of R^{d};
   (iii) the heterocyclyl formed by R^{f} and R^{g}, R^{h} and Rⁱ, or R^{j} and R^{k} combining with the nitrogen to which they are attached;
are each ((i), (ii), (iii)) optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy;
R⁴ is hydrogen or the group -L^{1A}-L^{2A}-L^{3A};
L^{1A} is absent or is C₁₋₃ alkylene optionally substituted by C₁₋₂ alkyl or oxo;
L^{2A} is absent or is -O-, -S-, -S(O)-, -S(O)₂-, -N(R^{a1})-, -C(O)-, -C(O)O-, -OC(O)-, -C(O)N(R^{a1})-, - N(R^{a1})C(O)-, -N(R^{a1})C(O)N(R^{b1})-, -S(O)₂N(R^{a1})-, or -N(R^{a1})S(O)₂-;
R^{a1} and R^{b1} are each independently hydrogen or C₁₋₂ alkyl;
L^{3A} is hydrogen, C₁₋₆ alkyl, C₃₋₆ cycloalkyl, phenyl, heterocyclyl or heteroaryl, wherein L^{3A} is optionally substituted by one or more substituents independently selected from halogen, cyano, hydroxy, carboxy, carbamoyl, sulphamoyl, C₁₋₄ alkyl, C₁₋₄ haloalkyl, C₁₋₄ alkoxy, -N(R^{c1})R^{d1}, -OR^{c1}, -C(O)R^{c1}, - C(O)OR^{c1}, -OC(O)R^{c1}, -C(O)N(R^{d1})R^{c1}, -N(R^{d1})C(O)R^{c1}, -S(O)yR^{c1} -S(O)₂N(R^{d1})R^{c1}, -N(R^{d1})S(O)₂R^{c1} and -(CH₂)_{z}N(R^{d1})R^{c1},
R^{c1} and R^{d1} are each independently hydrogen or C₁₋₄ alkyl;
y is 0, 1 or 2;
z is 1, 2 or 3;
R⁵ is hydrogen or the group -L^{1B}-L^{2B}-L^{3B};
L^{1B} is absent or is C₁₋₃ alkylene optionally substituted by C₁₋₂ alkyl or oxo;
L^{2B} is absent or is -O-, -S-, -S(O)-, -S(O)₂-, -N(R^{a2})-, -C(O)-, -C(O)O-, -OC(O)-, -C(O)N(R^{a2})-, - N(R^{a2})C(O)-, -N(R^{a2})C(O)N(R^{b2})-, -S(O)₂N(R^{a2})-, or -N(R^{a2})S(O)₂-;
R^{a2} and R^{b2} are each independently hydrogen or C₁₋₂ alkyl;
L^{3B} is hydrogen, C₁₋₆ alkyl, C₃₋₆ cycloalkyl, phenyl, heterocyclyl or heteroaryl, wherein L^{3B} is optionally substituted by one or more substituents independently selected from halogen, cyano, hydroxy, carboxy, carbamoyl, sulphamoyl, C₁₋₄ alkyl, C₁₋₄ haloalkyl, C₁₋₄ alkoxy, -N(R^{c2})R^{d2}, -OR^{c2}, -C(O)R^{c2}, - C(O)OR^{c2}, -OC(O)R^{c2}, -C(O)N(R^{d2})R^{c2}, -N(R^{d2})C(O)R^{c2}, -S(O)_{y'}R^{c2}, - S(O)₂N(R^{d2})R^{c3}, -N(R^{d2})S(O)₂R^{c2} and -(CH₂)_{z'}N(R^{d2})R^{c2};
R^{c2} and R^{d2} are each independently hydrogen or C₁₋₄ alkyl;
y' is 0, 1 or 2;
z' is 1, 2 or 3;
R⁶ is hydrogen or the group -L^{1C}-L^{2C}-L^{3C};
L^{1C} is absent or is C₁₋₃ alkylene optionally substituted by C₁₋₂ alkyl or oxo;
L^{2C} is absent or is -O-, -S-, -S(O)-, -S(O)₂-, -N(R^{a3})-, -C(O)-, -C(O)O-, -OC(O)-, -C(O)N(R^{a3})-, - N(R^{a3})C(O)-, -N(R^{a3})C(O)N(R^{b3})-, -S(O)₂N(R^{a3})-, or -N(R^{a3})S(O)₂-;
R^{a3} and R^{b3} are each independently hydrogen or C₁₋₂ alkyl;
L^{3C} is hydrogen, C₁₋₆ alkyl, C₃₋₆ cycloalkyl, phenyl, heterocyclyl or heteroaryl, wherein L^{3C} is optionally substituted by one or more substituents independently selected from halogen, cyano, hydroxy, carboxy, carbamoyl, sulphamoyl, C₁₋₄ alkyl, C₁₋₄ haloalkyl, C₁₋₄ alkoxy, -N(R^{c3})R^{d3}, -OR^{c3}, -C(O)R^{c3}, - C(O)OR^{c3}, -OC(O)R^{c2}, -C(O)N(R^{d3})R^{c3}, -N(R^{d3})C(O)R^{c3}, -S(O)_{y"}R^{c3}, -S(O)₂(R^{d3})R^{c3}, -N(R^{d3})S(O)₂R^{c3} and -(CH₂)_{z"}N(R^{d3})R^{c3};
R^{c3} and R^{d3} are each independently hydrogen or C₁₋₄ alkyl;
y" is 0, 1 or 2;
z" is 1, 2 or 3;
with the proviso that:
the groups -L^{1A}-L^{2A}-L^{3A}, -L^{1B}-L^{2B}-L^{3B}, and -L^{1C}-L^{2C}-L^{3C} do not contain an -O-O-, -S-O-, -O-S- or -S-S- unit as a linking unit within the group;
the group -L^{1A}-L^{2A}-L^{3A} does not place an -O-N- or -S-N- unit adjacent to the ring nitrogen atom connected to R⁴;
the group -L^{1B}-L^{2B}-L^{3B} does not place an N, O or S atom adjacent to the oxime oxygen atom connected to R⁵, and
the group -L^{1C}-L^{2C}-L^{3C} does not place an -O-N- or -S-N- unit adjacent to the ring nitrogen atom connected to R⁶.

In a further aspect, the invention provides compounds of formula (I) and pharmaceutically acceptable salts thereof for use in the treatment of neoplastic diseases, e.g. cancer, in a subject selected from a mammal, in particular a human.

In a further aspect, the invention provides use of compounds of formula (I) and pharmaceutically acceptable salts thereof in the manufacture of a medicament for the treatment of neoplastic diseases, e.g. cancer, in a subject selected from a mammal, in particular a human.

In a further aspect, the invention provides methods of treating neoplastic diseases, e.g. cancer, in a subject selected from a mammal, in particular a human, comprising administering a compound of formula (I) or a pharmaceutically acceptable salt thereof, e.g. in a therapeutically effective amount, to said subject.

In a further aspect, the invention provides pharmaceutical compositions comprising a compound of formula (I) or a pharmaceutically acceptable salt thereof and optionally one or more pharmaceutically acceptable excipients.

Whenever compounds of formula (I) contain one or two or more centers of chirality (for example when R¹, R² and R³ are different moieties) such compounds may be provided as pure enantiomers or pure diastereoisomers as well as mixtures thereof in any ratio and all such isomers are included within the scope of the compounds of formula (I). Both geometric isomers arising from the orientation of the bond between the oxime nitrogen atom and oxygen atom, namely compounds (Ia) and (Ib) as depicted below, and mixtures thereof in any ratio are included in the scope of the invention.

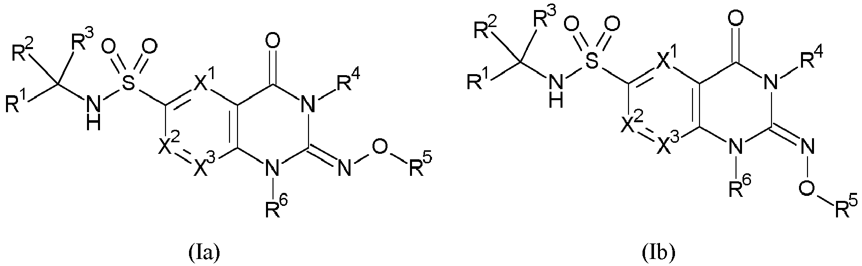

The compounds of the invention also include all tautomeric forms of the compounds of formula (I) and intermediates thereof, e.g. arising from tautomers of the -N(R⁴)-C(=NOR⁵)-N(R⁶)- moiety when R⁴ is a hydrogen atom and/or R⁶ is a hydrogen atom as shown below.

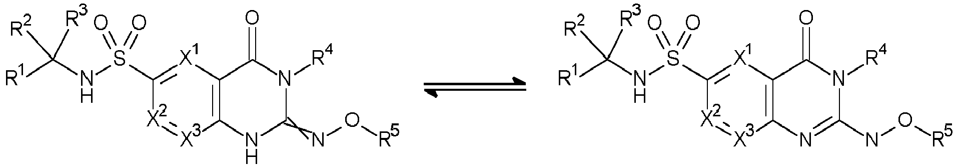

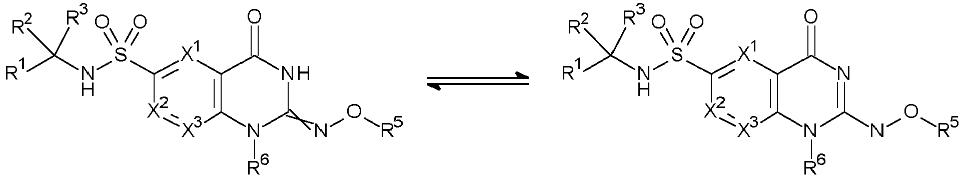

Isotopically labeled compounds including deuterium substitutions as well as carbon-13 and/or carbon-14 labels are also included within the scope of compounds of formula (I). The compounds of formula (I) may also be solvated, especially hydrated, and such solvated and hydrated forms of the compound of formula (I) are also included in the scope of the compounds of formula (I). Solvation and hydration may take place during the preparation process.

Reference to compounds of the invention includes pharmaceutically acceptable salts of said compounds. Such salts may also exist as hydrates and solvates. Examples of pharmacologically acceptable salts of the compounds of formula (I) are salts of physiologically acceptable mineral acids, such as hydrochloric acid, sulfuric acid and phosphoric acid, or salts of organic acids, such as methane-sulfonic acid, *p-*toluenesulfonic acid, lactic acid, acetic acid, trifluoroacetic acid, citric acid, succinic acid, fumaric acid, maleic acid and salicylic acid. Further examples of pharmacologically acceptable salts of the compounds of formula (I) are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts.

"Alkylene" alone or part of another substituent means a bivalent saturated straight or branched chain hydrocarbon having the number of carbon atoms designated (i.e. C₁₋₆ means 1 to 6 carbons). Examples of alkylene groups include -CH₂-, -CH₂-CH₂-, -CH(CH₃)-, -CH₂-CH₂-CH₂-, -CH(CH₃)-CH₂-, - CH(CH₂CH₃)- and -CH₂CH(CH₃)CH₂-.

"Alkoxy" and "haloalkoxy" mean alkyl and haloalkyl groups respectively that are attached to the remainder of the molecule via an oxygen atom.

"Alkyl" alone or as part of another substituent means a saturated straight or branched chain hydrocarbon radical, having the number of carbon atoms designated (i.e. C₁₋₆ means 1 to 6 carbons). Examples of alkyl groups include methyl, ethyl, *n*-propyl, isopropyl, *n*-butyl, *t*-butyl, isobutyl, *sec*-butyl, *n*-pentyl, *n*-hexyl, *n*-heptyl and *n*-octyl.

"Aminoalkyl," means alkyl that is substituted with one N(R)R' where R and R' are independently hydrogen, C₁₋₆ alkyl, hydroxyC₁₋₆ alkyl, C₁₋₆ alkoxyC₁₋₆ alkyl or -C(O)C₁₋₆ alkyl. For example "aminoC₁₋₆ alkyl" includes NH₂methyl, methylaminomethyl, methylaminoethyl, dimethylaminomethyl, diethylaminoethyl, dimethylaminoethyl, acetylaminomethyl and acetylaminoethyl. In some embodiments the N(R)R' is attached to the carbon atom of alkyl which is distal to the point of attachment to the rest of the molecule.

"Bridged heterocyclyl" means, unless otherwise stated, a saturated 5- to 7-membered monocyclic heterocycle having 2 non-adjacent ring atoms linked by a (X1)ₙ group where n is 1, 2 or 3, each X1 is CRR', NR, S, SO, SO₂ or O wherein no more than one X1 is NR, SO, SO₂ or O, and R and R' are independently H or methyl. The 5- to 7- membered heterocycle has 1, 2 or 3 heteroatoms as ring atoms independently selected from N, O, S, wherein the sulfur and nitrogen atoms are optionally oxidized, with the remaining ring atoms are carbon atoms. O and S are not bridgehead atoms and such rings do not contain adjacent oxygen atoms, adjacent sulfur atoms, or adjacent oxygen and sulfur atoms within the ring. Examples include 2-azabicyclo[2.2.2]octane, quinuclidine, 7-oxabicyclo[2.2.1]heptane and 3,8-diazabicyclo [3.2.1]octane.

"Cycloalkyl" means a saturated hydrocarbon ring having the indicated number of ring atoms (e.g. C₃₋₆ cycloalkyl). Examples include cyclopropyl, cyclobutyl, cyclopentyl and cyclohexyl.

"Fused heterocyclyl" means, unless otherwise stated, a saturated or partially saturated monocyclic ring of 4 to 7 ring atoms having 1, 2 or 3 heteroatoms independently selected from N, S and O, wherein the sulfur and nitrogen atoms are optionally oxidized, and the remaining ring atoms are carbon atoms, wherein the heterocyclyl ring is fused to two adjacent ring members of a phenyl, a 5- or 6-membered heteroaryl, a C₃₋₆ cycloalkyl or a heterocyclyl, each as defined herein. The fused heterocyclyl can be attached to the remainder of the molecule through any ring atom. The number of ring atoms in the saturated or partially saturated monocyclic ring includes the two common ring atoms shared with the fused group. Such rings do not contain adjacent oxygen atoms, adjacent sulfur atoms, or adjacent oxygen and sulfur atoms within the ring. Examples include 2,3-dihydrobenzo[b][1,4]-dioxinyl and 2-oxabicyclo[3.1.0]hexanyl.

"Halogen" by itself or as part of another substituent means a fluorine, chlorine, bromine or iodine atom.

"Haloalkyl" means alkyl that is substituted with 1 to 5 halogen atoms and includes monohaloalkyl and polyhaloalkyl. For example "C₁₋₄ haloalkyl" includes trifluoromethyl, 2,2,2-trifluoroethyl, 4-chlorobutyl and 3-bromopropyl. Likewise, fluoromethyl includes -CH₂F, -CHF₂ and -CF₃.

"Heteroaryl" means, unless otherwise stated, a 5- to 10-membered aromatic ring that contains 1 to 5 heteroatoms as ring atoms independently selected from N, S and O, wherein the nitrogen and sulfur atoms are optionally oxidized. A heteroaryl group can be attached to the remainder of the molecule through a heteroatom or a carbon atom. Such rings do not contain adjacent oxygen atoms, adjacent sulfur atoms, or adjacent oxygen and sulfur atoms within the ring. Examples of heteroaryl groups include pyridyl, pyridazinyl, pyrazinyl, pyrimidinyl, triazinyl, quinolinyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridines, benzothiaxolyl, benzofuranyl, benzothienyl, indolyl, quinolyl, isoquinolyl, isothiazolyl, pyrazolyl, indazolyl, pteridinyl, imidazolyl, triazolyl, tetrazolyl, oxazolyl, isoxazolyl, thiadiazolyl, pyrrolyl, thiazolyl, furyl and thienyl.

"Heterocyclyl" means, unless otherwise stated, a saturated or partially unsaturated 4- to 10-membered monocyclic or bicyclic ring having 1 to 4 heteroatoms as ring atoms independently selected from N, S and O, wherein the sulfur and nitrogen atoms are optionally oxidized, and the remaining ring atom are carbon atoms. Such rings do not contain adjacent oxygen atoms, adjacent sulfur atoms, or adjacent oxygen and sulfur atoms within the ring. Examples include pyrrolidinyl imidazolidinyl, pyrazolidinyl, butyrolactamyl, valerolactamyl, imidazolidinonyl, hydantoinyl, dioxolanyl, piperidinyl, 1,4-dioxanyl, morpholinyl, thiomorpholinyl, thiomorpholinyl-S-oxide, 1,1-dioxothiomorpholinyl, piperazinyl, pyranyl, pyridonyl, 3-pyrrolinyl, thiopyranyl, pyronyl, tetrahydrofuranyl and tetrahydrothiophenyl. A heterocycloalkyl group can be attached to the remainder of the molecule through a ring carbon atom or a heteroatom.

"Hydroxyalkyl" means alkyl, as defined above, that is substituted with 1 or 2 hydroxy moieties. For example "hydroxyC₁₋₄ alkyl" includes hydroxymethyl, 1- or 2-hydroxyethyl, 1,2-dihydroxyethyl and hydroxypropyl. In some embodiments one hydroxy moiety is attached to the carbon atom of alkyl which is distal to the point of attachment to the rest of the molecule.

Oxo is an =O group. Where a moiety is said to be "substituted by oxo" it is counted as substitution by one substituent.

"Spiro heterocyclyl" means a saturated or partially unsaturated bicyclic ring of 5 to 12 ring atoms wherein 1, 2 or 3 ring atoms are heteroatoms independently selected from N, S and O, wherein the sulfur and nitrogen atoms are optionally oxidized and the remaining ring atoms are carbon atoms, wherein the two rings are linked together by one common atom. Such rings do not contain adjacent oxygen atoms, adjacent sulfur atoms, or adjacent oxygen and sulfur atoms within the ring. Examples include 6-azaspiro[3.4]octanyl 2-oxa-6-azaspiro[3.4]octan-6-yl, 4-oxaspiro[2.4]heptanyl, spiro[3.5]non-6-enyl and 2,7-diazaspiro[4.4]nonanyl.

Where a substituent is said to be "optionally substituted" the substituent may be unsubstituted or may be substituted with the indicated substituents, e.g. by 1, 2 or 3, of the indicated substituents.

The following examples of substituent definitions and embodiments may be combined in any combination where possible.

R¹ is hydrogen, cyano, formyl, -CONH₂, -CH₂OH, -CH₂OC₁₋₂ alkyl, C₁₋₂ alkyl, C₁₋₂ haloalkyl or ethynyl. Preferably R¹ is cyano, C₁₋₂ alkyl or ethynyl.

In some embodiments R¹ is cyano or C₁₋₂ alkyl.

Specific examples of R¹ include cyano and -CH₃.

R² and R³ are independently C₁₋₂ alkyl, or R² and R³ together with the carbon atom to which they are attached form cyclopropyl or oxetanyl.

Preferably R² and R³ together with the carbon atom to which they are attached form cyclopropyl or oxetanyl (e.g. oxetanyl, wherein the oxygen atom is distal to the quaternary carbon atom).

Specific examples include cyclopropyl and oxetanyl (e.g. oxetanyl, wherein the oxygen atom is distal to the quaternary carbon atom).

X¹ is CR⁷ or N and R⁷ is hydrogen or fluoro.

In some embodiments X¹ is CR⁷.

In some embodiments X¹ is CR⁷ and R⁷ is H

X² is CR⁸ or N and R⁸ is hydrogen or fluoro.

In some embodiments X² is CR⁸.

In some embodiments X² is CR⁸ and R⁸ is H.

X³ is CR⁹ or N.

In some embodiments X³ is CR⁹.

In some embodiments no more than two of X¹, X² and X³ is N;

In some embodiments X¹ is CR⁷; X² is CR⁸; and X³ is CR⁹.

In some embodiments X¹ is CR⁷; X² is CR⁸; X³ is CR⁹; and R⁷ and R⁸ are hydrogen.

R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, heteroaryl, heterocyclyl, fused heterocyclyl, spiro heterocyclyl or bridged heterocyclyl, wherein the cycloalkyl, phenyl, heteroaryl, heterocyclyl, fused heterocyclyl, spiro heterocyclyl and bridged heterocyclyl are optionally substituted with R^{a}, R^{b} and/or R^{c}.

Preferably R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, 5- to 6-membered heteroaryl containing 1, 2 or 3 heteroatoms as ring atoms independently selected from N, S and O, 5- to 7-membered monocyclic heterocyclyl containing 1, 2 or 3 heteroatoms as ring atoms independently selected from N, S and O, wherein the cycloalkyl, phenyl, heteroaryl and heterocyclyl are optionally substituted with R^{a}, R^{b} and/or R^{c}; or

R⁹ is spiro heterocyclyl, wherein the first ring connected to X³ is a 5- to 7-membered monocyclic heterocyclyl containing 1, 2 or 3 heteroatoms as ring atoms independently selected from N, S and O and the second ring is connected to the first ring with a common carbon atom and is a 3- to 6-membered monocyclic cycloalkyl or a 3- to 6-membered monocyclic heterocyclyl containing 1, 2 or 3 heteroatoms as ring atoms independently selected from N, S and O, wherein spiro heterocyclyl is optionally substituted by R^{a}, R^{b} and/or R^{c}.

For example R⁹ is hydrogen, halogen, cyano, phenyl, 5- to 6-membered heteroaryl containing 1 or 2 heteroatoms as ring atoms selected from N, or 6-membered monocyclic heterocyclyl containing 1 to 2 heteroatoms as ring atoms selected from N and O, wherein phenyl, heteroaryl and heterocyclyl are optionally substituted with R^{a}, R^{b} and/or R^{c}; or

R⁹ is a spiro ring system wherein the first ring connected to X³ is a 4 to 6-membered monocyclic heterocyclyl containing 1 to 2 heteroatoms as ring atoms selected from N and the second ring is connected to the first ring with a common carbon atom and the second ring is a 4- to 6-membered heterocyclyl containing 1 heteroatom as ring atom independently selected from N, O and S, in particular selected from O, wherein the first ring of the spiro heterocyclyl is optionally substituted by R^{b} and/or R^{c}.

For example R⁹ is hydrogen, halogen, cyano, phenyl, 5- to 6-membered heteroaryl containing 1 or 2 heteroatoms as ring atoms selected from N, wherein the phenyl and 6-membered heteroaryl are optionally substituted at the para position with respect to the connection to the rest of the molecule with R^{a} and are additionally optionally substituted with R^{b}, and the 5-membered heteroaryl is optionally substituted at the 3 position distal to the connection to the rest of the molecule with R^{a} and is additionally optionally substituted with R^{b}; or R⁹ is the moiety (R^{9a}) or the moiety (R^{9b}): 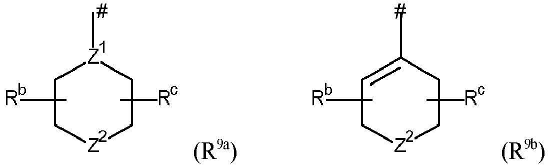 wherein Z¹ is N or CH, Z² is N(R^{a}), O, S, CH(R^{a}) or C(R^{x})(R^{y}), wherein R^{x} and R^{y} together form a 4- to 6-membered monocyclic heterocyclyl containing one heteroatom as ring atom selected from N, O and S; and wherein optionally at least one of Z¹ and Z² is N or N(R^{a}) respectively. As for defined for heterocyclyl moieties, the S and N ring atoms in R^{9a} and R^{9b} are optionally oxidized. For clarity in moieties (R^{9a}) and (R^{9b}) the substituent R^{b} occupies one of the two carbon atoms on the left hand side of the moiety as drawn and R^{c} occupies one of the two carbon atoms on the right hand side of the moiety as drawn.

Examples of the (R^{9a}) moiety depicting possible stereoisomers are as follows (R^{c} is hydrogen in the two structures at the bottom right):

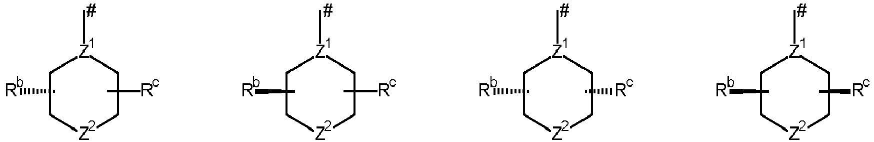

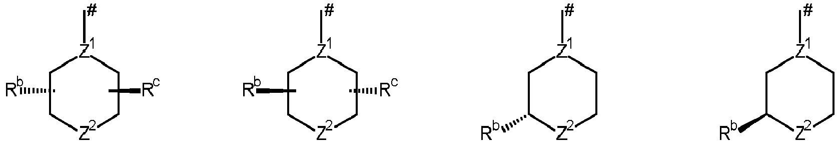

Additional stereoisomers are possible when Z¹ or Z² is CH or CH(R^{a}) respectively.

Examples of the (R^{9b}) moiety depicting possible stereoisomers are as follows (R^{c} is hydrogen in the two structures at the bottom right):

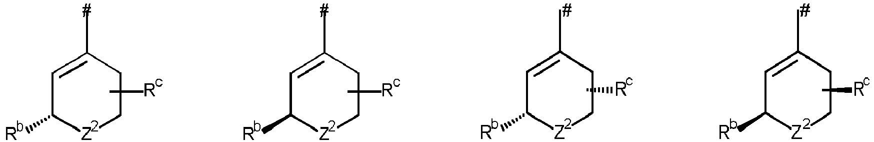

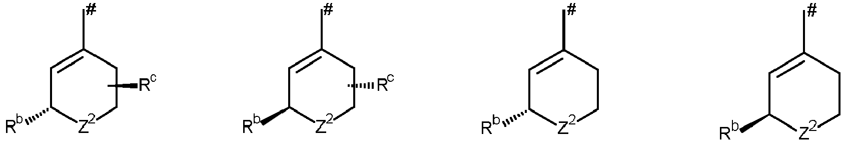

Additional stereoisomers are possible when Z² is CH or CH(R^{a}) respectively.

Specific examples of R⁹ which may be optionally substituted by R^{a}, R^{b} and/or R^{c} where possible include hydrogen, chloro, phenyl, piperidinyl (e.g. piperidin-1-yl, piperidin-4-yl), piperazinyl (e.g. piperazin-1-yl) and imidazolyl (e.g. imidazole-5-yl).

Specific examples of R⁹ when substituted by R^{a}, R^{b} and/or R^{c} include the following groups:

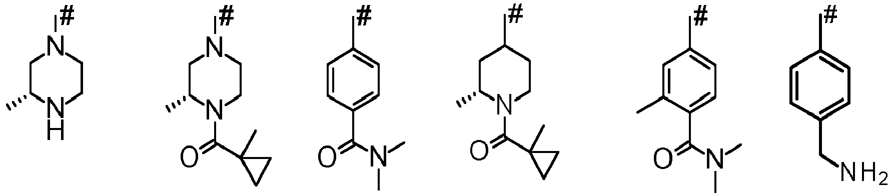

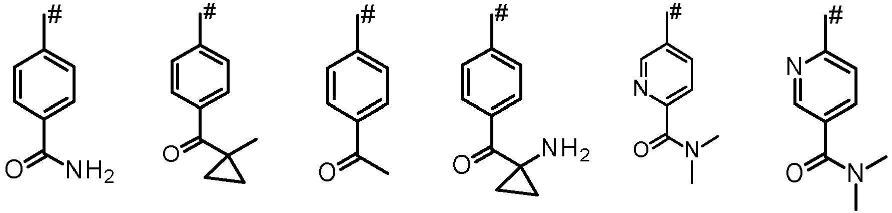

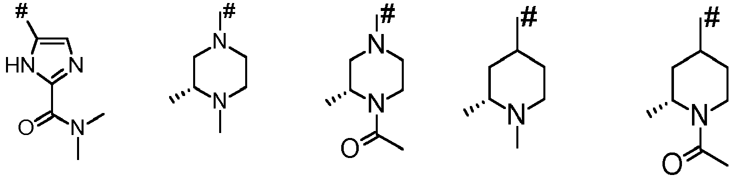

R^{a} is hydrogen, -N(R^{m})Rⁿ, C₁₋₆ alkyl, hydroxy, C₁₋₆ alkoxy, halogen, cyano, oxo, C₁₋₆ haloalkyl, C₁₋₆ haloalkoxy, hydroxyC₁₋₆ alkyl, heteroaryl, heterocyclyl, -C(O)R^{d}, -C(O)OR^{e}, -C(O)N(R^{f})R^{g}, - S(O)₂N(R^{h})Rⁱ or C₁₋₆ alkyl-N(R^{j})R^{k}, wherein the heteroaryl and heterocyclyl are optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy.

Preferably R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k}.

For example R^{a} is hydrogen, -NH₂, -NH(C₁₋₄ alkyl), -N(C₁₋₄ alkyl)₂, C₁₋₆ alkyl, oxo, -C(O)-C₃₋₆ cycloalkyl, -C(O)-C₃₋₆ cycloalkyl-NH₂, -C(O)-C₃₋₆ cycloalkyl-NH(C₁₋₄ alkyl), -C(O)-C₃₋₆ cycloalkyl-N(C₁₋₄ alkyl)₂, - C(O)-C₃₋₆ cycloalkyl-C₁₋₄ alkyl, -C(O)-C₃₋₆ cycloalkyl-C₁₋₄ alkyl-NH₂, -C(O)-C₃₋₆ cycloalkyl-C₁₋₄ alkyl-NH(C₁₋₄ alkyl), -C(O)-C₃₋₆ cycloalkyl-C₁₋₄ alkyl-N(C₁₋₄ alkyl)₂, -C(O)NH₂, -C(O)NH(C₁₋₄ alkyl), - C(O)N(C₁₋₄ alkyl)₂, C₁₋₆ alkyl-NH₂, C₁₋₆ alkyl-NH(C₁₋₄ alkyl), C₁₋₆ alkyl-N(C₁₋₄ alkyl)₂, -C(O)-C₁₋₆ alkyl, - C(O)-C₁₋₆ alkyl-NH₂,-C(O)-C₁₋₆ alkyl-NH(C₁₋₄ alkyl), -C(O)-C₁₋₆ alkyl-N(C₁₋₄ alkyl)₂,-C(O)-heterocyclyl, -C(O)-heterocyclyl-NH₂, -C(O)-heterocyclyl-NH(C₁₋₄ alkyl), -C(O)-heterocyclyl-N(C₁₋₄ alkyl)₂,-C(O)-heterocyclyl-C₁₋₄ alkyl, -C(O)-heterocyclyl-C₁₋₄ alkyl-NH₂, -C(O)-heterocyclyl-C₁₋₄ alkyl-NH(C₁₋₄ alkyl) or -C(O)-heterocyclyl-C₁₋₄ alkyl-N(C₁₋₄ alkyl)₂, wherein each heterocyclyl is a 4- to 6-membered monocyclic heterocyclic ring containing 1 or 2 heteroatoms as ring atoms independently selected from N, O and S, such as pyrrolidinyl or morpholinyl, and wherein heterocyclyl is optionally connected to the carbonyl moiety via a nitrogen atom.

For example R^{a} is hydrogen, C₁₋₄ alkyl, -C(O)-C₃₋₆ cycloalkyl, -C(O)-C₃₋₆ cycloalkyl-C₁₋₄ alkyl, -C(O)-C₃₋₆ cycloalkyl-NH₂, -C(O)-C₃₋₆ cycloalkyl-NH(C₁₋₄ alkyl), -C(O)-C₃₋₆ cycloalkyl-NH(C₁₋₄ alkyl)₂, - C(O)NH₂, -C(O)NH(C₁₋₄ alkyl), -C(O)N(C₁₋₄ alkyl)₂, C₁₋₄ alkyl-NH₂, C₁₋₄ alkyl-NH(C₁₋₄ alkyl) or C₁₋₄ alkyl-N(C₁₋₄ alkyl)₂.

Specific examples of R^{a} include hydrogen, -CH₃, -C(O)-CH₃, -C(O)-C(CH₂-CH₂)-CH₃, -C(O)-C(CH₂-CH₂)-NH₂, -C(O)-NH₂, -C(O)-N(CH₃)₂ and -CH₂-NH₂.

The moieties -C(O)-C(CH₂-CH₂)-CH₃ and -C(O)-C(CH₂-CH₂)-NH₂ are drawn respectively as

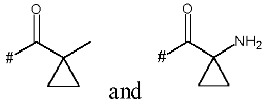

R^{b} is hydrogen, -N(R^{m})Rⁿ, C₁₋₆ alkyl, C₁₋₄ alkyl-N(R^{m})Rⁿ, hydroxy, C₁₋₆ alkoxy, halogen, cyano, C₁₋₆ haloalkyl or C₁₋₆ haloalkoxy.

Preferably R^{b} is hydrogen, amino, -NH(C₁₋₄ alkyl), -N(C₁₋₄ alkyl)₂, C₁₋₄ alkyl, C₁₋₄ alkyl-NH₂, C₁₋₄ alkyl-NH(C₁₋₄ alkyl), C₁₋₄ alkyl-N(C₁₋₄ alkyl)₂.

In some embodiments R^{b} is hydrogen or C₁₋₄ alkyl.

Specific examples of R^{b} include hydrogen and -CH₃.

R^{c} is hydrogen, -N(R^{m})Rⁿ, C₁₋₆ alkyl, C₁₋₄ alkyl-N(R^{m})Rⁿ, hydroxy, C₁₋₆ alkoxy, halogen, cyano, C₁₋₆ haloalkyl or C₁₋₆ haloalkoxy.

Preferably R^{c} is hydrogen or C₁₋₄ alkyl.

Specific examples of R^{c} include hydrogen and -CH₃.

R^{d} is hydrogen, C₁₋₆ alkyl, C₁₋₆ haloalkyl, C₃₋₆ cycloalkyl, phenyl, heteroaryl or heterocyclyl, wherein the cycloalkyl, phenyl, heteroaryl and heterocyclyl are optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy.

Preferably R^{d} is hydrogen, C₁₋₄ alkyl, C₃₋₆ cycloalkyl or 4- to 7-membered monocyclic heterocyclyl containing 1, 2 or 3 heteroatoms as ring atoms independently selected from N, S, and O, and wherein the cycloalkyl and heterocyclyl are optionally substituted by 1 or 2 substituents independently selected from C₁₋₄ alkyl, amino, -NH(C₁₋₄ alkyl), -N(C₁₋₄ alkyl)₂, C₁₋₆ alkyl-NH₂, C₁₋₆ alkyl-NH(C₁₋₄ alkyl), C₁₋₆ alkyl-N(C₁₋₄ alkyl)₂ and C₁₋₄ haloalkyl.

For example R^{d} is hydrogen, C₁₋₄ alkyl or C₃₋₆ cycloalkyl, wherein the cycloalkyl is optionally substituted by 1 or 2 substituents independently selected from C₁₋₄ alkyl, amino, -NH(C₁₋₄ alkyl), -N(C₁₋₄ alkyl)₂ and C₁₋₄ haloalkyl.

For example, R^{d} is hydrogen, C₁₋₆ alkyl, C₃₋₆ cycloalkyl, C₃₋₆ cycloalkyl-NH₂, C₃₋₆ cycloalkyl-NH(C₁₋₄ alkyl), C₃₋₆ cycloalkyl-N(C₁₋₄ alkyl)₂, C₃₋₆ cycloalkyl-C₁₋₄ alkyl, C₃₋₆ cycloalkyl-C₁₋₄ alkyl-NH₂, C₃₋₆ cycloalkyl-C₁₋₄ alkyl-NH(C₁₋₄ alkyl), C₃₋₆ cycloalkyl-C₁₋₄ alkyl-N(C₁₋₄ alkyl)₂, heterocyclyl, heterocyclyl-NH₂, heterocyclyl-NH(C₁₋₄ alkyl), heterocyclyl-N(C₁₋₄ alkyl)₂, heterocyclyl-C₁₋₄ alkyl, heterocyclyl-C₁₋₄ alkyl-NH₂, heterocyclyl-C₁₋₄ alkyl-NH(C₁₋₄ alkyl), heterocyclyl-C₁₋₄ alkyl-N(C₁₋₄ alkyl)₂, wherein each heterocyclyl is a 4- to 6-membered monocyclic heterocyclic ring containing 1 or 2 heteroatoms as ring atoms independently selected from N, O and S, such as pyrrolidinyl or morpholinyl.

Specific examples of R^{d} include hydrogen, -CH₃, -C(CH₂-CH₂)-NH₂ and -C(CH₂-CH₂)-CH₃.

R^{e} is hydrogen or C₁₋₆ alkyl.

R^{f} is hydrogen, C₁₋₆ alkyl, C₁₋₆ haloalkyl, aminoC₁₋₆ alkyl or hydroxyC₁₋₆ alkyl.

Preferably R^{f} is hydrogen or C₁₋₄ alkyl.

Specific examples of R^{f} include hydrogen.

R^{g} is hydrogen, C₁₋₆ alkyl, C₁₋₆ haloalkyl, aminoC₁₋₆ alkyl or hydroxyC₁₋₆ alkyl.

Preferably R^{g} is hydrogen or C₁₋₄ alkyl.

Specific examples of R^{g} include hydrogen.

Alternatively R^{f} and R^{g} together with the nitrogen atom to which they are attached form heterocyclyl, wherein heterocyclyl is optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy.

R^{h} is hydrogen, C₁₋₆ alkyl, C₁₋₆ haloalkyl, aminoC₁₋₆ alkyl or hydroxyC₁₋₆ alkyl.

Rⁱ is hydrogen, C₁₋₆ alkyl, C₁₋₆ haloalkyl, aminoC₁₋₆ alkyl or hydroxyC₁₋₆ alkyl.

Alternatively R^{h} and Rⁱ together with the nitrogen atom to which they are attached form heterocyclyl, wherein heterocyclyl is optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy .

R¹ is hydrogen, C₁₋₆ alkyl, C₁₋₆ haloalkyl, aminoC₁₋₆ alkyl or hydroxyC₁₋₆ alkyl.

Preferably R¹ is hydrogen or C₁₋₄ alkyl.

Specific examples of R^{j} include hydrogen.

R^{k} is hydrogen, C₁₋₆ alkyl, C₁₋₆ haloalkyl, aminoC₁₋₆ alkyl or hydroxyC₁₋₆ alkyl.

Preferably R^{k} is hydrogen or C₁₋₄ alkyl.

Specific examples of R^{k} include hydrogen.

Alternatively R^{j} and R^{k} together with the nitrogen atom to which they are attached form heterocyclyl, wherein heterocyclyl is optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy .

Each R^{m} is independently hydrogen, C₁₋₆ alkyl, C₁₋₆ haloalkyl, aminoC₁₋₆ alkyl or hydroxyC₁₋₆ alkyl. Preferably each R^{m} is independently hydrogen or C₁₋₄ alkyl.

Each Rⁿ is independently hydrogen, C₁₋₆ alkyl, C₁₋₆ haloalkyl, aminoC₁₋₆ alkyl or hydroxyC₁₋₆ alkyl. Preferably each Rⁿ is independently hydrogen or C₁₋₄ alkyl.

R⁴ is hydrogen or the group -L^{1A}-L^{2A}-L^{3A}.

In some embodiments R⁴ is the group -L^{1A}-L^{2A}-L^{3A}.

Specific examples of R⁴ include -CH₂-(methylpyrazol-4-yl) (e.g. -CH₂-(1-methylpyrazol-4-yl), -CH₂-(trifluoromethylpyrazol-4-yl) (e.g. -CH₂-(1-trifluoromethylpyrazol-4-yl) and -CH₂-(methylthiazol-5-yl) (e.g. -CH₂-(2-methylthiazol-5-yl)).

L^{1A} is absent or is C₁₋₃ alkylene optionally substituted by C₁₋₂ alkyl or oxo.

In some embodiments L^{1A} is C₁₋₃ alkylene.

In some embodiments L^{1A} is absent.

Specific examples of L^{1A} include -CH₂-.

L^{2A} is absent or is -O-, -S-, -S(O)-, -S(O)₂-, -N(R^{a1})-, -C(O)-, -C(O)O-, -OC(O)-, -C(O)N(R^{a1})-, - N(R^{a1})C(O)-, -N(R^{a1})C(O)N(R^{b1})-, -S(O)₂N(R^{a1})-, or -N(R^{a1})S(O)₂-.

In some embodiments L^{2A} is absent.

R^{a1} is hydrogen or C₁₋₂alkyl.

R^{b1} is hydrogen or C₁₋₂alkyl.

L^{3A} is hydrogen, C₁₋₆ alkyl, C₃₋₆ cycloalkyl, phenyl, heterocyclyl or heteroaryl, wherein L^{3A} is optionally substituted by one or more (e.g. 1, 2 or 3) substituents independently selected from halogen, cyano, hydroxy, carboxy, carbamoyl, sulphamoyl, C₁₋₄ alkyl, C₁₋₄ haloalkyl, C₁₋₄ alkoxy, -N(R^{c1})R^{d1}, -OR^{c1}, - C(O)R^{c1}, -C(O)OR^{c1}, -OC(P)R^{c1}, -C(O)N(R^{d1})R^{c1}, -N(R^{d1})C(O)R^{c1}, -S(O)_{y}R^{c1}, -S(O)₂N(R^{d1})R^{c1}, - N(R^{d1})S(O)₂R^{c1} and -(CH₂)_{z}N(R^{d1})R^{c1}.

In some embodiments L^{3A} is hydrogen, C₁₋₆ alkyl, C₃₋₆ cycloalkyl, phenyl, 5- to 7-membered monocyclic heterocyclyl containing 1, 2 or 3 heteroatoms as ring atoms independently selected from N, S and O, or 5-to 6-membered heteroaryl containing 1, 2 or 3 heteroatoms as ring atoms independently selected from N, S and O, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, - S(O)₂N(R^{d1})R^{c1}, -N(R^{d1})S(O)₂R^{c1} and -(CH₂)_{z}N(R^{d1})R^{c1}.

In some embodiments L^{3A} is phenyl or 5- to 6-membered heteroaryl containing 1, 2 or 3 heteroatoms as ring atoms independently selected from N, S and O, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, - C(O)N(R^{d1})R^{c1}, -N(R^{d1})C(O)R^{c1}, -S(O)yR^{c1} -S(O)₂N(R^{d1})R^{c1}, -N(R^{d1})S(O)₂R^{c1} and -(CH₂)_{z}N(R^{d1})R^{c1}. In some embodiments L^{3A} is phenyl or 5- to 6-membered heteroaryl containing 1 or 2 heteroatoms as ring atoms independently selected from N, S and O, wherein at least one heteroatom is N, wherein L^{3A} is optionally substituted by one or two substituents independently selected from halogen, cyano, methyl and trifluoromethyl.

Specific examples of L^{3A} include methylpyrazol-4-yl (e.g. 1-methylpyrazol-4-yl), trifluoromethylpyrazol-4-yl (e.g. 1-trifluoromethylpyrazol-4-yl) and methylthiazol-5-yl (e.g. -CH₂-(2-methylthiazol-5-yl).

R^{c1} is hydrogen or C₁₋₄ alkyl.

R^{d1} is hydrogen or C₁₋₄ alkyl.

y is 0, 1 or 2.

z is 1, 2 or 3.

In some embodiments
L^{1A} is C₁₋₃ alkylene;
L^{2A} is absent.
L^{3A} is phenyl or a 5- to 6-membered heteroaryl containing 1, 2 or 3 heteroatoms as ring atoms independently selected from N, O and S, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)yR^{c1} -S(O)₂N(R^{d1})R^{c1}, -N(R^{d1})S(O)₂R^{c1} and -(CH₂)_{z}N(R^{d1})R^{c1};
R^{c1} is hydrogen or C₁₋₄ alkyl;
R^{d1} is hydrogen or C₁₋₄ alkyl;
y is 0, 1 or 2; and
z is 1, 2 or 3.

In some embodiments
L^{1A} is C₁₋₃ alkylene;
L^{2A} is absent; and
L^{3A} is phenyl or 5- to 6-membered heteroaryl containing 1 or 2 heteroatoms as ring atoms independently selected from N, S and O, wherein at least one heteroatom is N, wherein L^{3A} is optionally substituted by one or two substituents independently selected from halogen, cyano, methyl and trifluoromethyl.

R⁵ is hydrogen or the group -L^{1B}-L^{2B}-L^{3B}.

In some embodiments R⁵ is the group -L^{1B}-L^{2B}-L^{3B}.

In some embodiments R⁵ is hydrogen or the group -L^{1B}-L^{2B}-L^{3B}, wherein the group -L^{1B}-L^{2B}-L^{3B} is C₁₋₆ alkyl.

Specific examples of R⁵ include -CH₃, -CH₂CH₃, -CH(CH₃)(CH₃) and -CH₂-(dimethylthiazol-5-yl) (e.g. - CH₂-(2,4-dimethylthiazol-5-yl)).

L^{1B} is absent or is C₁₋₃ alkylene optionally substituted by C₁₋₂ alkyl or oxo.

In some embodiments L^{1B} is C₁₋₃ alkylene.

In some embodiments L^{1B} is absent.

Specific examples of L^{1B} include -CH₂-.

L^{2B} is absent or is -O-, -S-, -S(O)-, -S(O)₂-, -N(R^{a2})-, -C(O)-, -C(O)O-, -OC(O)-, -C(O)N(R^{a2})-, - N(R^{a2})C(O)-, -N(R^{a2})C(O)N(R^{b2})-, -S(O)₂N(R^{a2})-, or -N(R^{a2})S(O)₂-.

In some embodiments L^{2B} is absent.

R^{a2} is hydrogen or C₁₋₂ alkyl.

R^{b2} is hydrogen or C₁₋₂ alkyl.

L^{3B} is hydrogen, C₁₋₆ alkyl, C₃₋₆ cycloalkyl, phenyl, heterocyclyl or heteroaryl, wherein L^{3B} is optionally substituted by one or more (e.g. 1, 2 or 3) substituents independently selected from halogen, trifluoromethyl, trifluoromethoxy, cyano, hydroxy, carboxy, carbamoyl, sulphamoyl, C₁₋₄ alkyl, C₁₋₄ haloalkyl, C₁₋₄ alkoxy, -N(R^{c2})R^{d2}, -OR^{c2}, -C(O)R^{c2}, -C(O)OR^{c2}, -OC(O)R^{c2}, -C(O)N(R²)R^{c2}, - N(R^{d2})C(O)R^{c2}, -S(O)_{y'}R^{c2}, -S(O)₂N(R^{d2})R^{c2}, -N(R^{d2})S(O)₂R^{c2} and -(CH₂)_{z'}N(R^{d2})R².

In some embodiments L^{3B} is hydrogen, C₁₋₆ alkyl, C₃₋₆ cycloalkyl, phenyl, 5- to 7-membered monocyclic heterocyclyl containing 1, 2 or 3 heteroatoms as ring atoms independently selected from N, S and O, or 5-to 6-membered heteroaryl containing 1, 2 or 3 heteroatoms as ring atoms independently selected from N, S and O, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y'}R^{c1}, - S(O)₂N(R^{d1})R^{c1}, -N(R^{d1})S(O)₂R^{c1} and -(CH₂)_{z'}N(R^{d1})R^{c1}.

In some embodiments L^{3B} is phenyl or 5- to 6-membered heteroaryl containing 1, 2 or 3 heteroatoms as ring atoms independently selected from N, S and O, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, - C(O)N(R^{d1})R^{c1}, -N(R^{d1})C(O)R^{c1}, -S(O)_{y'}R^{c1}, -S(O)₂N(R^{d1})R^{c1}, -N(R^{d1})S(O)₂R^{c1} and -(CH₂)_{z'}N(R^{d1})R^{c1}. In some embodiments L^{3B} is phenyl or 5- to 6-membered heteroaryl containing 1 or 2 heteroatoms as ring atoms independently selected from N, S and O, wherein at least one heteroatom is N, wherein L^{3B} is optionally substituted by one or two substituents independently selected from halogen, cyano, methyl and trifluoromethyl.

Specific examples of L^{3B} include -CH₃, -CH₂CH₃, -CH(CH₃)(CH₃) and dimethylthiazol-5-yl (e.g. 2,4-dimethylthiazol-5 -yl).

R^{c2} is hydrogen or C₁₋₄ alkyl.

R^{d2} is hydrogen or C₁₋₄ alkyl.

y' is 0, 1 or 2.

z' is 1, 2 or 3.

In some embodiments
L^{1B} is C₁₋₃ alkylene;
L^{2B} is absent;
L^{3B} is phenyl or a 5- to 6-membered heteroaryl containing 1, 2 or 3 heteroatoms as ring atoms selected from N, O and S, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, -C(O)R^{c2}, -C(O)OR^{c2}, -OC(O)R^{c2}, -C(O)N(R^{d2})R^{c2}, - N(R^{d2})C(O)R^{c2}, -S(O)_{y}^{,}R^{c2}, -S(O)₂N(R^{d2})R^{c2}, -N(R^{d2})S(O)₂R^{c2} and -(CH₂)_{z}^{,}N(R^{d2})R^{c2};
R^{c2} is hydrogen or C₁₋₄ alkyl;
R^{d2} is hydrogen or C₁₋₄ alkyl;
y' is 0, 1 or 2; and
z' is 1, 2 or 3.

In some embodiments
L^{1B} is C₁₋₃alkylene;
L^{2B} is absent; and
L^{3B} is phenyl or 5- to 6-membered heteroaryl containing 1 or 2 heteroatoms as ring atoms independently selected from N, S and O, wherein at least one heteroatom is N, wherein L^{3B} is optionally substituted by one or two substituents independently selected from halogen, cyano, methyl and trifluoromethyl.
In some embodiments -L^{1B}-L^{2B}-L^{3B} is C₁₋₆ alkyl.

R⁶ is hydrogen or the group -L^{1C}-L^{2C}-L^{3C}.

In some embodiments R⁶ is hydrogen or the group -L^{1C}-L^{2C}-L^{3C}, wherein the group -L^{1C}-L^{2C}-L^{3C} is C₁₋₆ alkyl.

Specific examples R⁶ include hydrogen.

L^{1C} is absent or is C₁₋₃ alkylene optionally substituted by C₁₋₂ alkyl or oxo.

In some embodiments L^{1C} is C₁₋₃ alkylene.

In some embodiments L^{1C} is absent.

L^{2C} is absent or is -O-, -S-, -S(O)-, -S(O)₂-, -N(R^{a3})-, -C(O)-, -C(O)O-, -OC(O)-, -C(O)N(R^{a3})-,-N(R^{a3})C(O)-, -N(R^{a3})C(O)N(R^{b3})-, -S(O)₂N(R³)-, or -N(R^{a3})S(O)₂-.

In some embodiments L^{2C} is absent.

R^{a3} is hydrogen or C₁₋₂ alkyl.

R^{b3} is hydrogen or C₁₋₂ alkyl.

L^{3C} is hydrogen, C₁₋₆ alkyl, C₃₋₆ cycloalkyl, phenyl, heterocyclyl or heteroaryl, wherein L^{3C} is optionally substituted by one or more substituents independently selected from halogen, trifluoromethyl, trifluoromethoxy, cyano, hydroxy, carboxy, carbamoyl, sulphamoyl, C₁₋₄ alkyl, C₁₋₄ haloalkyl, C₁₋₄ alkoxy, -N(R^{c3})R^{d3}, -OR^{c3}, -C(O)R^{c3}, -C(O)OR^{c3}, -OC(O)R^{c3}, -C(O)N(R^{d3})R^{c3}, -N(R^{d3})C(O)R^{c3},-S(O)_{y}^{,,}R^{c3}, -S(O)₂N(R^{d3})R^{c3}, -N(R^{d3})S(O)₂R^{c3} and -(CH₂)_{z}^{,,}N(R^{d3})R^{c3}.

In some embodiments L^{3C} is hydrogen, C₁₋₆ alkyl, C₃₋₆ cycloalkyl, phenyl, 5- to 7-membered monocyclic heterocyclyl containing 1, 2 or 3 heteroatoms as ring atoms independently selected from N, S and O, or 5-to 6-membered heteroaryl containing 1, 2 or 3 heteroatoms as ring atoms independently selected from N, S and O, wherein L^{3C} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}^{,,}R^{c1}, - S(O)₂N(R^{d1})R^{c1}, -N(R^{d1})S(O)₂R^{c1} and -(CH₂)_{z}^{,,}N(R^{d1})R^{c1}.

In some embodiments L^{3C} is phenyl or 5- to 6-membered heteroaryl containing 1, 2 or 3 heteroatoms as ring atoms independently selected from N, S and O, wherein L^{3C} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, - C(O)N(R^{d1})R^{c1}, -N(R^{d1})C(O)R^{c1}, -S(O)_{y}^{,,}R^{c1}, -S(O)₂N(R^{d1})R^{c1}, -N(R^{d1})S(O)₂R^{c1} and -(CH₂)_{z"}N(R^{d1})R^{c1}. In some embodiments L^{3C} is phenyl or 5- to 6-membered heteroaryl containing 1 or 2 heteroatoms as ring atoms independently selected from N, S and O, wherein at least one heteroatom is N, wherein L^{3C} is optionally substituted by one or two substituents independently selected from halogen, cyano, methyl and trifluoromethyl.

R^{c3} is hydrogen or C₁₋₄ alkyl.

R^{d3} is hydrogen or C₁₋₄ alkyl.

y" is 0, 1 or 2.

z" is 1, 2 or 3.

In the compounds of formula (I) the groups -L^{1A}-L^{2A}-L^{3A}, -L^{1B}-L^{2B}-L^{3B}, and -L^{1C}-L^{2C}-L^{3C} do not contain an -O-O-, -S-O-, -O-S- or -S-S- unit as a linking unit within the group. For the avoidance of doubt this does not exclude oxidized forms of sulfur where the oxygen atom is not part of the linking unit, e.g. -S(O)-, - S(O)₂-.

In the compounds of formula (I) the group -L^{1A}-L^{2A}-L^{3A} does not place an -O-N- or -S-N- unit adjacent to the ring nitrogen atom connected to R⁴; the group -L^{1B}-L^{2B}-L^{3B} does not place an N, O or S atom adjacent to the oxime oxygen atom connected to R⁵, and the group -L^{1C}-L^{2C}-L^{3C} does not place an -O-N- or -S-N-unit adjacent to the ring nitrogen atom connected to R⁶.

In some embodiments the compound of formula (I) is a compound of formula (la).

In some embodiments the compound of formula (I) is a compound of formula (Ib).

In some embodiments
R¹ is methyl or cyano; and
R² and R³ together with the carbon atom to which they are attached form cyclopropyl or oxetanyl.

In some embodiments R⁹ is C₃₋₆ cycloalkyl, phenyl, heteroaryl (in particular 5-to 6-membered heteroaryl containing 1, 2 or 3 heteroatoms as ring atoms independently selected from N, S and O), heterocyclyl (in particular 5- to 7-membered monocyclic heterocyclyl containing 1, 2 or 3 heteroatoms as ring atoms independently selected from N, S and O), wherein the cycloalkyl, phenyl, heteroaryl and heterocyclyl are optionally substituted with R^{a}, R^{b} and/or R^{c};
or R⁹ is spiro heterocyclyl (in particular a spiro ring system wherein the first ring connected to X³ is a 5-to 7-membered monocyclic heterocyclyl containing 1, 2 or 3 heteroatoms as ring atoms independently selected from N, S and O, in particular 1 to 2 heteroatoms as ring atoms selected from N) and the second ring is connected to the first ring with a common carbon atom and the second ring is a 3- to 6-membered monocyclic cycloalkyl or a 3- to 6-membered monocyclic heterocyclyl containing 1 or 2 heteroatoms as ring atoms independently selected from N, S and O, in particular 1 heteroatom as ring atom selected from O and S, in particular selected from O), wherein spiro heterocyclyl is optionally substituted by R^{a}, R^{b} and/or R^{c}.

In some embodiments R⁹ is phenyl, 5- to 6-membered heteroaryl containing 1 or 2 heteroatoms as ring atoms selected from N, or 6-membered monocyclic heterocyclyl containing 1 to 2 heteroatoms as ring atoms selected from N and O, wherein phenyl, heteroaryl and heterocyclyl are optionally substituted with R^{a}, R^{b} and/or R^{c}; or
R⁹ is a spiro ring system wherein the first ring connected to X³ is a 4 to 6-membered monocyclic heterocyclyl containing 1 to 2 heteroatoms as ring atoms selected from N and the second ring is connected to the first ring with a common carbon atom and the second ring is a 4- to 6-membered monocyclic heterocyclyl containing 1 heteroatom as ring atom independently selected from N, O and S, in particular selected from O, wherein the first ring of the spiro heterocyclyl is optionally substituted by R^{b} and/or R^{c}.

In some embodiments R⁹ is phenyl, 5- to 6-membered heteroaryl containing 1 or 2 heteroatoms as ring atoms selected from N, wherein the phenyl and 6-membered heteroaryl are optionally substituted at the para position with respect to the connection to the rest of the molecule with R^{a} and are additionally optionally substituted with R^{b}, and the 5-membered heteroaryl is optionally substituted at the 3 position distal to the connection to the rest of the molecule with R^{a} and is additionally optionally substituted with R^{b}; or
R⁹ is the moiety (R^{9a}) or (R^{9b}) as depicted above wherein Z¹ is N or CH, Z² is N(R^{a}), O, S, CH(R^{a}) or C(R^{x})(R^{y}), wherein R^{x} and R^{y} together form a 4- to 6-membered monocyclic heterocyclyl containing one heteroatom selected from N, O and S; and wherein optionally at least one of Z¹ and Z² is N or N(R^{a}) respectively;
and wherein R^{a} may be C₁₋₄ alkyl, -C(O)R^{d}, -C(O)N(R^{f})R^{g} or C₁₋₆ alkyl-N(R^{j})R^{k}, and optionally R^{d} may be C₁₋₄ alkyl or C₃₋₆ cycloalkyl, wherein the cycloalkyl is optionally substituted by 1 or 2 substituents independently selected from C₁₋₄ alkyl, amino, -NH(C₁₋₄ alkyl), -N(C₁₋₄ alkyl)₂ and C₁₋₄ haloalkyl.

In some embodiments
R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k}.
R^{b} is hydrogen, amino, -NH(C₁₋₄ alkyl), -N(C₁₋₄ alkyl)₂, C₁₋₄ alkyl, C₁₋₄ alkyl-NH₂, C₁₋₄ alkyl-NH(C₁₋₄ alkyl) or C₁₋₄ alkyl-N(C₁₋₄ alkyl)₂;
R^{c} is hydrogen or C₁₋₄ alkyl;
R^{d} is hydrogen, C₁₋₄ alkyl, C₃₋₆ cycloalkyl or 4- to 7-membered monocyclic heterocyclyl containing 1, 2 or 3 heteroatoms as ring atoms independently selected from N, S, and O, and wherein the cycloalkyl and heterocyclyl are optionally substituted by 1 or 2 substituents independently selected from C₁₋₄ alkyl, amino, -NH(C₁₋₄ alkyl), -N(C₁₋₄ alkyl)₂, C₁₋₆ alkyl-NH₂, C₁₋₆ alkyl-NH(C₁₋₄ alkyl), C₁₋₆ alkyl-N(C₁₋₄ alkyl)₂ and C₁₋₄ haloalkyl;
R^{f} is hydrogen or C₁₋₄ alkyl;
R^{g} is hydrogen or C₁₋₄ alkyl;
R^{j} is hydrogen or C₁₋₄ alkyl;
R^{k} is hydrogen or C₁₋₄ alkyl;
R^{m} is hydrogen or C₁₋₄ alkyl; and
Rⁿ is hydrogen or C₁₋₄ alkyl.

In some embodiments L^{3C} is not cycloalkyl, phenyl, heterocyclyl or heteroaryl, each optionally substituted, when R⁹ is cycloalkyl, phenyl, heteroaryl, heterocyclyl, fused heterocyclyl, spiro heterocyclyl or bridged heterocyclyl, each optionally substituted.

In some embodiments no more than two of L^{3A}, L^{3B}, and L^{3C} are cycloalkyl, phenyl, heterocyclyl or heteroaryl, each optionally substituted.

In some embodiments no more than two of L^{3A}, L^{3B}, and L^{3C} are cycloalkyl, phenyl, heterocyclyl or heteroaryl each optionally substituted; and L^{3C} is not cycloalkyl, phenyl, heterocyclyl or heteroaryl, each optionally substituted, when R⁹ is cycloalkyl, phenyl, heteroaryl, heterocyclyl, fused heterocyclyl, spiro heterocyclyl or bridged heterocyclyl, each optionally substituted.

In some embodiments at least one of L^{3A}, L^{3B}, and L^{3C} is present and is cycloalkyl, phenyl, heterocyclyl or heteroaryl, each optionally substituted.

In some embodiments at least one of L^{3A}, L^{3B}, and L^{3C} is present and is phenyl or heteroaryl, each optionally substituted.

In some embodiments at least one of L^{3A} and L^{3B} is present and is cycloalkyl, phenyl, heterocyclyl or heteroaryl, each optionally substituted.

In some embodiments at least one of L^{3A} and L^{3B} is present and is phenyl or heteroaryl, each optionally substituted.

In some embodiments L^{3A} is present and is cycloalkyl, phenyl, heterocyclyl or heteroaryl, each optionally substituted.

In some embodiments L^{3A} is present and is phenyl or heteroaryl, each optionally substituted.

In some embodiments R⁴ is the group -L^{1A}-L^{2A}-L^{3A}, wherein L^{2A} is absent and L^{3A} is cycloalkyl, phenyl, heterocyclyl or heteroaryl, each optionally substituted; and R⁶ is hydrogen or C₁₋₆ alkyl.

In some embodiments R⁴ is the group -L^{1A}-L^{2A}-L^{3A}, wherein L^{2A} is absent and L^{3A} is phenyl or heteroaryl, each optionally substituted; and R⁶ is hydrogen or C₁₋₆ alkyl.

In some embodiments R⁴ is the group -L^{1A}-L^{2A}-L^{3A}, wherein L^{2A} is absent and L^{3A} is cycloalkyl, phenyl, heterocyclyl or heteroaryl, each optionally substituted; R⁵ is hydrogen or the group -L^{1B}-L^{2B}-L^{3B}, wherein L^{2B} is absent; and R⁶ is hydrogen or C₁₋₆ alkyl.

In some embodiments R⁴ is the group -L^{1A}-L^{2A}-L^{3A} , wherein L^{2A} is absent and L^{3A} is phenyl or heteroaryl, each optionally substituted; R⁵ is hydrogen or the group -L^{1B}-L^{2B}-L^{3B}, wherein L^{2B} is absent; and R⁶ is hydrogen or C₁₋₆ alkyl.

In some embodiments L^{2A}, L^{2B} and L^{2C} are absent.

In some embodiments R⁴ is the group -L^{1A}-L^{2A}-L^{3A}, wherein L^{3A} is cycloalkyl, phenyl, heterocyclyl or heteroaryl, each optionally substituted; R⁵ is hydrogen or the group -L^{1B}-L^{2B}-L^{3B}, wherein L^{3B} is cycloalkyl, phenyl, heterocyclyl or heteroaryl, each optionally substituted, or the group -L^{1B}-L^{2B}-L^{3B} is C₁₋₆ alkyl; and R⁶ is hydrogen or C₁₋₆alkyl.

In some embodiments R⁴ is the group -L^{1A}-L^{2A}-L^{3A}, wherein L^{3A} is phenyl or heteroaryl, each optionally substituted; R⁵ is hydrogen or the group -L^{1B}-L^{2B}-L^{3B}, wherein L^{3B} is phenyl or heteroaryl, each optionally substituted, or the group -L^{1B}-L^{2B}-L^{3B} is C₁₋₆ alkyl; and R⁶ is hydrogen or C₁₋₆alkyl.

In some embodiments R⁴ is the group -L^{1A}-L^{2A}-L^{3A}; R⁵ is hydrogen or C₁₋₆ alkyl; and R⁶ is hydrogen or C₁₋₆ alkyl.

In some embodiments R⁴ is the group -L^{1A}-L^{2A}-L^{3A}, wherein L^{2A} is absent and L^{3A} is cycloalkyl, phenyl, heterocyclyl or heteroaryl, each optionally substituted; R⁵ is hydrogen or C₁₋₆ alkyl; and R⁶ is hydrogen or C₁₋₆ alkyl.

In some embodiments R⁴ is the group -L^{1A}-L^{2A}-L^{3A}, wherein L^{2A} is absent and L^{3A} is phenyl or heteroaryl, each optionally substituted; R⁵ is hydrogen or C₁₋₆ alkyl; and R⁶ is hydrogen or C₁₋₆ alkyl.

In some embodiments
R⁴ is the group -L^{1A}-L^{2A}-L^{3A};
L^{1A} is C₁₋₃ alkylene;
L^{2A} is absent;
L^{3A} is phenyl or a 5- to 6-membered heteroaryl containing 1, 2 or 3 heteroatoms as ring atoms independently selected from N, O and S, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, -N(R^{d1})S(O)₂R^{c1} and -(CH₂)_{z}N(R^{d1})R^{c1};
R^{c1} is hydrogen or C₁₋₄ alkyl;
R^{d1} is hydrogen or C₁₋₄ alkyl;
y is 0, 1 or 2;
z is 1, 2 or 3;
R⁵ is hydrogen or the group -L^{1B}-L^{2B}-L^{3B};
L^{1B} is C₁₋₃ alkylene;
L^{2B} is absent;
L^{3B} is phenyl or a 5- to 6-membered heteroaryl containing 1, 2 or 3 heteroatoms as ring atoms selected from N, O and S, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, -C(O)R^{c2}, -C(O)OR^{c2}, -OC(O)R^{c2}, -C(O)N(R^{d2})R^{c2},-N(R^{d2})C(0)Rc^{z}, -S(O)_{y}^{,}R^{c2}, -S(O)₂N(R^{d2})R^{c2}, -N(R^{d2})S(O)₂R^{c2} and -(CH₂)_{z}^{,}N(R^{d2})R^{c2};
R^{c2} is hydrogen or C₁₋₄ alkyl;
R^{d2} is hydrogen or C₁₋₄ alkyl;
y' is 0, 1 or 2; and
z' is 1, 2 or 3.
or the group -L^{1B}-L^{2B}-L^{3B} is C₁₋₆ alkyl; and
R⁶ is hydrogen or the group -L^{1C}-L^{2C}-L^{3C}, wherein -L^{1C}-L^{2C}-L^{3C} is C₁₋₆ alkyl.

In some embodiments
R⁴ is the group -L^{1A}-L^{2A}-L^{3A};
L^{1A} is C₁₋₃ alkylene;
L^{2A} is absent;
L^{3A} is phenyl or a 5- to 6-membered heteroaryl containing 1, 2 or 3 heteroatoms as ring atoms independently selected from N, O and S, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, -N(R^{d1})S(O)₂R^{c1} and -(CH₂)_{z}N(R^{d1})R^{c1};
R^{c1} is hydrogen or C₁₋₄ alkyl;
R^{d1} is hydrogen or C₁₋₄ alkyl;
y is 0, 1 or 2;
z is 1, 2 or 3;
R⁵ is hydrogen or C₁₋₆ alkyl; and
R⁶ is hydrogen or C₁₋₆ alkyl.

In some embodiments
R⁴ is the group -L^{1A}-L^{2A}-L^{3A};
L^{1A} is C₁₋₃ alkylene;
L^{2A} is absent;
L^{3A} is phenyl or 5- to 6-membered heteroaryl containing 1 or 2 heteroatoms as ring atoms independently selected from N, S and O, wherein at least one heteroatom is N, wherein L^{3A} is optionally substituted by one or two substituents independently selected from halogen, cyano, methyl and trifluoromethyl;
R⁵ is hydrogen or the group -L^{1B}-L^{2B}-L^{3B};
L^{1B} is C₁₋₃alkylene;
L^{2B} is absent; and
L^{3B} is phenyl or 5- to 6-membered heteroaryl containing 1 or 2 heteroatoms as ring atoms independently selected from N, S and O, wherein at least one heteroatom is N, wherein L^{3B} is optionally substituted by one or two substituents independently selected from halogen, cyano, methyl and trifluoromethyl;
or -L^{1B}-L^{2B}-L^{3B} is C₁₋₆ alkyl; and
R⁶ is hydrogen.

In some embodiments
R⁴ is the group -L^{1A}-L^{2A}-L^{3A};
L^{1A} is C₁₋₃ alkylene;
L^{2A} is absent;
L^{3A} is phenyl or 5- to 6-membered heteroaryl containing 1 or 2 heteroatoms as ring atoms independently selected from N, S and O, wherein at least one heteroatom is N, wherein L^{3A} is optionally substituted by one or two substituents independently selected from halogen, cyano, methyl and trifluoromethyl;
R⁵ is hydrogen or C₁₋₆ alkyl; and
R⁶ is hydrogen.

In some embodiments (Embodiment A) the compound is a compound of formula (I) wherein
R¹ is cyano, C₁₋₂ alkyl or ethynyl;
R² and R³ together with the carbon atom to which they are attached form cyclopropyl or oxetanyl;
X¹ is CR⁷;
X² is CR⁸;
X³ is CR⁹;
Rand R⁸ are hydrogen;
R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, 5- to 6-membered heteroaryl containing 1, 2 or 3 heteroatoms as ring atoms independently selected from N, S and O, 5- to 7-membered monocyclic heterocyclyl containing 1, 2 or 3 heteroatoms as ring atoms independently selected from N, S and O, wherein the cycloalkyl, phenyl, heteroaryl and heterocyclyl are optionally substituted with R^{a}, R^{b} and/or R^{c};
or R⁹ is spiro heterocyclyl, wherein the first ring connected to X³ is a 5- to 7-membered monocyclic heterocyclyl containing 1, 2 or 3 heteroatoms as ring atoms independently selected from N, S and O and the second ring is connected to the first ring with a common carbon atom and is a 3- to 6-membered monocyclic cycloalkyl or a 3- to 6-membered monocyclic heterocyclyl containing 1, 2 or 3 heteroatoms as ring atoms independently selected from N, S and O, wherein spiro heterocyclyl is optionally substituted by R^{a}, R^{b} and/or R^{c};
R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k};
R^{b} is hydrogen -amino, -NH(C₁₋₄ alkyl), -N(C₁₋₄ alkyl)₂, C₁₋₄ alkyl, C₁₋₄ alkyl-NH₂, C₁₋₄ alkyl-NH(C₁₋₄ alkyl), C₁₋₄ alkyl-N(C₁₋₄ alkyl)₂;
R^{c} is hydrogen:
   R^{d} is hydrogen, C₁₋₄ alkyl, C₃₋₆ cycloalkyl or 4- to 7-membered monocyclic heterocyclyl containing 1, 2 or 3 heteroatoms as ring atoms independently selected from N, S, and O, and wherein the cycloalkyl and heterocyclyl are optionally substituted by 1 or 2 substituents independently selected from C₁₋₄ alkyl, amino, -NH(C₁₋₄ alkyl), -N(C₁₋₄ alkyl)₂, C₁₋₆ alkyl-NH₂, C₁₋₆ alkyl-NH(C₁₋₄ alkyl), C₁₋₆ alkyl-N(C₁₋₄ alkyl)₂ and C₁₋₄ haloalkyl;
   R^{f} is hydrogen or C₁₋₄ alkyl;
   R^{g} is hydrogen or C₁₋₄ alkyl;
   R^{j} is hydrogen or C₁₋₄ alkyl;
   R^{k} is hydrogen or C₁₋₄ alkyl;
   each R^{m} is independently hydrogen or C₁₋₄ alkyl;
   each Rⁿ is independently hydrogen or C₁₋₄ alkyl;
   R⁴ is the group -L^{1A}-L^{2A}-L^{3A};
   L^{1A} is C₁₋₃alkylene;
   L^{2A} is absent;
   L^{3A} is phenyl or 5- to 6-membered heteroaryl containing 1, 2 or 3 heteroatoms as ring atoms independently selected from N, S and O, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, -N(R^{d1})S(O)₂R^{c1} and -(CH₂)_{z}N(R^{d1})R^{c1};
   or the group -L^{1A}-L^{2A}-L^{3A} is C₁₋₆ alkyl;
   R^{c1} is hydrogen or C₁₋₄ alkyl;
   R^{d1} is hydrogen or C₁₋₄ alkyl;
   y is 0, 1 or 2;
   z is 1, 2 or 3;
   R⁵ is hydrogen or the group -L^{1B}-L^{2B}-L^{3B};
   L^{1B} is C₁₋₃ alkylene;
   L^{2B} is absent;
   L^{3B} is phenyl or a 5- to 6-membered heteroaryl containing 1, 2 or 3 heteroatoms as ring atoms independently selected from N, O and S, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, -C(O)R^{c2}, -C(O)OR^{c2}, -OC(O)R^{c2}, -C(O)N(R^{d2})R^{c2}, -N(R^{d2})C(O)R^{c2}, -S(O)_{y}^{,}R^{c2}, -S(O)₂N(R^{d2})R^{c2}, -N(R^{d2})S(O)₂R^{c2} and -(CH₂)_{z}^{,}N(R^{d2})R^{c2};
   R^{c2} is hydrogen or C₁₋₄ alkyl;
   R^{d2} is hydrogen or C₁₋₄ alkyl;
   y' is 0, 1 or 2;
   z' is 1, 2 or 3;
   or the group -L^{1B}-L^{2B}-L^{3B} is C₁₋₆ alkyl;
   R⁶ is hydrogen or the group -L^{1C}-L^{2C}-L^{3C}, wherein the group -L^{1C}-L^{2C}-L^{3C} is C₁₋₆ alkyl.

In some embodiments (Embodiment B) the compound is a compound of formula (I) wherein
R¹ is cyano or C₁₋₂ alkyl;
R² and R³ together with the carbon atom to which they are attached form cyclopropyl or oxetanyl;
X¹ is CR⁷;
X² is CR⁸;
X³ is CR⁹;
R⁷ and R⁸ are hydrogen;
R⁹ is hydrogen, halogen, cyano, phenyl, 5- to 6-membered heteroaryl containing 1 or 2 heteroatoms as ring atoms selected from N, wherein the phenyl and 6-membered heteroaryl are optionally substituted at the para position with respect to the connection to the rest of the molecule with R^{a} and are additionally optionally substituted with R^{b}, and the 5-membered heteroaryl is optionally substituted at the 3 position distal to the connection to the rest of the molecule with R^{a} and is additionally optionally substituted with R^{b}; or R⁹ is the moiety (R^{9a}) or the moiety (R^{9b}): 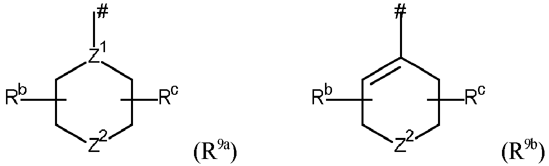 wherein Z¹ is N or CH, Z² is N(R^{a}), O, S, CH(R^{a}) or C(R^{x})(R^{y}), wherein R^{x} and R^{y} together form a 4- to 6-membered monocyclic heterocyclyl containing one heteroatom selected from N, O and S; and wherein optionally at least one of Z¹ and Z² is N or N(R^{a}) respectively;
R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k};
R^{b} is hydrogen or C₁₋₄ alkyl;
R^{c} is hydrogen:
   R^{d} is hydrogen, C₁₋₄ alkyl or C₃₋₆ cycloalkyl, wherein the cycloalkyl is optionally substituted by 1 or 2 substituents independently selected from C₁₋₄ alkyl, amino, -NH(C₁₋₄ alkyl), -N(C₁₋₄ alkyl)₂ and C₁₋₄ haloalkyl;
   R^{f} is hydrogen or C₁₋₄ alkyl;
   R^{g} is hydrogen or C₁₋₄ alkyl;
   R^{j} is hydrogen or C₁₋₄ alkyl;
   R^{k} is hydrogen or C₁₋₄ alkyl;
   R^{m} is hydrogen or C₁₋₄ alkyl;
   Rⁿ is hydrogen or C₁₋₄ alkyl;
   R⁴ is the group -L^{1A}-L^{2A}-L^{3A};
   L^{1A} is C₁₋₃ alkylene;
   L^{2A} is absent;
   L^{3A} is phenyl or 5- to 6-membered heteroaryl containing 1 or 2 heteroatoms as ring atoms independently selected from N, S and O, wherein at least one heteroatom is N, wherein L^{3A} is optionally substituted by one or two substituents independently selected from halogen, cyano, methyl and trifluoromethyl;
   R⁵ is hydrogen or the group -L^{1B}-L^{2B}-L^{3B};
   L^{1B} is C₁₋₃ alkylene;
   L^{2B} is absent;
   L^{3B} is phenyl or 5- to 6-membered heteroaryl containing 1 or 2 heteroatoms as ring atoms independently selected from N, S and O, wherein at least one heteroatom is N, wherein L^{3B} is optionally substituted by one or two substituents independently selected from halogen, cyano, methyl and trifluoromethyl;
   or the group -L^{1B}-L^{2B}-L^{3B} is C₁₋₆ alkyl;
   R⁶ is hydrogen or the group -L^{1C}-L^{2C}-L^{3C}, wherein the group -L^{1C}-L^{2C}-L^{3C} is C₁₋₆ alkyl.

In an embodiment (Embodiment C) the compound is a compound of formula (I) wherein
R¹ is cyano or -CH₃;
R² and R³ together with the carbon atom to which they are attached form cyclopropyl or oxetanyl;
X¹ is CR⁷;
X² is CR⁸;
X³ is CR⁹;
R⁷ and R⁸ are hydrogen;
R⁹ is hydrogen, chloro, phenyl, piperidinyl (e.g. piperidin-1-yl, piperidin-4-yl), piperazinyl (e.g. piperazin-1-yl) or imidazolyl (e.g. imidazole-5-yl), wherein the phenyl, piperidinyl, piperazinyl and imidazolyl are optionally substituted by R^{a}, R^{b} and/or R^{c};
R^{a} is hydrogen, -CH₃, -C(O)-CH₃, -C(O)-C(CH₂-CH₂)-CH₃, -C(O)-C(CH₂-CH₂)-NH₂, -C(O)-NH₂, -C(O)-N(CH₃)₂ or -CH₂-NH₂;
R^{b} is hydrogen or -CH₃;
R^{c} is hydrogen or -CH₃;
R⁴ is -CH₂-(methylpyrazol-4-yl) (e.g. -CH₂-(1-methylpyrazol-4-yl), -CH₂-(trifluoromethylpyrazol-4-yl) (e.g. -CH₂-(1-trifluoromethylpyrazol-4-yl) or -CH₂-(methylthiazol-5-yl) (e.g. -CH₂-(2-methylthiazol-5-yl));
R⁵ is -CH₃, -CH₂CH₃, -CH(CH₃)(CH₃) or -CH₂-(dimethylthiazol-5-yl) (e.g. -CH₂-(2,4-dimethylthiazol-5-yl)); and
R⁶ is hydrogen.

In further embodiments the invention provides the following compounds and pharmaceutically acceptable salts thereof:
2-Methoxyimino-*N*-(1-methylcyclopropyl)-3-[(2-methylthiazol-5-yl)methyl]-4-oxo-1*H*-quinazoline-6-sulfonamide;
2-methoxyimino-*N*-(3-methyloxetan-3-yl)-3-[(1-methylpyrazol-4-yl)methyl]-4-oxo-1*H*-quinazoline-6-sulfonamide;
2-[(2,4-dimethylthiazol-5-yl)methoxyimino]*-N*-(1-methylcyclopropyl)-3-[(1-methylpyrazol-4-yl)methyl] - 4-oxo-1*H*-quinazoline-6-sulfonamide;
8-chloro-2-methoxyimino-*N*-(1-methylcyclopropyl)-3-[(1-methylpyrazol-4-yl)methyl]-4-oxo-1*H-*quinazoline-6-sulfonamide;
4-[2-methoxyimino-6-[(1-methylcyclopropyl)sulfamoyl]-3-[(1-methylpyrazol-4-yl)methyl]-4-oxo-1*H-*quinazolin-8-yl]-*N*,*N*-dimethyl-benzamide;
2-methoxyimino-*N*(1-methylcyclopropyl)-4-oxo-3-[[1-(trifluoromethyl)pyrazol-4-yl]methyl]-1*H-*quinazoline-6-sulfonamide;
2-methoxyimino-*N*(1-methylcyclopropyl)-3-[(1-methylpyrazol-4-yl)methyl]-4-oxo-8-[(3*R*)-3-methylpiperazin-1-yl]-1*H*-quinazoline-6-sulfonamide;
2-methoxyimino-*N*-(1-methylcyclopropyl)-3-[(1-methylpyrazol-4-yl)methyl]-4-oxo-8-[(3*R*)-3-methyl-4-(1-methylcyclopropanecarbonyl)piperazin-1-yl]-1*H*-quinazoline-6-sulfonamide;
N(1-cyanocyclopropyl)-2-methoxyimino-3-[(1-methylpyrazol-4-yl)methyl]-4-oxo-1*H*-quinazoline-6-sulfonamide;
2-ethoxyimino-*N*-(1-methylcyclopropyl)-3-[(1-methylpyrazol-4-yl)methyl]-4-oxo-1*H*-quinazoline-6-sulfonamide; and
2-isopropoxyimino-*N*-(1-methylcyclopropyl)-3-[(1-methylpyrazol-4-yl)methyl]-4-oxo-1*H*-quinazoline-6-sulfonamide.

Some intermediates useful for the preparation of compounds of formula (I) are new and form further aspects of the invention. Accordingly, in a further aspect the invention provides compounds of formula (Int-I) 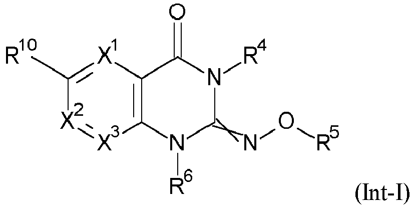 or a salt thereof, wherein
R¹⁰ is hydrogen, halogen (e.g. chloro, bromo, iodo), -B(OH)₂, -B(-O-C(CH₃)₂-C(CH₃)₂-O-) (i.e. pinacol boronate), -S(O)₂OH, -S(O)₂Cl or -S-CH₂-phenyl; and
X¹, X², X³, R⁴, R⁵ and R⁶ are as defined for the compound of formula (I), including preferred definitions and embodiments thereof;
and wherein the compound of (Int-I) is not
2-(Hydroxyamino)-6-iodo-3-phenyl-4(3*H*)-quinazolinone (CAS RN: 221657-59-6);
6-Iodo-2-(propoxyamino)-3-propyl-4(3*H*)-quinazolinone (CAS RN: 1101794-34-6);
6-Bromo-2-(propoxyamino)-3-propyl-4(3*H*)-quinazolinone (CAS RN: 1101796-68-2);
6-Iodo-2-[(2-methylpropoxy)amino]-3-propyl-4(3*H*)-quinazolinone (CAS RN: 1101796-45-5);
6-Bromo-2-[(2-methylpropoxy)amino]-3-propyl-4(3*H*)-quinazolinone (CAS RN: 1101794-11-9);
2-[[(3,4-Dihydro-6-iodo-4-oxo-3-phenyl-2-quinazolinyl)amino]oxy]acetic acid (CAS RN: 221657-88-1);
(3,4-Dihydro-6-iodo-4-oxo-3-phenyl-2-quinazolinyl)azanyl acetate (CAS RN: 221658-07-7);
2,4(1*H*,3*H*)-Quinazolinedione, 6-iodo-3-phenyl-, 2-[O-(ethoxycarbonyl)oxime] (CAS RN: 221657-74-5);
(3,4-Dihydro-6-iodo-4-oxo-3-phenyl-2-quinazolinyl)azanyl 2-chloroacetate (CAS RN: 221657-64-3);
Ethyl 2-[[(3,4-dihydro-6-iodo-4-oxo-3-phenyl-2-quinazolinyl)amino]oxy]acetate (CAS RN: 221657-82-5);
(3,4-Dihydro-6-iodo-4-oxo-3-phenyl-2-quinazolinyl)azanyl benzoate (CAS RN: 221658-09-9);
(3,4-Dihydro-6-iodo-4-oxo-3-phenyl-2-quinazolinyl)azanyl benzeneacetate (CAS RN: 221658-10-2);
1-[(3,4-Dihydro-6-iodo-4-oxo-3-phenyl-2-quinazolinyl)azanyl] 2-ethyl ethanedioate (CAS RN: 221657-99-4).

Regarding the excluded compounds listed above, see WO 1994/26722 and Abdel-Hamide et al. Acta Pharmaceutica (Zagreb) 1998, 48(4): 249-258.

In some embodiments of the compound of formula (Int-I) R¹⁰ is hydrogen.

In some embodiments of the compound of formula (Int-I) R¹⁰ is halogen (e.g. chloro, bromo, iodo).

In some embodiments of the compound of formula (Int-I) R¹⁰ is -B(OH)₂ or -B(-O-C(CH₃)₂-C(CH₃)₂-O-).

In some embodiments of the compound of formula (Int-I) R¹⁰ is -S(O)₂Cl.

In some embodiments of the compound of formula (Int-I) R¹⁰ is -S-CH₂-phenyl.

In some embodiments of the compound of formula (Int-I) X¹, X², X³, R⁴, R⁵ and R⁶ are as defined in Embodiment A.

In some embodiments of the compound of formula (Int-I) X¹, X², X³, R⁴, R⁵ and R⁶ are as defined in Embodiment B.

In some embodiments of the compound of formula (Int-I) X¹, X², X³, R⁴, R⁵ and R⁶ are as defined in Embodiment C.

In a further aspect the invention provides compounds of formula (Int-II) 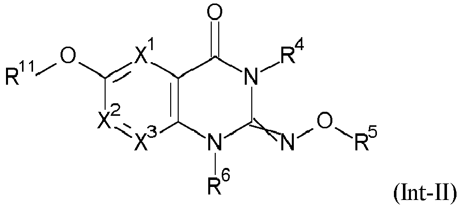 or a salt thereof, wherein
R¹¹ is hydrogen or C₁₋₈ alkyl; and
X¹, X², X³, R⁴, R⁵ and R⁶ are as defined for the compound of formula (I) including preferred definitions and embodiments thereof.

In some embodiments of the compound of formula (Int-II) X¹, X², X³, R⁴, R⁵ and R⁶ are as defined in Embodiment A.

In some embodiments of the compound of formula (Int-II) X¹, X², X³, R⁴, R⁵ and R⁶ are as defined in Embodiment B.

In some embodiments of the compound of formula (Int-II) X¹, X², X³, R⁴, R⁵ and R⁶ are as defined in Embodiment C.

The present invention relates also to pharmaceutical compositions that comprise a compound of formula (I) or a pharmaceutically acceptable salt thereof as active ingredient, which can be used especially in the treatment of neoplastic diseases, in particular cancer, as described herein.

The compounds of the invention may be formulated as pharmaceutical compositions for non-parenteral administration, such as nasal, buccal, rectal, pulmonary, vaginal, sublingual, topical, transdermal, ophthalmic, otic or, especially, for oral administration, e.g. in the form of oral solid dosage forms, e.g. granules, pellets, powders, tablets, film or sugar coated tablets, effervescent tablets, hard and soft gelatin or HPMC capsules, coated as applicable, orally disintegrating tablets, oral solutions, lipid emulsions or suspensions, or for parenteral administration, such as intravenous, intramuscular, or subcutaneous, intrathecal, intradermal or epidural administration, to mammals, especially humans, e.g. in the form of solutions, lipid emulsions or suspensions containing microparticles or nanoparticles. The compositions may comprise the active ingredient(s) alone or, preferably, together with a pharmaceutically acceptable excipient.

The pharmaceutical compositions can be processed with pharmaceutically inert, inorganic or organic excipients for the production of oral solid dosage forms, e.g. granules, pellets, powders, tablets, film or sugar coated tablets, effervescent tablets, hard gelatin or HPMC capsules or orally disintegrating tablets. Fillers e.g. lactose, cellulose, mannitol, sorbitol, calcium phosphate, starch or derivatives thereof, binders e.g. cellulose, starch, polyvinylpyrrolidone, or derivatives thereof, glidants e.g. talcum, stearic acid or its salts, flowing agents e.g. fumed silica, can be used as such excipients for formulating and manufacturing of oral solid dosage forms, such as granules, pellets, powders, tablets, film or sugar coated tablets, effervescent tablets, hard gelatin or HPMC capsules, or orally disintegrating tablets. Suitable excipients for soft gelatin capsules are e.g. vegetable oils, waxes, fats, semisolid and liquid polyols etc.

Suitable excipients for the manufacture of oral solutions, lipid emulsions or suspensions are e.g. water, alcohols, polyols, saccharose, invert sugar, glucose etc. Suitable excipients for parenteral formulations are e.g. water, alcohols, polyols, glycerol, vegetable oils, lecithin, surfactants etc. Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain other therapeutically valuable substances.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor^{®} EL or phosphate buffered saline (PBS). The carrier can be a solvent or dispersion medium containing, for example, water, ethanol, polyol (for example, glycerol, propylene glycol, and liquid polyethylene glycol, and the like), and suitable mixtures thereof. For intravenous injection of strongly lipophilic molecules it can be advantageous to include solubilizers in the formulation, for example surfactants, polymeric surfactants, polymers, complexing agents and/or cosolvents, which may significantly increase the solubility of the compounds in water. Examples of solubilizers include polyethylene glycol, propylene glycol, ethanol, glycerol and cyclodextrins.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In addition pharmaceutical compositions used in the invention optionally include buffers such as phosphate, citrate, or other organic acids; antioxidants including butylated hydroxytoluene (BHT), butylated hydroxyanisole (BHA), ascorbic acid; low molecular weight (less than about 10 residues) polypeptides; proteins, such as serum albumin, gelatin or immunoglobulins; hydrophilic polymers such as polyvinylpyrrolidone, amino acids such as glycine, glutamine, asparagines, arginine or lysine; monosaccharides, disaccharides, or other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, PLURONICS^{™}, or PEG.

Optionally, the pharmaceutical compositions contain a pharmaceutically acceptable preservative. In some embodiments the preservative concentration ranges from 0.1 to 2.0 percent, typically v/v. Suitable preservatives include those known in the pharmaceutical arts, such as benzyl alcohol, phenol, m-cresol, methylparaben, and propylparaben.

The dosage can vary within wide limits and will, of course, be fitted to the individual requirements in each particular case. In general, in the case of oral administration a daily dosage of about 1 to 1000 mg per person of a compound of general formula (I) should be appropriate, although the above lower or upper limit can also be exceeded when necessary.

Compounds of formula (I) according to the invention as described above or pharmaceutically acceptable salts thereof are particularly useful for the treatment of neoplastic diseases such as cancer, e.g. when administered in therapeutically effective amounts. Examples of neoplastic diseases include, but are not limited to, epithelial neoplasms, squamous cell neoplasms, basal cell neoplasms, transitional cell papillomas and carcinomas, adenomas and adenocarcinomas, adnexal and skin appendage neoplasms, mucoepidermoid neoplasms, cystic neoplasms, mucinous and serous neoplasms, ducal-, lobular and medullary neoplasms, acinar cell neoplasms, complex epithelial neoplasms, specialized gonadal neoplasms, paragangliomas and glomus tumours, naevi and melanomas, soft tissue tumours and sarcomas, fibromatous neoplasms, myxomatous neoplasms, lipomatous neoplasms, myomatous neoplasms, complex mixed and stromal neoplasms, fibroepithelial neoplasms, synovial-like neoplasms, mesothelial neoplasms, germ cell neoplasms, trophoblastic neoplasms, mesonephromas, blood vessel tumours, lymphatic vessel tumours, osseous and chondromatous neoplasms, giant cell tumours, miscellaneous bone tumours, odontogenic tumours, gliomas, neuroepitheliomatous and neuroendocrine neoplasms, meningiomas, nerve sheath tumours, granular cell tumours and alveolar soft part sarcomas, Hodgkin's and non-Hodgkin's lymphomas, B-cell lymphoma, T-cell lymphoma, hairy-cell lymphoma, Burkitts lymphoma and other lymphoreticular neoplasms, plasma cell tumours, mast cell tumours, immunoproliferative diseases, leukemias, miscellaneous myeloproliferative disorders, lymphoproliferative disorders and myelodysplastic syndromes.

Examples of cancers in terms of the organs and parts of the body affected include, but are not limited to, the breast, cervix, ovaries, colon, rectum (including colon and rectum i.e. colorectal cancer), lung (including small cell lung cancer, non-small cell lung cancer, large cell lung cancer and mesothelioma), endocrine system, bone, adrenal gland, thymus, liver, stomach (gastric cancer), intestine, pancreas, bone marrow, hematological malignancies (such as lymphoma, leukemia, myeloma or lymphoid malignancies), bladder, urinary tract, kidneys, skin, thyroid, brain, head, neck, prostate and testis. Preferably the cancer is selected from the group consisting of breast cancer, prostate cancer, cervical cancer, ovarian cancer, gastric cancer, colorectal cancer, pancreatic cancer, liver cancer, brain cancer, neuroendocrine cancer, lung cancer, kidney cancer, bladder cancer, mesothelioma, hematological malignancies, melanomas and sarcomas.

The cancer may be a primary tumor and/or metastases. The cancer may be derived from a solid or liquid (e.g. hematological or intraperitoneal) tumor. In some embodiments the neoplastic disease (e.g. cancer) to be treated is a tumor, e.g. a solid tumor.

In some embodiments, the cancer to be treated by the compounds of the present invention is mediated by modulation of PARG. In some embodiments the compounds of the invention may treat the cancer by modulation of PARG, e.g. by inhibiting PARG. Such cancers may be ovarian cancer, lung cancer or breast cancer, for example.

"Pharmaceutically acceptable" as used herein refers to items such as compounds and salts thereof, materials, compositions and/or dosage forms, which are, within the scope of sound medical judgment, suitable for contact with the tissues of a warm-blooded animal, e.g., a mammal or human, without excessive toxicity or other complications commensurate with a reasonable benefit/risk ratio.

"Pharmaceutical composition" is defined herein to refer to a solid or liquid formulation containing at least one therapeutic agent to be administered to a subject, e.g., a mammal, particularly a human, with one or more pharmaceutically acceptable excipients, in order to prevent or treat a particular disease or condition affecting the mammal.

"Prevent", "preventing" or "prevention" as used herein comprises the prevention of at least one symptom associated with or caused by the state, disease or disorder being prevented.

"Therapeutically-effective amount," as used herein, pertains to that amount of a compound, or a material, composition or dosage form comprising a compound, which is effective for producing some desired therapeutic effect, commensurate with a reasonable benefit/risk ratio, when administered in accordance with a desired treatment regimen.

"Treatment" or "treating" as used herein in the context of treating a disease or disorder, pertains generally to treatment and therapy, whether of a human or an animal (e.g., in veterinary applications), in which some desired therapeutic effect is achieved, for example, the inhibition of the progress of the disease or disorder, and includes a reduction in the rate of progress, a halt in the rate of progress, alleviation of symptoms of the disease or disorder, amelioration of the disease or disorder, and cure of the disease or disorder. Treatment as a prophylactic measure (i.e., prophylaxis) is also included. For example, use with patients who have not yet developed the disease or disorder, but who are at risk of developing the disease or disorder, is encompassed by the term "treatment." For example, treatment includes the prophylaxis of cancer, reducing the incidence of cancer, alleviating the symptoms of cancer, etc.

The term "subject" refers to a mammal and preferably refers to a human and the term "patient" refers to a human presenting themselves for therapeutic treatment.

The compounds of formula (I) can be synthesized by methods given below or by analogous methods, e.g. as shown in Scheme I. The scheme described herein is not intended to present an exhaustive list of methods for preparing the compounds of formula (I); rather, additional techniques of which the skilled chemist is aware may be also used for the compound synthesis.

It is understood by one skilled in the art of organic synthesis that optimum reaction conditions may vary with the particular reactants or solvents used, but such conditions can be determined by routine optimization procedures. In some cases, the order of performing the following reaction schemes, and/or reaction steps, may be varied to facilitate the reaction or to avoid the formation of unwanted side products. In addition, the functionality present at various positions of the molecule must be compatible with the reagents and reactions proposed. Such restrictions to the substituents, which are compatible with the reaction conditions, will be readily apparent to one skilled in the art and alternate methods must then be used. Furthermore in some of the reactions mentioned herein it may be necessary or desirable to protect any sensitive groups in compounds and it will be assumed that such protecting groups (PG) as necessary are in place. Conventional protecting groups may be used in accordance with standard practice, well known in the art (for illustration see Wuts P.G.M, Greene's Protective Groups in Organic Synthesis, 5th Edition, Publisher: John Wiley & Sons, 2014). The protecting groups may be removed at any convenient stage in the synthesis using conventional techniques well known in the art, or they may be removed during a later reaction step or work-up.

In the general sequence of reactions outlined below in Scheme 1, the abbreviations X¹, X², X³ and the generic groups, R¹, R², R³, R⁴, R⁵ and R⁶ are as defined for formula (I), unless otherwise specified. The generic group E1 is generally a hydrogen atom, a -OH, a methoxy group, a halogen atom, a sulfonic acid, a sulfonyl chloride, a boronic acid, a boronic ester, or -O-L1 and -O-L1 is a leaving group in which L1 is selected from a perfluoroalkylsulfonyl such as triflyl (trifluoromethanesulfonyl) and a sulfonyl such as tosyl (*p*-toluenesulfonyl) or mesyl (methanesulfonyl).

The compounds according to the present invention, pharmaceutically acceptable salts, solvates, and hydrates thereof can be prepared according to the general sequence of reactions outlined below in Scheme 1, followed, if necessary, by:
- manipulation of substituents to give a new final product. These manipulations may include, but are not limited to, reduction, oxidation, alkylation, acylation, substitution, coupling including transition-metal catalyst coupling and hydrolysis reactions which are commonly known by those skilled in the art;
- removing any protecting groups;
- forming a pharmaceutically acceptable salt; or
- forming a pharmaceutically acceptable solvate or hydrate.

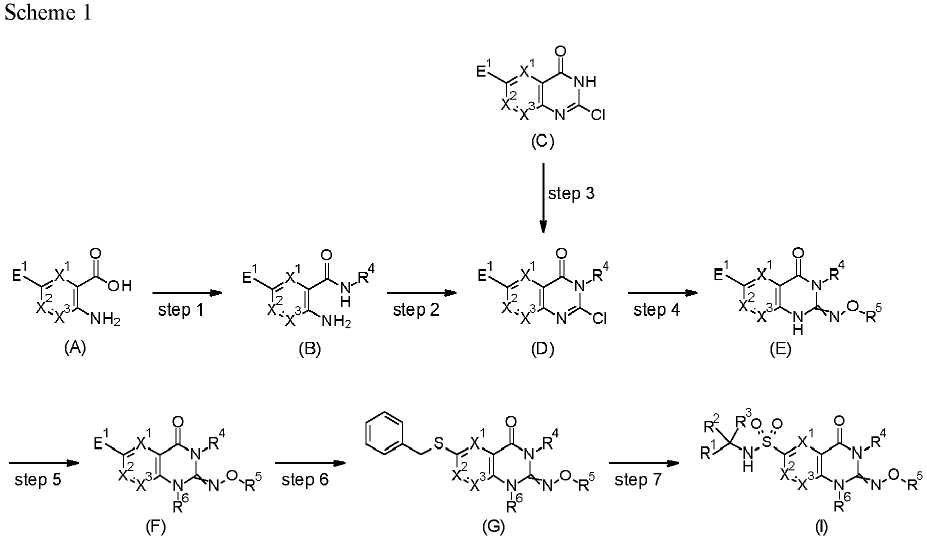

The necessary starting materials for the synthetic methods as described herein, if not commercially available, may be made by procedures which are described in the scientific literature or may be made from commercially available compounds using adaptations of processes reported in the scientific literature. The reader is further referred to e.g. March J., Smith M., Advanced Organic Chemistry, 7th Edition, Publisher: John Wiley & Sons, 2013 for general guidance on reaction conditions and reagents.

Compounds of formulae (A) to (F) wherein E1 is an -O-L1 group can be prepared by reacting the corresponding alcohols (A) to (F), wherein E1 is -OH, with methanesulfonyl chloride or methanesulfonic anhydride, *p*-toluenesulfonyl chloride, trifluoromethanesulfonyl chloride or trifluoromethanesulfonic anhydride, respectively, in presence of a base such as triethylamine or the like in a dry aprotic solvent such as pyridine, acetonitrile, tetrahydrofuran or dichloromethane between -30°C and 80°C.

Compounds of formulae (A) to (F) wherein E1 is a halogen atom can be prepared from compounds of formulae (A) to (F) wherein E1 is an -O-L1 group *via* a transition-metal catalyst coupling reaction. Typical catalysts include palladium(II) acetate, tris(dibenzylideneacetone)dipalladium(0) or the like. The reaction is typically run at a temperature from 0°C to 150°C, more frequently from 80°C to 120°C.

Usually the reaction is performed in the presence of a ligand such as di-*tert*-butyl-[3,6-dimethoxy-2-(2,4,6-triisopropylphenyl)phenyl]phosphane, di-*tert*-butyl-[2,3,4,5-tetramethyl-6-(2,4,6-triisopropylphenyl)phenyl]phosphane, 2-(dicyclohexylphosphino)biphenyl, 4,5-bis(diphenylphosphino)-9,9-dimethylxanthene or the like and a base such as sodium *tert*-butylate, cesium carbonate, potassium carbonate, more frequently cesium carbonate in a large variety of inert solvents such as toluene, tetrahydrofuran, dioxane, 1,2-dichloroethane, *N,N*-dimethylformamide, dimethylsulfoxide, water and acetonitrile, or a mixture of solvents, more frequently in dioxane. As there are numerous components in transition-metal catalyst coupling reactions such as the particular palladium catalyst, the ligand, additives, solvent, temperature, numerous protocols have been identified. One skilled in the art will be able to identify a satisfactory protocol without undue experimentation.

Alternatively, compounds of formulae (A) to (F) wherein E1 is a halogen atom can be prepared from compounds of formulae (A) to (F) wherein E1 is a boronic acid or a boronic ester *via* a halogenodeboronation reaction. The reaction is typically performed in presence of copper(II) halides in methanol at a temperature ranging from 0°C to 100°C, more frequently at 90°C.

Compounds of formulae (C) to (F) wherein E1 is a sulfonic acid can be prepared from compounds of formulae (C) to (F) wherein E1 is a hydrogen atom *via* sulfonation reaction. The reaction is typically performed in presence of concentrated sulfuric acid, fuming sulfuric acid, sulfur trioxide or chlorosulfuric acid.

Compounds of formulae (C) to (F) wherein E1 is a sulfonyl chloride can be prepared from compounds of formulae (C) to (F) wherein E1 is a sulfonic acid using chlorosulfuric acid or sulfonyl chloride.

Alternatively, compounds of formulae (C) to (F) wherein E1 is a sulfonyl chloride can be prepared from compounds of formulae (C) to (F) wherein E1 is a hydrogen atom *via* halosulfonation using chlorosulfuric acid.

Additionally, compounds of formula (I) wherein X³ is C-R⁹ with R⁹ being a C₃₋₆ cycloalkyl, a phenyl or a heteroaryl group can be prepared from compounds of formula (I) wherein X³ is C-R⁹ with R⁹ being a halogen atom that react with a boronic acid or a boronic ester via a Suzuki reaction. The Suzuki reaction is a palladium-catalyzed cross coupling between organoboronic acids and aryl or vinyl halides or triflates. Typical catalysts include palladium(II) acetate, tetrakis(triphenylphosphine)palladium(0), bis(triphenylphosphine)palladium(II) dichloride and
[1,1'bis(diphenylphosphino)ferrocene]dichloropalladium(II). The reaction can be carried out in a variety of organic solvents including toluene, tetrahydrofuran, dioxane, 1,2-dichloroethane, *N,N-*dimethylformamide, dimethylsulfoxide and acetonitrile, aqueous solvents and under biphasic conditions.

Reactions are typically run from room temperature to 150°C. Additives such as cesium fluoride, potassium fluoride, potassium hydroxide or sodium ethylate frequently accelerate the coupling. Potassium trifluoroborates and organoboranes or boronate esters may be used in place of boronic acids. Although there are numerous components in the Suzuki reaction such as the particular palladium catalyst, the ligand, additives, solvent, temperature, numerous protocols have been identified. One skilled in the art will be able to identify a satisfactory protocol without undue experimentation.

Compounds of formula (I) wherein X³ is C-R⁹ with R⁹ being a heterocyclyl system can be prepared from compounds of formula (I) wherein X³ is C-R⁹ with R⁹ being a halogen atom that react with a heterocyclyl group via a transition-metal catalyst coupling reaction, using conditions previously described.

### Step 1:

Compounds of formula (B) can be prepared from compounds of formula (A) and an amine *via* a coupling reaction in the presence of an activating agent such as *N,N*'-dicyclohexylcarbodiimide or *N-*(3-dimethylaminopropyl)-*N*'-ethylcarbodiimide hydrochloride, with the optional addition of 1-hydroxybenzotriazole. Other suitable coupling agents may be used such as *O*-(7-azabenzotriazol-1-yl)-*N,N,N',N'*-tetramethyluronium hexafluorophosphate, 2-ethoxy-1-ethoxycarbonyl-1,2-dihydroquinoline, carbonyldiimidazole or diethylphosphorylcyanide. Optionally, a base like triethylamine, *N,N-*diisopropylethylamine or pyridine can be added to perform the coupling. The amide coupling is conducted at a temperature between -20°C and 100°C, in an inert solvent, preferably a dry aprotic solvent like dichloromethane, acetonitrile, *N,N*-dimethylformamide and chloroform.

### Step 2:

Compounds of formula (D) can be prepared from compounds of formula (B) via cyclization using thiophosgene in 1,4-dioxane under refluxing conditions (Pave G., Org. Lett., 2015, 17, 4930-4932).

### Step 3:

Alternatively, compounds of formula (D) can be prepared from compounds of formula (C) and a halide, a mesylate, tosylate or triflate *via* a substitution reaction. Substitution reaction is generally performed at a temperature between -20°C and 100°C in a dry aprotic solvent like dichloromethane, acetonitrile, *N.N-*dimethylformamide, dimethyl sulfoxide or tetrahydrofuran without or with an inorganic base such as potassium carbonate or cesium carbonate, or an organic base such as trimethylamine, pyridine or *N,N-*diisopropy lethylamine.

### Step 4:

Compounds of formula (E) can be prepared from compounds of formula (D) and a *O*-substituted hydroxylamine *via* condensation reaction. The reaction is performed at a temperature between room temperature and 150°C in a solvent like *N,N*-dimethylformamide or *N,N*-dimethylacetamide with or without an organic base such as sodium acetate, triethylamine, pyridine.

### Step 5:

Compounds of formula (F) can be prepared from compounds of formula (E) and a halide, a mesylate, tosylate or triflate *via* a substitution reaction, using conditions previously described.

### Step 6:

Compounds of formula (G) can be prepared from compounds of formula (F) wherein E1 is a halogen atom or wherein E1 is an -O-L1 group *via* a transition-metal catalyst coupling reaction, using benzyl mercaptan and conditions previously described.

### Step 7:

Compounds of formula (I) can be prepared from compounds of formula (G) *via* oxidative chlorination using NCS or DCDMH under acidic conditions followed by substitution of the generated sulfonyl chloride with an amine, using conditions previously described.

Alternatively, compounds of formula (I) can be directly prepared from compounds of formula (F) wherein E1 is a sulfonyl chloride via substitution with an amine, using conditions previously described.

Oximes of compounds of formulae (I), (E), (F) and (G) can be formed as a mixture of the two isomeric forms (E and Z) or only one isomer can be formed. In case two isomers are formed, they could be separated at any convenient stage.

Particular embodiments of the invention are described in the following Examples, which serve to illustrate the invention in more detail and should not be construed as limiting the invention in any way.

### Description of the Figures

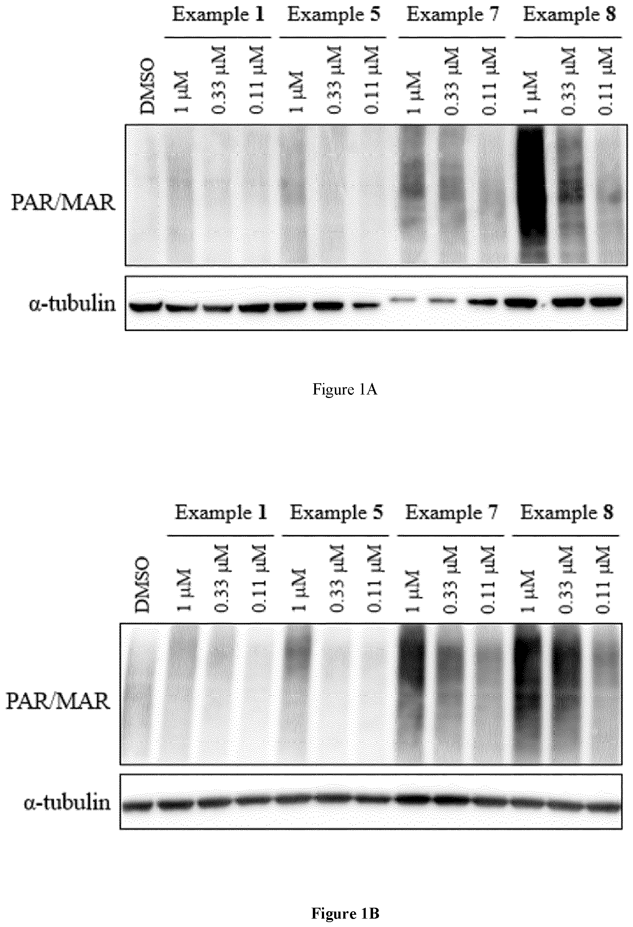

Figure 1 shows the increase in cellular protein-bound PAR levels following PARG inhibitor treatment. A: Kuramochi HGSOC cells were treated for 8 hours either with the compound vehicle DMSO, or with increasing concentrations of Examples 1, 5, 7 or 8, followed by cell extraction and detection of cellular PAR levels by Western blotting. α-tubulin was blotted for as a loading control. B: NCI-H1650 NSCLC cells were treated for 8 hours either with the compound vehicle DMSO, or with increasing concentrations of Examples 1, 5, 7 or 8, followed by Western blotting as described above.

### Examples

All reagents and solvents are generally used as received from the commercial supplier;
reactions are routinely performed with anhydrous solvents in well-dried glassware under an argon or nitrogen atmosphere, unless otherwise specified;
evaporations are carried out by rotary evaporation under reduced pressure and work-up procedures are carried out after removal of residual solids by filtration;
all temperatures are given in degree Celsius (°C) and are approximate temperatures; unless otherwise noted, operations are carried out at room temperature (rt), that is typically in the range 18°C - 25°C;
column chromatography (by the flash procedure) is used to purify compounds;
classical flash chromatography is often replaced by automated systems. This does not change the separation process per se. A person skilled in the art will be able to replace a classical flash chromatography process by an automated one, and vice versa. Typical automated systems can be used, as they are provided by Büchi, Biotage or Isco (combiflash) for instance;
reaction mixture can often be separated by preparative HPLC using e.g. water and acetonitrile as system of eluents, unless otherwise stated. A person skilled in the art will find suitable conditions for each separation; the compounds are isolated after purification as a parent compound or in a form of the corresponding trifluoroacetic acid (TFA) salt or the respective formic acid salt;
reactions, which required higher temperature, are usually performed using classical heating instruments; but can also be performed using microwave apparatus (CEM Explorer) at a power of 250 W, unless otherwise noted;
hydrogenation or hydrogenolysis reactions can be performed using hydrogen gas in balloon or using Parr-apparatus system or other suitable hydrogenation equipment;
concentration of solutions and drying of solids are performed under reduced pressure unless otherwise stated;
in general, the course of reactions is followed by TLC, HPLC, or LC/MS and reaction times are given for illustration only; yields are given for illustration only and are not necessarily the maximum attainable; the structure and purity of the final products of the invention are generally confirmed by NMR spectroscopy, HPLC and mass spectral techniques.

Proton NMR spectra are recorded on a Brucker 400 MHz spectrometer. Chemical shifts (δ) are reported in ppm relative to Me₄Si or the solvent peak as internal standard, and NMR coupling constants (J values) are in Hertz (Hz). Each peak is denoted as a broad singlet (br), singlet (s), doublet (d), triplet (t), quadruplet (q), doublet of doublets (dd), triplet of doublets (td) or multiplet (m).

HPLC of the final products are generated using a Dionex Ultimate 3000^{™} instrument coupled with Dionex MSQ ESI mode and the following conditions:

| | | |
|---|---|---|
| Mobile Phase A: | Water with 0.1% Formic acid | |
| Mobile Phase B: | Acetonitrile with 0.1% Formic acid | |
| Column: | YMC triart C18 5 µm 100 mm x 4.6 mm | |
| Column Temperature: | 25°C | |
| Detection: | UV 250 nm | |
| Injection: | 2 µL of 10 mM sample DMSO solution | |
| Flow: | 1.6 mL/min | |
| Gradient | Time (min) | % Mobile Phase B |
| | 0 | 5 |
| | 8 | 95 |
| 10 | 95 | |
| 10.1 | 5 | equilibration |
| 13 | 5 | equilibration |

Mass spectra are generated using a q-Tof Ultima^{™} (Waters AG or Thermo Scientific MSQ Plus) mass spectrometer in the positive or negative ESI mode. The system is equipped with the standard Lockspray interface;
each intermediate is purified to the standard required for the subsequent stage and is characterized in sufficient detail to confirm that the assigned structure is correct;
analytical and preparative HPLC on non-chiral phases are performed using RP-C18 based columns;
the following abbreviations may be used (reference can also be made to The *Journal of Organic Chemistry* Guidelines for Authors, for a comprehensive list of standard abbreviations and acronyms):
   - AcOH: Acetic acid
   - ACN: Acetonitrile
   - Bn: Benzyl
   - BnSH: Benzyl mercaptan
   - CAS: compound having Chemical Abstracts Services registry number
   - Cbz: *N*-Carboxybenzyl
   - CDCl₃: Deuterated chloroform
   - DCDMH: 1,3-Dichloro-5,5-dimethylhydantoin
   - DCM: Dichloromethane
   - Diox: 1,4-Dioxane
   - DIPEA: *N*,*N*-Diisopropylethylamine
   - DMA: *N*,*N*-Dimethylacetamide
   - DME: Dimethoxyethane
   - DMF: *N*,*N*-Dimethylformamide
   - DMSO: Dimethyl sulfoxide
   - DMSO-*d₆*: Deuterated dimethyl sulfoxide
   - EA: Ethyl acetate
   - EDCI: 1-(3-Dimethylaminopropyl)-3-ethylcarbodiimide hydrochloride
   - ELSD: Evaporative light scattering detection
   - ESI: Electrospray ionization
   - EtOH: Ethanol
   - Ex.: Example
   - HATU: 1-[Bis(dimethylamino)methylene]-1*H*-1,2,3-triazolo[4,5-b]pyridinium 3-oxide hexafluorophosphate
   - HOBt: *N*-Hydroxybenzotriazole

   - HPLC: High performance liquid chromatography
   - LC/MS: Liquid chromatography coupled to mass spectroscopy
   - MeOH: Methanol
   - Me₄Si: Tetramethylsilane
   - MS: Mass spectrometry
   - MW: Microwave
   - NCS: N-Chlorosuccinimide
   - NMR: Nuclear magnetic resonance
   - Pd/C: Palladium on activated carbon
   - Pd₂(dba)₃: Tris(dibenzylideneacetone)dipalladium(0)
   - Pd(dppf)Cl₂: [1,1-Bis(diphenylphosphino)ferrocene]dichloropalladium(II)
   - Pd(PPh₃)₄: Tetrakis(triphenlyphosphine)-palladium(0)
   - PE: Petroleum ether
   - rt: Room temperature
   - TEA: Triethylamine
   - TFA: Trifluoroacetic acid
   - THF: Tetrahydrofuran
   - Xant-Phos: 4,5-Bis(diphenylphosphino)-9,9-dimethylxanthene

The following Examples refer to the compounds of formula (I) as indicated in Table 1.

The Examples listed in the following table can be prepared using procedures described above, and detailed synthesis methodology is described in detail below. The Example numbers used in the leftmost column are used in the application text for identifying the respective compounds.

**Table 1. Exemplified compounds**

| Ex. | Formula | Reference for Preparation | **¹H-NMR** (400 MHz) **δ** ppm | HPLC Retention time (min) | **MS m/z** (+ESI) |
|---|---|---|---|---|---|
| 1 | 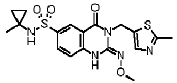 | - | DMSO-*d₆*+D₂O: 8.19 (d, J= 2.0 Hz, 1H), 7.86 (dd, *J* = 2.0, 8.8 Hz, 1H), 7.65 (s, 1H), 7.49 (d, *J=* 8.8 Hz, 1H), 5.11 (s, 2H), 3.83 (s, 3H), 2.57 (s, 3H), 1.03 (s, 3H), 0.57 (m, 2H), 0.35 (m, 2H) | 5.42 | 436.2 [M+H]⁺ |
| 2 | 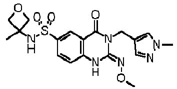 | Example 1 using 735241-98-2, 593-56-6 and 874473-14-0 | DMSO-*d₆*+D₂O: 8.21 (d, *J* = 2.4 Hz, 1H), 7.87 (dd, *J* = 2.4, 8.8 Hz, 1H), 7.67 (s, 1H), 7.48 (d, *J* = 8.8 Hz, 1H), 7.42 (s, 1H), 4.81 (s, 2H), 4.49 (d, J= 6.0 Hz, 2H), 4.09 (d, J= 6.0 Hz, 2H), 3.81 (s, 3H), 3.75 (s, 3H), 1.38 (s, 3H) | 4.31 | 435.3 [M+H]⁺ |
| 3 | 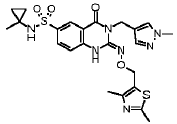 | - | DMSO-*d₆*+D₂O: 8.18 (d, J = 2.4 Hz, 1H), 7.84 (dd, *J* = 2.4, 8.8 Hz, 1H), 7.47 (m, 2H), 7.32 (s, 1H), 5.13 (s, 2H), 4.80 (s, 2H), 3.72 (s, 3H), 2.56 (s, 3H), 2.35 (s, 3H), 1.02 (s, 3H), 0.56 (m, 2H), 0.35 (m, 2H) | 5.04 | 530.4 [M+H]⁺ |
| 4 | 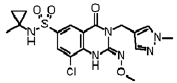 | Example 1 using 62484-39-3, 735241-98-2, 593-56-6 and 88887-87-0 | DMSO-*d₆*: 8.45 (s, 1H), 8.20 (s, 1H), 8.18 (d, *J* = 1.6 Hz, 1H), 8.02 (d, *J* = 2.0 Hz, 1H), 7.70 (s, 1H), 7.44 (s, 1H), 4.83 (s, 2H), 3.87 (s, 3H), 3.77 (s, 3H), 1.08 (s, 3H), 0.60 (m, 2H), 0.41 (m, 2H) | 6.17 | 453.2, 454.9 [M+H]⁺ |
| 5 | 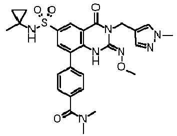 | - | DMSO-*d₆*+D₂O: 8.28 (d, *J* = 2.0 Hz, 1H), 7.83 (d, J= 2.0 Hz, 1H), 7.69 (s, 1H), 7.62 (m, 4H), 7.43 (s, 1H), 4.82 (s, 2H), 3.75 (s, 3H), 3.69 (s, 3H), 3.01 (s, 3H), 2.94 (s, 3H), 1.08 (s, 3H), 0.62 (m, 2H), 0.40 (m, 2H) | 5.46 | 566.4 [M+H]⁺ |
| 6 | 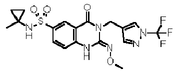 | Example 1 using 2169440-61-1, 593-56-6 | DMSO-*d₆*+D₂O: 8.38 (s, 1H), 8.20 *(d, J=* 1.6 Hz, 1H), 7.95 (s, 1H), 7.86 (dd, *J* = 2.0, 8.0 Hz, 1H), 7.50 (d, *J* = 8.4 Hz, 1H), 4.90 (s, 2H), 3.78 (s, | 6.40 | 473.2 [M+H]⁺ |
| | | and 88887-87-0 | 3H), 1.04 (s, 3H), 0.57 (m, 2H), 0.36 (m, 2H) | | |
| 7 | 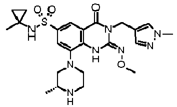 | - | DMSO-*d₆*+D₂O: 8.01 *(d, J=* 2.0 Hz, 1H), 7.69 (d, *J* = 2.0 Hz, 1H), 7.67 (s, 1H), 7.42 (s, 1H), 4.81 (s, 2H), 3.83 (s, 3H), 3.74 (s, 3H), 3.19 (m, 2H), 3.01 (m, 3H), 2.81 (m, 1H), 2.62 (m, 1H), 1.16 (d, *J* = 6.4 Hz, 3H), 1.01 (s, 3H), 0.57 (m, 2H), 0.37 (m, 2H) | 3.83 | 517.4 [M+H]⁺ |
| 8 | 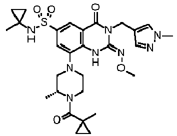 | - | DMSO-*d₆*+D₂O: 8.03 (d*, J* = 2.0 Hz, 1H), 7.76 (d, *J* = 2.0 Hz, 1H), 7.69 (s, 1H), 7.43 (s, 1H), 4.82 (s, 2H), 4.61 (m, 1H), 4.26 (m, 1H), 3.82 (s, 3H), 3.76 (s, 3H), 2.96 (m, 3H), 2.67 (m, 2H), 1.41 (br, 3H), 1.25 (s, 3H), 1.01 (s, 3H), 0.83 (m, 2H), 0.57 (m, 4H), 0.37 (m, 2H) | 5.96 | 519.5 [M+H]⁺ |
| 9 | 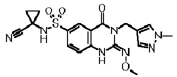 | Example 1 using 735241-98-2, 593-56-6 and 127946-77-4 | DMSO-*d₆*: 10.82 (s, 1H), 9.08 (s, 1H), 8.26 (d, *J* = 2.0 Hz, 1H), 7.91 (dd, J= 2.4, 8.8 Hz, 1H), 7.69 (s, 1H), 7.54 (d, *J=* 8.8 Hz, 1H), 7.43 (s, 1H), 4.83 (s, 2H), 3.84 (s, 3H), 3.77 (s, 3H), 1.40 (m, 2H), 1.25 (m, 2H) | 4.70 | 430.3 [M+H]⁺ |
| 10 | 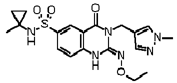 | Example 1 using 735241-98-2, 3332-29-4 and 88887-87-0 | DMSO-*d₆*: 10.60 (s, 1H), 8.21 (d, *J=* 2.0 Hz, 1H), 8.01 (s, 1H), 7.85 (dd, *J* = 2.4, 8.8 Hz, 1H), 7.67 (s, 1H), 7.52 (d, *J=* 8.8 Hz, 1H), 7.42 (s, 1H), 4.84 (s, 2H), 4.07 (q*, J* = 6.8 Hz, 2H), 3.76 (s, 3H), 1.29 (t, *J=* 6.8 Hz, 3H), 1.05 | 5.51 | 433.3 [M+H]⁺ |
| | | | (s, 3H), 0.58 (m, 2H), 0.36 (m, 2H) | | |
| 11 | 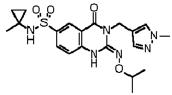 | Example 3 using 735241-98-2, 4490-81-7 and 88887-87-0 | DMSO-*d₆*: 10.37 (s, 1H), 8.21 (d, *J=* 2.0 Hz, 1H), 8.00 (s, 1H), 7.85 (dd, *J* = 2.4, 8.8 Hz, 1H), 7.65 (s, 1H), 7.55 (d, *J=* 8.8 Hz, 1H), 7.41 (s, 1H), 4.85 (s, 2H), 4.25 (m, 1H), 3.76 (s, 3H), 1.28 (d, *J* = 6.0 Hz, 6H), 1.05 (s, 3H), 0.58 (m, 2H), 0.36 (m, 2H) | 5.95 | 447.3 [M+H]⁺ |

A hyphen in the third column indicates that the synthesis procedure is described below.

### Preparation of Example 1: 2-methoxyimino-N-(1-methylcyclopropyl)-3-[(2-methylthiazol-5-yl)methyl]-4-oxo-1H-quinazoline-6-sulfoonamide:

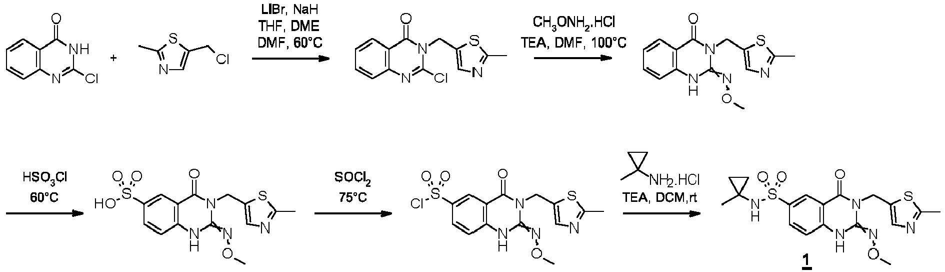

### Step 1: Preparation of 2-chloro-3-[(2-methylthiazol-5-yl)methyl]quinazolin-4-one:

NaH (0.76 g, 19.1 mmol, 60% dispersion in mineral oil) was added at 0°C to a stirred solution of 5-(chloromethyl)-2-methyl-thiazole (2.70 g, 17.4 mmol) in DME (50 mL) and DMF (10 mL), followed by LiBr (3.05 g, 34.7 mmol) and 2-chloro-3*H*-quinazolin-4-one (3.3 g, 17.4 mmol). After 16 h stirring at 60°C, the reaction mixture was extracted with EA and H₂O. The combined organic layers were dried over Na₂SO₄, filtered, concentrated and purified by column chromatography (silica gel; PE:EA; 7:3; v:v) to afford 2-chloro-3-[(2-methylthiazol-5-yl)methyl]quinazolin-4-one as an off-white solid (3.8 g, 71% yield).

MS m/z (+ESI): 292.1, 294.1 [M+H]⁺.

### Step 2: Preparation of 2-methoxyimino-3-[(2-methylthiazol-5-yl)methyl]-1H-quinazolin-4-one:

Methoxyamine hydrochloride (0.7 g, 8.14 mmol) was added to a stirred solution of 2-chloro-3-[(2-methylthiazol-5-yl)methyl]quinazolin-4-one (0.5 g, 1.63 mmol) in DMF (5 mL), followed by TEA (1.0 g, 9.77 mmol). After 2 h stirring at 100°C, the reaction mixture was poured into H₂O (50 mL) and the resulting precipitate was collected by filtration to afford 2-methoxyimino-3-[(2-methylthiazol-5-yl)methyl]-1*H*-quinazolin-4-one as a white solid (0.4 g, 77% yield).

MS m/z (+ESI): 303.1 [M+H]⁺.

### Step 3: Preparation of 2-methoxyimino-3-[(2-methylthiazol-5-yl)methyl]-4-oxo-1H-quinazoline-6-sulfonic acid:

A solution of 2-methoxyimino-3-[(2-methylthiazol-5-yl)methyl]-1*H*-quinazolin-4-one (0.35 g, 1.10 mmol) in chlorosulfonic acid (1 mL) was stirred at 60°C for 1 h. The reaction mixture was then poured into ice and purified by Biotage combiflash to afford 2-methoxyimino-3-[(2-methylthiazol-5-yl)methyl]-4-oxo-1*H*-quinazoline-6-sulfonic acid as a light yellow solid (0.42 g, 90% yield).

MS m/z (+ESI): 333.1 [M+H]⁺.

### Step 4: Preparation of 2-methoxyimino-3-[(2-methylthiazol-5-yl)methyl]-4-oxo-1H-quinazoline-6-sulfonyl chloride:

A solution of 2-methoxyimino-3-[(2-methylthiazol-5-yl)methyl]-4-oxo-1*H*-quinazoline-6-sulfonic acid (0.42 g, 0.99 mmol) in SOCh (4 mL) was stirred at 75°C for 2 h. The reaction mixture was then concentrated and purified by preparative HPLC to afford 2-methoxyimino-3-[(2-methylthiazol-5-yl)methyl]-4-oxo-1*H*-quinazoline-6-sulfonyl chloride as a light yellow solid (0.21 g, 48% yield).

MS m/z (+ESI): 401.0, 403.0 [M+H]⁺.

### Step 5: Preparation of 2-methoxyimino-N-(1-methylcyclopropyl)-3-[(2-methylthiazol-5-yl)methyl]-4-oxo-1H-quinazoline-6-sulfonamide:

1-Methylcycloproanamine hydrochloride (0.5 g, 4.49 mmol) was added to a stirred solution of 2-methoxyimino-3-[(2-methylthiazol-5-yl)methyl]-4-oxo-1*H*-quinazoline-6-sulfonyl chloride (0.2 g, 0.45 mmol) in DCM (10 mL), followed by TEA (1.92 mL, 13.5 mmol). After 1 h stirring, the reaction mixture was concentrated and purified by preparative HPLC to afford 2-methoxyimino-*N*-(1-methylcyclopropyl)-3-[(2-methylthiazol-5-yl)methyl]-4-oxo-1*H*-quinazoline-6-sulfonamide as a white solid (35 mg, 17% yield).

¹H NMR (400 MHz, DMSO-*d₆*+D₂O) δ ppm: 8.19 (d, *J* = 2.0 Hz, 1H), 7.86 (dd, J= 2.0, 8.8 Hz, 1H), 7.65 (s, 1H), 7.49 (d, *J* = 8.8 Hz, 1H), 5.11 (s, 2H), 3.83 (s, 3H), 2.57 (s, 3H), 1.03 (s, 3H), 0.57 (m, 2H), 0.35 (m, 2H).

MS m/z (+ESI): 436.5 [M+H]⁺.

### Preparation of Example 3: 2-[(2,4-dimethylthiazol-5-yl)methoxyimino]-N-(1-methylcyclopropyl)-3-[(1-methylpyrazol-4-yl)methyl]-4-oxo-1H-quinazoline-6-sulfonamide:

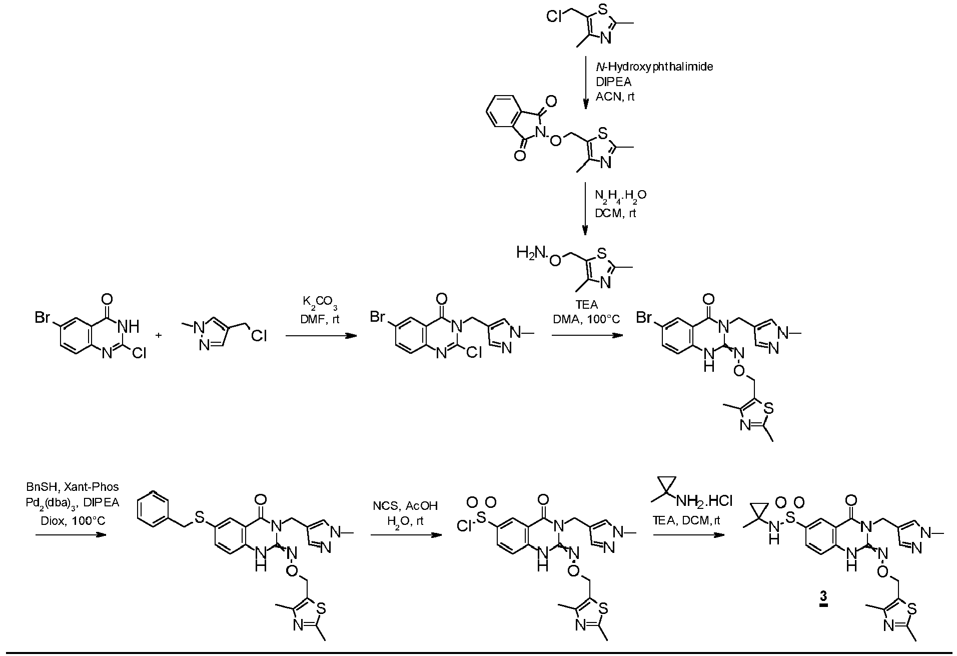

### Step 1: Preparation of 2-[(2,4-dimethylthiazol-5-yl)methoxy]isoindoline-1,3-dione:

*N*-Hydroxyphthalimide (93 mg, 0.56 mmol) was added to a stirred solution of 5-(chloromethyl)-2,4-dimethyl-thiazole (100 mg, 0.56 mmol) in ACN (5 mL), followed by DIPEA (202 µL, 1.11 mmol). After 16 h stirring, the reaction mixture was concentrated and purified by column chromatography (silica gel; PE:EA; 2:1; v:v) to afford 2-[(2,4-dimethylthiazol-5-yl)methoxy]isoindoline-1,3-dione as a white solid (140 mg, 83% yield).

MS m/z (+ESI): 289.1 [M+H]⁺.

### Step 2: Preparation of O-[(2,4-dimethylthiazol-5-yl)methyl]hydroxylamine:

Hydrazine hydrate (0.93 g, 18.3 mmol) was added to a stirred solution of 2-[(2,4-dimethylthiazol-5-yl)methoxy]isoindoline-1,3-dione (3.7 g, 12.2 mmol) in DCM (100 mL). After 4 h stirring, the reaction mixture was filtered and the filtrate was concentrated to afford *O*-[(2,4-dimethylthiazol-5-yl)methyl]hydroxylamine as a yellow oil (1.9 g, 89% yield) which was used in the next step without further purification.

MS m/z (+ESI): 159.1 [M+H]⁺.

### Step 3: Preparation of 6-bromo-2-chloro-3-[(1-methylpyrazol-4-yl)methyl]quinazolin-4-one:

4-(Chloromethyl)-1-methyl-pyrazole (3.42 g, 23.6 mmol) was added to a stirred solution of 6-bromo-2-chloro-*3H*-quinazolin-4-one (4.3 g, 12.4 mmol) in DMF (40 mL), followed by K₂CO₃ (5.25 g, 37.3 mmol). After 16 h stirring, the reaction mixture was diluted with H₂O (150 mL) and the resulting suspension was filtered. The cake was washed with H₂O and dried under high vacuum to give a yellow solid that was suspended in PE:EA (60 mL, 2:1, v:v). The resulting suspension was sonicated for 30 minutes and filtered. The cake was dried under high vacuum to afford 6-bromo-2-chloro-3-[(1-methylpyrazol-4-yl)methyl]quinazolin-4-one as a yellow solid (2.5 g, 51% yield).

¹H NMR (400 MHz, DMSO-*d₆*) δ ppm: 8.21 (d, *J* = 2.4 Hz, 1H), 8.01 (dd, *J* = 2.4, 8.4 Hz, 1H), 7.76 (s, 1H), 7.58 (d, *J=* 8.4 Hz, 1H), 7.48 (s, 1H), 5.21 (s, 2H), 3.77 (s, 3H).

MS m/z (+ESI): 353.0, 355.0 [M+H]⁺.

### Step 4: Preparation of 6-bromo-2-[(2,4-dimethylthiazol-5-yl)methoxyimino]-3-[(1-methylpyrazol-4-yl)methyl]-1H-quinazolin-4-one:

*O*-[(2,4-dimethylthiazol-5-yl)methyl]hydroxylamine (180 mg, 1.02 mmol) was added to a stirred solution of 6-bromo-2-chloro-3-[(1-methylpyrazol-4-yl)methyl]quinazolin-4-one (200 mg, 0.51 mmol) in DMA (5 mL), followed by TEA (290 µL, 2.04 mmol). After 3 h stirring at 100°C, the reaction mixture was diluted with H₂O (50 mL) and the resulting suspension was filtered. The cake was washed with H₂O and dried under high vacuum to afford 6-bromo-2-[(2,4-dimethylthiazol-5-yl)methoxyimino]-3-[(1-methylpyrazol-4-yl)methyl]-1*H*-quinazolin-4-one as a yellow solid (220 mg, 82% yield) which was used in the next step without further purification.

MS m/z (+ESI): 475.0, 477.0 [M+H]⁺.

### Step 5: Preparation of 6-benzylsulfanyl-2-[(2,4-dimethylthiazol-5-yl)methoxyimino]-3-[(1-methylpyrazol-4-yl)methyl]-1H-quinazolin-4-one:

Benzyl mercaptan (221 µL, 1.84 mmol) was added to a stirred suspension of 6-bromo-2-[(2,4-dimethylthiazol-5-yl)methoxyimino]-3-[(1-methylpyrazol-4-yl)methyl]-1*H*-quinazolin-4-one (650 mg, 1.23 mmol) in Diox (30 mL), followed by Xant-Phos (145 mg, 0.24 mmol), Pd₂(dba)₃ (115 mg, 0.12 mmol) and DIPEA (415 µL, 2.46 mmol). After 2 h stirring at 100°C, the reaction mixture was concentrated and purified by column chromatography (silica gel; PE:EA; 1:1; v:v) to afford 6-benzylsulfanyl-2-[(2,4-dimethylthiazol-5-yl)methoxyimino]-3-[(1-methylpyrazol-4-yl)methyl]-1*H-*quinazolin-4-one as a yellow solid (530 mg, 75% yield).

¹H NMR (400 MHz, DMSO-*d₆*) δ ppm: 10.35 (s, 1H), 7.70 (d, *J=* 2.0 Hz, 1H), 7.48 (dd, *J* = 2.0, 8.4 Hz, 1H), 7.45 (s, 1H), 7.30 (m, 2H), 7.25 (m, 4H), 7.21 (m, 1H), 5.11 (s, 2H), 4.78 (s, 2H), 4.15 (s, 2H), 3.72 (s, 3H), 2.56 (s, 3H), 2.35 (s, 3H).

MS m/z (+ESI): 519.2 [M+H]⁺.

### Step 6: Preparation of -2-[(2,4-dimethylthiazol-5-yl)methoxyimino]-3-[(1-methylpyrazol-4-yl)methyl]-4-oxo-1H-quinazoline-6-sulfonyl chloride:

NCS (293 mg, 2.08 mmol) was added to a stirred solution of 6-benzylsulfanyl-2-[(2,4-dimethylthiazol-5-yl)methoxyimino]-3-[(1-methylpyrazol-4-yl)methyl]-1*H*-quinazolin-4-one (300 mg, 0.52 mmol) in AcOH (3 mL) and H₂O (1 mL). After 2 h stirring, the reaction mixture was diluted with EA (20 mL), dried over Na₂SO₄, filtered and concentrated to afford -2-[(2,4-dimethylthiazol-5-yl)methoxyimino]-3-[(1-methylpyrazol-4-yl)methyl]-4-oxo-1*H*-quinazoline-6-sulfonyl chloride as a yellow oil (260 mg, 40% yield) which was used in the next step without further purification.

MS m/z (+ESI): 495.1, 497.1 [M+H]⁺.

### Step 7: Preparation of 2-[(2,4-dimethylthiazol-5-yl)methoxyimino]-N-(1-methylcyclopropyl)-3-[(1-methylpyrazol-4-yl)methyl]-4-oxo-1H-quinazoline-6-sulfonamide:

The title compound was prepared as a white solid (17 mg, 14% yield) following Scheme 1 and in analogy to Example 1 (step 5) using 2-[(2,4-dimethylthiazol-5-yl)methoxyimino]-3-[(1-methylpyrazol-4-yl)methyl]-4-oxo-1*H*-quinazoline-6-sulfonyl chloride (260 mg, 0.21 mmol) as starting material.

¹H NMR (400 MHz, DMSO-*d₆*+D₂O) δ ppm: 8.18 (d, *J* = 2.4 Hz, 1H), 7.84 (dd, J= 2.4, 8.8 Hz, 1H), 7.47 (m, 2H), 7.32 (s, 1H), 5.13 (s, 2H), 4.80 (s, 2H), 3.72 (s, 3H), 2.56 (s, 3H), 2.35 (s, 3H), 1.02 (s, 3H), 0.56 (m, 2H), 0.35 (m, 2H).

MS m/z (+ESI): 530.4 [M+H]⁺.

### Preparation of Example 5: 4-[2-methoxyimino-6-[(1-methylcyclopropyl)sulfamoyl]-3-[(1-methylpyrazol-4-yl)methyl]-4-oxo-1H-quinazolin-8-yl]-N,N-dimethyl-benzamide:

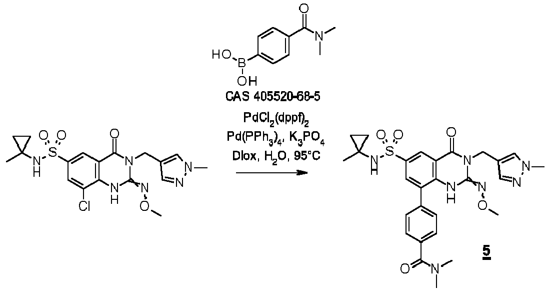

[4-(Dimethylcarbamoyl)phenyl]boronic acid (60 mg, 0.30 mmol) was added to a stirred solution of 8-chloro-2-methoxyimino-*N-*(1-methylcyclopropyl)-3-[(1-methylpyrazol-4-yl)methyl]-4-oxo-1*H-*quinazoline-6-sulfonamide (120 mg, 0.25 mmol) in Diox (1.5 mL) and H₂O (0.3 mL), followed by K₃PO₄ (163 mg, 0.76 mmol), Pd(PPh₃)₄ (45 mg, 0.04 mmol) and PdCl₂(dppf)₂ (28 mg, 0.04 mmol). After 4 h stirring at 95°C, the reaction mixture was filtered. The filtrate was concentrated and purified by preparative HPLC to afford 4-[2-methoxyimino-6-[(1-methylcyclopropyl)sulfamoyl]-3-[(1-methylpyrazol-4-yl)methyl]-4-oxo-1*H*-quinazolin-8-yl]-*N*,*N*-dimethyl-benzamide as a white solid (38 mg, 26% yield).

¹H NMR (400 MHz, DMSO-*d₆*+D₂O) δ ppm: 8.28 (d, J= 2.0 Hz, 1H), 7.83 (d, J= 2.0 Hz, 1H), 7.69 (s, 1H), 7.62 (m, 4H), 7.43 (s, 1H), 4.82 (s, 2H), 3.75 (s, 3H), 3.69 (s, 3H), 3.01 (s, 3H), 2.94 (s, 3H), 1.08 (s, 3H), 0.62 (m, 2H), 0.40 (m, 2H).

MS m/z (+ESI): 566.4 [M+H]⁺.

### Preparation of Examples 7 and 8: 2-methoxyimino-N-(1-methylcyclopropyl)-3-[(1-methylpyrazo1-4-yl)methyl]-4-oxo-8-[(3R)-3-methylpiperazin-1-yl]-1H-quinazoline-6-sulfonamide and 2-methoxyimino-N-(1-methylcyclopropyl)-3-[(1-methylpyrazol-4-yl)methyl]-4-oxo-8-[(3R)-3-methyl-4-(1-methylcyclopropanecarbonyl)piperazin-1-yl]-1H-quinazoline-6-sulfonamide:

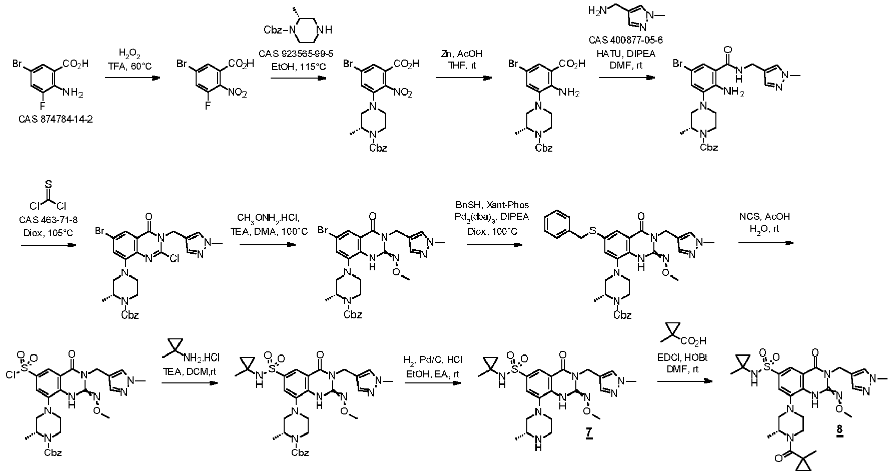

### Step 1: Preparation of 5-bromo-3-fluoro-2-nitro-benzoic acid:

30% aqueous H₂O₂ (5.9 mL, 58.0 mmol) was added to a stirred solution of 2-amino-5-bromo-3-fluorobenzoic acid in TFA (10 mL). After 2 h stirring at 60°C, the reaction mixture was quenched by addition at 0°C of aqueous NaHSO₃, concentrated and purified by Biotage combiflash to afford 5-bromo-3-fluoro-2-nitro-benzoic acid as a yellow solid (1.52 g, 63% yield).

¹H NMR (400 MHz, DMSO-*d₆*) δ ppm: 8.31 (dd, *J* = 9.2 Hz, 2.0 Hz, 1H), 7.98 (t, J= 1.6 Hz, 1H).

MS m/z (+ESI): 263.9, 265.9 [M+H]⁺.

### Step 2: Preparation of 3-[(3R)-4-benzyloxycarbonyl-3-methyl-piperazin-1-yl]-5-bromo-2-nitro-benzoic acid:

Benzyl (2*R*)-2-methylpiperazine-1-carboxylate (416 mg, 1.70 mmol) was added to a stirred solution of 5-bromo-3-fluoro-2-nitro-benzoic acid (100 mg, 0.34 mmol) in EtOH (1 mL). After 2 h stirring at 115°C, the reaction mixture was concentrated and purified by Biotage combiflash to afford 3-[(3*R*)-4-benzyloxycarbonyl-3-methyl-piperazin-1-yl]-5-bromo-2-nitro-benzoic acid as a yellow solid (160 mg, 88% yield).

¹H NMR (400 MHz, DMSO-*d₆*) δ ppm: 14.28 (br, 1H), 7.98 (d, J= 2.0 Hz, 1H), 7.87 (d, *J=* 2.0 Hz, 1H), 7.36 (m, 5H), 5.10 (m, 2H), 4.25 (m, 1H), 3.86 (m, 1H), 3.08 (m, 1H), 2.93 (m, 4H), 1.14 (d, *J=* 6.8 Hz, 3H).

MS m/z (+ESI): 476.4, 478.4 [M+H]⁺.

### Step 3: Preparation of 2-amino-3-[(3R)-4-benzyloxycarbonyl-3-methyl-piperazin-1-yl]-5-bromo-benzoic acid:

Zn (186 mg, 2.82 mmol) was added to a stirred solution of 3-[(3*R*)-4-benzyloxycarbonyl-3-methyl-piperazin-1-yl]-5-bromo-2-nitro-benzoic acid (150 mg, 0.28 mmol) in THF (2 mL) and AcOH (1 mL). After 16 h stirring, the reaction mixture was diluted with THF and filtered. The filtrate was concentrated and purified by Biotage combiflash to afford 2-amino-3-[(3*R*)-4-benzyloxycarbonyl-3-methyl-piperazin-1-yl]-5-bromo-benzoic acid as a yellow solid (110 mg, 78% yield).

¹H NMR (400 MHz, DMSO-*d₆*) δ ppm: 7.61 (d, *J=* 2.0 Hz, 1H), 7.38 (m, 4H), 7.33 (m, 1H), 7.17 (d, *J=* 2.0 Hz, 1H), 5.11 (m, 2H), 4.30 (m, 1H), 3.85 (m, 1H), 3.38 (m, 1H), 2.83 (m, 1H), 2.83 (m, 2H), 2.46 (m, 1H), 1.32 (d, *J=* 6.8 Hz, 3H).

MS m/z (+ESI): 448.2, 450.2 [M+H]⁺.

### Step 4: Preparation of benzyl (2R)-4-[2-amino-5-bromo-3-[(1-methylpyrazol-4-yl)methylcarbamoyl]phenyl]-2-methyl-piperazine-1-carboxylate:

(1-Methylpyrazol-4-yl)methanamine (563 mg, 5.02 mmol) was added to a stirred solution of 2-amino-3-[(3*R*)-4-benzyloxycarbonyl-3-methyl-piperazin-1-yl]-5-bromo-benzoic acid (1.25 g, 2.51 mmol) in DMF (8 mL), followed by HATU (1.28 g, 3.26 mmol) and DIPEA (1.3 mL, 7.53 mmol). After 4 h stirring, the reaction mixture was concentrated and purified by Biotage combiflash to afford benzyl (2*R*)-4-[2-amino-5-bromo-3-[(1-methylpyrazol-4-yl)methylcarbamoyl]phenyl]-2-methyl-piperazine-1-carboxylate as a white solid (1.18 g, 78% yield).

¹H NMR (400 MHz, DMSO-*d₆*) δ ppm: 8.72 (t, J= 5.6 Hz, 1H), 7.58 (s, 1H), 7.48 (d, J= 2.0 Hz, 1H), 7.35 (m, 6H), 7.09 (d, *J=* 2.0 Hz, 1H), 6.28 (s, 2H), 5.10 (m, 2H), 4.29 (m, 1H), 4.21 (d, *J=* 5.6 Hz, 2H), 3.85 (m, 1H), 3.77 (s, 3H), 3.37 (m, 1H), 2.99 (m, 1H), 2.80 (m, 2H), 2.46 (m, 1H), 1.31 (d, *J=* 6.8 Hz, 3H).

MS m/z (+ESI): 541.2, 543.2 [M+H]⁺.

### Step 5: Preparation of benzyl (2R)-4-[6-bromo-2-chloro-3-[(1-methylpyrazol-4-yl)methyl]-4-oxo-quinazolin-8-yl]-2-methyl-piperazine-1-carboxylate:

Thiophosgene (81 mg, 0.70 mmol) was added to a stirred solution of benzyl (2*R*)-4-[2-amino-5-bromo-3-[(1-methylpyrazol-4-yl)methylcarbamoyl]phenyl]-2-methyl-piperazine-1-carboxylate (105 mg, 0.17 mmol) in Diox (4 mL). After 1 h stirring at rt and 1 h at 105 °C, the reaction mixture was concentrated and purified by Biotage combiflash to afford benzyl (2*R*)-4-[6-bromo-2-chloro-3-[(1-methylpyrazol-4-yl)methyl]-4-oxo-quinazolin-8-yl]-2-methyl-piperazine-1-carboxylate as a yellow solid (87 mg, 76% yield).

¹H NMR (400 MHz, DMSO-*d₆*) δ ppm: 7.75 (m, 2H), 7.47 (s, 1H), 7.35 (m, 6H), 5.19 (s, 2H), 5.11 (m, 2H), 4.29 (m, 1H), 3.92 (m, 1H), 3.78 (m, 1H), 3.77 (s, 3H), 3.48 (m, 1H), 3.27 (m, 1H), 2.82 (m, 2H), 1.37 (d, *J* = 6.8 Hz, 3H).

MS m/z (+ESI): 584.9, 586.8 [M+H]⁺.

### Steps 6-9: Preparation of benzyl (2R)-4-[2-methoxyimino-6-[(1-methylcyclopropyl)sulfamoyl]-3-[(1-methylpyrazol-4-yl)methyl]-4-oxo-1H-quinazolin-8-yl]piperazine-1-carboxylate:

The title compound was prepared as a light yellow solid following Scheme 1 and in analogy to Example 3 (steps 4-7) using benzyl (2*R*)-4-[6-bromo-2-chloro-3-[(1-methylpyrazol-4-yl)methyl]-4-oxo-quinazolin-8-yl]-2-methyl-piperazine-1-carboxylate, benzyl mercaptan, methoxyamine hydrochloride and 1-methylcyclopropanamine hydrochloride as starting materials.

¹H NMR (400 MHz, DMSO-*d₆*) δ ppm: 8.59 (s, 1H), 8.03 (d, *J=* 2.0 Hz, 1H), 8.01 (s, 1H), 7.73 (d, *J=* 2.0 Hz, 1H), 7.69 (s, 1H), 7.43 (s, 1H), 7.36 (m, 5H), 5.12 (m, 2H), 4.82 (m, 2H), 4.35 (m, 1H), 4.00 (m, 1H), 3.82 (s, 3H), 3.76 (s, 3H), 3.28 (m, 1H), 2.94 (m, 3H), 2.73 (m, 1H), 1.40 (d, *J* = 6.8 Hz, 3H), 1.03 (s, 3H), 0.58 (m, 2H), 0.37 (m, 2H).

MS m/z (+ESI): 651.0 [M+H]⁺.

### Step 10: Preparation of 2-methoxyimino-N-(1-methylcyclopropyl)-3-[(1-methylpyrazol-4-yl)methyl]-4-oxo-8-[(3R)-3-methylpiperazin-1-yl]-1H-quinazoline-6-sulfonamide:

10% Pd/C (200 mg, 0.19 mmol) was added to a stirred solution of benzyl (2*R*)-4-[2-methoxyimino-6-[(1-methylcyclopropyl)sulfamoyl]-3-[(1-methylpyrazol-4-yl)methyl]-4-oxo-1*H*-quinazolin-8-yl]piperazine-1-carboxylate (350 mg, 0.48 mmol) in EtOH (5 mL) and EA (5 mL), followed by 37% aqueous HCl (1.4 mL, 16.8 mmol). After 24 h stirring under hydrogen flow, the reaction mixture was diluted with MeOH/DCM (10/1, v/v) and filtered. The filtrate was concentrated and purified by preparative HPLC to afford 2-methoxyimino-*N-*(1-methylcyclopropyl)-3-[(1-methylpyrazol-4-yl)methyl]-4-oxo-8-[(3*R*)-3-methylpiperazin-1-yl]-1*H*-quinazoline-6-sulfonamide as a light yellow solid (124 mg, 45% yield).

¹H NMR (400 MHz, DMSO-*d₆*+D₂O) δ ppm: 8.01 (d, *J=* 2.0 Hz, 1H), 7.69 (d, *J=* 2.0 Hz, 1H), 7.67 (s, 1H), 7.42 (s, 1H), 4.81 (s, 2H), 3.83 (s, 3H), 3.74 (s, 3H), 3.19 (m, 2H), 3.01 (m, 3H), 2.81 (m, 1H), 2.62 (m, 1H), 1.16 (d, *J=* 6.4 Hz, 3H), 1.01 (s, 3H), 0.57 (m, 2H), 0.37 (m, 2H).

MS m/z (+ESI): 517.4 [M+H]⁺.

### Step 11: Preparation of 2-methoxyimino-N-(1-methylcyclopropyl)-3-[(1-methylpyrazol-4-yl)methyl]-4-oxo-8-[(3R)-3-methyl-4-(1-methylcyclopropanecarbonyl)piperazin-1-yl]-1H-quinazoline-6-sulfonamide:

1-Methylcyclopropanecarboxylic acid (32 mg, 0.31 mmol) was added to a stirred solution of 2-methoxyimino-*N-*(1-methylcyclopropyl)-3-[(1-methylpyrazol-4-yl)methyl]-4-oxo-8-[(3*R*)-3-methylpiperazin-1-yl]-1*H*-quinazoline-6-sulfonamide (90 mg, 0.16 mmol) in DMF (5 mL), followed by EDCI (62 mg, 0.31 mmol) and HOBt (44 mg, 0.31 mmol). After 3 h stirring, the reaction mixture was concentrated and purified by preparative HPLC to afford 2-methoxyimino-*N-*(1-methylcyclopropyl)-3-[(1-methylpyrazol-4-yl)methyl]-4-oxo-8-[(3*R*)-3-methyl-4-(1-methylcyclopropanecarbonyl)piperazin-1-yl]-1*H*-quinazoline-6-sulfonamide as a white solid (28 mg, 29% yield).

¹H NMR (400 MHz, DMSO-*d₆*+D₂O) δ ppm: 8.03 (d, *J=* 2.0 Hz, 1H), 7.76 (d, *J=* 2.0 Hz, 1H), 7.69 (s, 1H), 7.43 (s, 1H), 4.82 (s, 2H), 4.61 (m, 1H), 4.26 (m, 1H), 3.82 (s, 3H), 3.76 (s, 3H), 2.96 (m, 3H), 2.67 (m, 2H), 1.41 (br, 3H), 1.25 (s, 3H), 1.01 (s, 3H), 0.83 (m, 2H), 0.57 (m, 4H), 0.37 (m, 2H).

MS m/z (+ESI): 599.5 [M+H]⁺.

### Biological Examples

### PARG enzymatic assay

Inhibition of PARG enzymatic activity by compounds was determined as follows: Recombinant Histagged human PARG protein expressed in Sf21 insect cells (ProSci Incorporated, # 90-350 or Adipogen AG, # 40T-0022) was diluted to 100 nM in dilution buffer (50 mM Tris-HCl pH 7.5, 100 mM NaCl, 0.2% NP-40, 10% glycerol) and stored at -80°C until use. The artificial enzyme substrate 4-(trifluoromethyl)umbelliferone (TFMU)-ADPr was prepared essentially as described (Drown BS et al, *Cell Chemical Biology* 2018) and stored at -20°C as 10 mM stock solutions in DMSO (dimethyl sulfoxide) until use. For reactions, typical final concentration of the enzyme was 2 nM and of the TFMU-ADPr 200 µM. Reactions were carried out in black 384-well low volume round bottom assay plates (Corning, # 4514) in a final volume of 10 µL. To this end, PARG was diluted to 2.062 nM in reaction buffer (50 mM K₂HPO₄, 50 mM KCl, 10 mM β-mercaptoethanol, pH 7.4) and 9.7 µL were dispensed per assay well using a multistep pipette. Then, 0.1 µL compound was dispensed into each well using a Tecan D300e digital dispenser (Tecan AG), based on 10 mM compound DMSO stocks, to yield the prespecified final concentration of the range of concentrations to be tested (typically 9-point serial concentrations ranging from either from 0.01 to 100 µM, or from 0.001 to 10 µM, or from 0.0001 to 1 µM), and being volume-corrected with 100% DMSO (3% final DMSO concentration in reaction mixtures). Respective compound concentrations were assessed at minimum in duplicates. Plates were mixed, and after 5 minutes 0.2 µL TFMU-ADPr was added into each well using a Tecan D300e digital dispenser (Tecan AG) followed by brief mixing. Fluorescence intensity signals were then measured at time 0 and after 12-15 minutes (depending on specific enzyme activity) on a Synergy 4 microplate reader (BioTek Instruments) using 380/20 nm and 530/25 nm filters for the excitation and emission, respectively (gain: 50). The assay window was set using DMSO control (top) and a control containing no PARG enzyme (bottom). Delta values were plotted as concentration-response curves fitted to a sigmoidal 4-parameter logistic model to calculate IC₅₀ values (GraphPadPrism^{™} version 9.3.1). Results are shown in Table 2 below.

### PARG NanoBRET-based cellular target engagement assay

PARG Nano-luciferase construct: The PARG-NanoLuc^{®} fusion vector was generated by Promega, by inserting the full length human PARG cDNA sequence encoding the 976 amino acid protein (Gene ID: 8505, UniProt: Q86W56) into the pNLF1-C [CMV/Hygro] vector (C-terminal fusions with NanoLuc^{®} enzyme).

PARG target engagement tracer synthesis: The NanoBRET tracer for PARG was prepared following a previously reported general tracer synthesis procedure.

PARG Probe Displacement Assay Using NanoBRET: HEK293T (DSMZ # ACC 635) cells transiently transfected with the PARG-NanoLuc fusion construct were incubated for 24 hours under standard growth conditions. Transfected cells were resuspended in phenol red-free OptiMEM (Gibco #11058-021) to a concentration of 3 × 10⁵ cells/mL, and 20 µL were then added per well of a white polystyrene 384-well non-binding surface microplate. Then, the NanoBRET probe (at 1 µM) and each test compound in DMSO were added to the microplate using the Tecan D300e digital dispenser (Tecan AG). The plate was incubated in a 5% CO₂ environment at 37°C for 2 hours, and then 10 µL per well of a solution consisting of a 1:200 dilution of Nano-Glo substrate (Promega) and a 1:750 dilution of NanoLuc extracellular inhibitor (Promega) in phenol red-free OptiMEM was added to each well. Filtered luminescence was measured on a Synergy4 plate reader (BioTek) equipped with a 460 nm BP filter (donor) and a 610 nm LP filter (acceptor). BRET ratios were plotted as concentration-response curves fitted to a sigmoidal 4-parameter logistic model to calculate IC₅₀ values (GraphPadPrism^{™} version 9.3.1). Results are shown in Table 2 below.

### Detection of cellular PAR levels by Western blotting

Kuramochi high grade serous ovarian cancer (HGSOC) (JCRB, # JCRB0098) or NCI-H1650 non-small cell lung cancer (NSCLC) (ATCC, # CRL-5883) cells were cultured in RPMI (BioConcept, # 1-41F03-I) containing 10% FCS (Sigma # F9665) and 1% Penicillin-Streptomycin (BioConcept, # 4-01F00-H).When cells reached a density of 70% in 6-well plates (TPP, # 92006), they were treated either with the test compound vehicle DMSO, or with test compounds at indicated final concentrations for either 4, 6 or 8 hours. Thereafter, the culture medium was removed and cells were collected by scraping in Lysis Buffer (20 mM Tris HCl pH 7.5, 150 mM NaCl, 1 mM EGTA, 1 mM EDTA, 1% Triton and 1% NP-40) containing protease and phosphatase inhibitors (Halt^{™} Protease&Phosphatase inhibitor cocktail 100× ThermoScientific, #1861281) and 1 mM PMSF (Fluka, # 93482). Cleared lysates were stored in Eppendorf Tubes^{™} at -80°C until use, and protein concentration in lysates was determined using Pierce 660 nm Protein Assay Reagent (ThermoScientific, # 22660). 20 µg proteins were diluted in 4x Laemmli buffer (Biorad, # 161-0747) containing 10% β-mercaptoethanol, separated by SDS-PAGE using 10% gels, and then transferred to PVDF membranes (Trans Blot Turbo Transfer Pack BioRad, # 1704156) by Trans-Blot^{®} Turbo^{™} System semidry blotting methodology (BioRad). Primary antibodies were incubated in TBS-0.1% Tween-20 buffer containing 3% bovine serum albumin (Millipore, # 81-053-3) overnight at 4°C. The following primary antibodies were used: anti-PAR (Ab-1) mouse monoclonal antibody (10H) (Millipore, # AM80-100UG), Poly/Mono-ADP ribose (PAR/MAR) (E6F6A) rabbit monoclonal antibody (Cell Signaling, # 83732), and rabbit anti-GAPDH clone 14C10 (Cell Signaling, # 2118S) diluted 1:2000 or mouse α-tubulin (Sigma, # T5168) diluted 1:5000 to control for equal protein loading. Horseradish peroxidase (HRP)-conjugated secondary antibodies were incubated one hour at room temperature diluted 1:5000 in TBS-0.1% Tween-20 buffer containing 5% nonfat-dried bovine milk (Sigma, # M7409). The following secondary antibodies were used: Goat anti-mouse-HRP (Jackson, # 115-035-146) and goat anti-rabbit-HRP (Jackson, # 111-035-144). Membranes were incubated with enhanced chemiluminescence (ECL) Prime Western Blot Detection Reagent (Amersham, # RPN2236) to detect specific signals using the Fusion SOLO S imaging system (Vilber Lourmat). Results are shown in Figure 1.

### Cell proliferation assays

RMUG-S mucinous cystadenocarcinoma (JCRB, # IFO50320) and TOV-112D endometrioid adenocarcinoma (ATCC, # CRL-11731) ovarian cancer cell lines were grown in DMEM/F-12 with glutamine (BioConcept, # 1-26F09-I) and RPMI with glutamine (BioConcept, # 1-41F03-1), respectively, and both media containing 10% FBS (Sigma-Aldrich, # F9665) and supplemented with 1% Penicillin-Streptomycin (BioConcept, # 4-01F00-H) using standard cell culture techniques. RMUG-S and TOV-112D ovarian cancer cell lines were selected as highly PARG dependent and having low PARG dependency, respectively, based on their publically available PARG shRNA (short hairpin ribonucleic acid) dropout profiles in the Cancer Dependency Map (DepMap) data portal (Broad Institute DepMap Project; Cheung HW et al, Proceedings of the National Academy of Sciences 2011; Cowley GS et al, Scientific Data 2014; McDonald ER et al, Cell 2017). PARG dependency of these cell lines was independently verified and confirmed by genetic and pharmacological means using shRNA-mediated PARG depletion, and the previously reported cell-permeable quinazolinedione PARG inhibitor tool compound PDD00017273 (Pillay N et al, Cancer Cell 2019). Thus, growth inhibition of the RMUG-S cell line serves as a functional on-target inhibition readout, while observing potent growth inhibition of TOV-112D is indicative of compound off-target activity. Cells were seeded in 96-well plates with clear bottom (Huberlab, # 7.655 090) at a seeding density of 5000 cells per well in 100 µL of medium, and the plates were incubated overnight at 37°C with 5% CO₂ prior to treatment with compounds. Experimental compounds were prepared in DMSO at a concentration of 10 mM. Compounds were distributed at the desired final concentrations using the Tecan D300e Digital Dispenser (TECAN) and normalized to the highest DMSO volume. The plates were incubated for 120 hours and cell numbers were then measured using CellTiter-Glo^{®} (Promega, # G9241), essentially as recommended by the manufacturer. Briefly, for the CellTiter-Glo^{®} proliferation readout, 50 µL of the reconstituted CellTiter-Glo^{®} 2.0 Reagent were added to 100 µL of medium containing cells. The plates were incubated at room temperature for 10 minutes to stabilize the luminescent signal, and luminescence intensity signals were then measured with a Synergy 4^{™} microplate reader (BioTek Instruments). Relative proliferation values were calculated by normalizing the raw data using DMSO-treated cells (100% proliferation) and the signals obtained from cells that were evaluated at the time of compound addition (0% proliferation). The concentration-response data were fitted to a sigmoidal 4-parameter logistic model with the maximum constrained to 100%. GI₅₀ values were calculated from the curves as the compound concentrations reducing proliferation to 50%. Results are shown in Table 2 below.

**Table 2: PARG biochemical enzyme inhibition, intra-cellular PARG target engagement (TE), and growth inhibition of RMUG-S (high PARG dependency) and TOV-112D (low PARG dependency) ovarian cancer cell lines.**

| Example | **PARG** IC₅₀ | PARG TE ICso | **RMUG-S** GI₅₀ (high PARG dependency) | **TOV-112D** GI₅₀ (low PARG dependency) |
|---|---|---|---|---|
| 1 | +++ | ++ | ++ | - |
| 2 | ++ | + | + | - |
| 3 | +++ | ++ | + | - |
| 4 | +++ | + | ND | ND |
| 5 | +++ | ++ | ++ | - |
| 6 | + | + | ND | ND |
| 7 | +++ | +++ | ++ | - |
| 8 | +++ | +++ | +++ | + |
| 9 | +++ | ++ | ND | ND |
| 10 | +++ | ++ | ND | ND |
| 11 | ++ | ++ | ND | ND |

| | | | | |
|---|---|---|---|---|
| For PARG IC₅₀ (nM): +++ when < 30, ++ when 30-99, + when 100-1000. For PARG TE IC₅₀ (nM): +++ when < 30, ++ when 30-99, + when 100-1000. For RMUG-S GI₅₀ (nM): +++ when < 500, ++ when 500-1999, + when 2000-8999. Highest concentration tested is 9000 nM. ND: Not determined. For TOV-112D GI₅₀ (nM): +++ when < 500, ++ when 500-1999, + when 2000-8999, - when ≥ 9000. Highest concentration tested is 9000 nM. ND: Not determined. | | | | |

### Literature References

*Ordered as citations appear in the text*
Hanahan D, Cancer Discovery 2022, 12(1):31-46
Gaillard H et al, Nature Reviews Cancer 2015, 15(5):276-89
Brown JS et al, Cancer Discovery 2017, 7(1):20-37
Curtin NJ, Nature Reviews Cancer 2012, 12(12):801-17
Hartwell LH et al, Science 1997, 278(5340):1064-8
Farmer H et al, Nature 2005, 434(7035):917-21
Bryant HE et al, Nature 2005, 434(7035):913-7
Turner N et al, Nature Reviews Cancer 2004, 4(10):814-9
Hottiger MO et al, Trends in Biochemical Sciences 2010, 35(4):208-19
Leung AKL, Trends in Cell Biology 2020, 30(5):370-383
Barkauskaite E at al, Nature Communications 2013, 4:2164
Pourfarjam Y et al, Biochemical and Biophysical Research Communications 2020, 527(3):818-823
Marques M et al, Oncogene 2019, 38(12):2177-2191
Pillay N et al, Cancer Cell 2019, 35(3):519-533
Coulson-Gilmer C et al, Journal of Experimental & Clinical Cancer Research 2021, 40(1):323
Prokhorova E et al, Molecular Cell, 2021, 81(12):2640-2655
Chen S-H and Yu X, Science Advances 2019, 5(4):eaav4340
Slade D, Genes & Development 2020, 34(5-6):360-394
Houl JH et al, Nature Communications 2019, 10(1):5654
Wuts P.G.M, Greene's Protective Groups in Organic Synthesis, 5th Edition, Publisher: John Wiley & Sons, 2014
Drown BS et al, Cell Chemical Biology 2018, 25(12):1562-1570
Cheung HW et al, Proceedings of the National Academy of Sciences USA 2011, 108(30):12372-7
Cowley GS et al, Scientific Data 2014, 1:140035
McDonald ER et al, Cell 2017, 170(3):577-592

## Claims

1. A compound of formula (I) 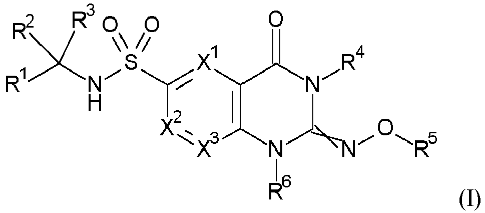 or a pharmaceutically acceptable salt thereof,
wherein
R¹ is hydrogen, cyano, formyl, -CONH₂, -CH₂OH, -CH₂OC₁₋₂ alkyl, C₁₋₂ alkyl, C₁₋₂ haloalkyl or ethynyl;
R² and R³ are independently C₁₋₂ alkyl;
or R² and R³ together with the carbon atom to which they are attached form cyclopropyl or oxetanyl;
X¹ is CR⁷ or N;
R⁷ is hydrogen or fluoro;
X² is CR⁸ or N;
R⁸ is hydrogen or fluoro;
X³ is CR⁹ or N;
R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, heteroaryl, heterocyclyl, fused heterocyclyl, spiro heterocyclyl or bridged heterocyclyl, wherein the cycloalkyl, phenyl, heteroaryl, heterocyclyl, fused heterocyclyl, spiro heterocyclyl and bridged heterocyclyl are optionally substituted with R^{a}, R^{b} and/or R^{c};
R^{a} is hydrogen, -N(R^{m})Rⁿ, C₁₋₆ alkyl, hydroxy, C₁₋₆ alkoxy, halogen, cyano, oxo, C₁₋₆ haloalkyl, C₁₋₆ haloalkoxy, hydroxyC₁₋₆ alkyl, heteroaryl, heterocyclyl, -C(O)R^{d}, -C(O)OR^{e}, -C(O)N(R^{f})R^{g}, - S(O)₂N(R^{h})Rⁱ or C₁₋₆ alkyl-N(R^{j})R^{k};
R^{b} and R^{c} are independently hydrogen, -N(R^{m})Rⁿ, C₁₋₆ alkyl, C₁₋₄ alkyl-N(R^{m})Rⁿ, hydroxy, C₁₋₆ alkoxy, halogen, cyano, C₁₋₆ haloalkyl or C₁₋₆ haloalkoxy;
R^{d} is hydrogen, C₁₋₆ alkyl, C₁₋₆ haloalkyl, C₃₋₆ cycloalkyl, phenyl, heteroaryl or heterocyclyl;
R^{e} is hydrogen or C₁₋₆ alkyl;
R^{f}, R^{g}, R^{h}, Rⁱ, R^{j}, R^{k}, R^{m} and Rⁿ are each independently hydrogen, C₁₋₆ alkyl, C₁₋₆ haloalkyl, aminoC₁₋₆ alkyl or hydroxyC₁₋₆ alkyl; or
R^{f} and R^{g}, R^{h} and Rⁱ, or R^{j} and R^{k} together with the nitrogen atom to which they are attached form heterocyclyl;
wherein
(i) the heteroaryl and heterocyclyl of R^{a};
(ii) the cycloalkyl, phenyl, heteroaryl and heterocyclyl of R^{d};
(iii) the heterocyclyl formed by R^{f} and R^{g}, R^{h} and Rⁱ, or R^{j} and R^{k} combining with the nitrogen to which they are attached;
are each ((i), (ii), (iii)) optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy;
R⁴ is hydrogen or the group -L^{1A}-L^{2A}-L^{3A};
L^{1A} is absent or is C₁₋₃ alkylene optionally substituted by C₁₋₂ alkyl or oxo;
L^{2A} is absent or is -O-, -S-, -S(O)-, -S(O)₂-, -N(R^{a1})-, -C(O)-, -C(O)O-, -OC(O)-, -C(O)N(R^{a1})-, - N(R^{a1})C(O)-, -N(R^{a1})C(O)N(R^{b1})-, -S(O)₂N(R^{a1})-, or -N(R^{a1})S(O₂-;
R^{a1} and R^{b1} are each independently hydrogen or C₁₋₂ alkyl;
L^{3A} is hydrogen, C₁₋₆ alkyl, C₃₋₆ cycloalkyl, phenyl, heterocyclyl or heteroaryl, wherein L^{3A} is optionally substituted by one or more substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, - C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, - N(R^{d1})S(O)₂R^{c1} and -(CH₂)_{z}(R^{d1})R^{c1};
R^{c1} and R^{d1} are each independently hydrogen or C₁₋₄ alkyl;
y is 0, 1 or 2;
z is 1, 2 or 3;
R⁵ is hydrogen or the group -L^{1B}-L^{2B}-L^{3B};
L^{1B} is absent or is C₁₋₃ alkylene optionally substituted by C₁₋₂ alkyl or oxo;
L^{2B} is absent or is -O-, -S-, -S(O)-, -S(O)₂-, -N(R^{a2})-, -C(O)-, -C(O)O-, -OC(O)-, -C(O)N(R^{a2})-, - N(R^{a2})C(O)-, -N(R^{a2})C(ON(R^{b2})-, -S(O₂N(R^{a2})-, or -N(R^{a2})S(O₂-;
R^{a2} and R^{b2} are each independently hydrogen or C₁₋₂ alkyl;
L^{3B} is hydrogen, C₁₋₆ alkyl, C₃₋₆ cycloalkyl, phenyl, heterocyclyl or heteroaryl, wherein L^{3B} is optionally substituted by one or more substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, - C(O)R^{c2}, -C(O)OR^{c2}, -OC(O)R^{c2}, -C(O)N(R^{d2})R^{c2}, -N(R^{d2})C(O)R^{c2}, -S(O)_{y'}R^{c2}, -S(O)₂N(R^{d2})R^{c2}, - N(R^{d2})S(O)₂R^{c2} and -(CH₂)_{z'}N(R^{d2})R^{c2};
R^{c2} and R^{d2} are each independently hydrogen or C₁₋₄ alkyl;
y' is 0, 1 or 2;
z' is 1, 2 or 3;
R⁶ is hydrogen or the group -L^{1C}-L^{2C}-L^{3C};
L^{1C} is absent or is C₁₋₃ alkylene optionally substituted by C₁₋₂ alkyl or oxo;
L^{2C} is absent or is -O-, -S-, -S(O)-, -S(O)₂-, -N(R^{a3})-, -C(O)-, -C(O)O-, -OC(O)-, -C(O)N(R^{a3})-, - N(R^{a3})C(O)-, -N(R^{a3})C(O)N(R^{b3})-, -S(O)₂N(R^{a3})-, or -N(R^{a3})S(O)₂-;
R^{a3} and R^{b3} are each independently hydrogen or C₁₋₂ alkyl;
L^{3C} is hydrogen, C₁₋₆ alkyl, C₃₋₆ cycloalkyl, phenyl, heterocyclyl or heteroaryl, wherein L^{3C} is optionally substituted by one or more substituents independently selected from halogen, trifluoromethyl, trifluoromethoxy, cyano, hydroxy, carboxy, carbamoyl, sulphamoyl, C₁₋₄ alkyl, -N(R^{c3})R^{d3}, -OR^{c3}, - C(O)R^{c3}, -C(O)OR^{c3}, -OC(O)R^{c3}, -C(O)N(R^{d3})R^{c3}, -N(R^{d3})C(O)R^{c3}, -S(O)_{y"}R^{c3}, -S(O)₂N(R^{d3})R^{c3}, - N(R^{d3})S(O)₂R^{c3} and -(CH₂)_{z"}N(R^{d3})Rc³;
R^{c3} and R^{d3} are each independently hydrogen or C₁₋₄ alkyl;
y" is 0, 1 or 2;
z" is 1, 2 or 3;
with the proviso that
the groups -L^{1A}-L^{2A}-L^{3A}, -L^{1B}-L^{2B}-L^{3B}, and -L^{1C}-L^{2C}-L^{3C} do not contain an -O-O-, -S-O-, -O-S- or -S-S- unit as a linking unit within the group;
the group -L^{1A}-L^{2A}-L^{3A} does not place an -O-N- or -S-N- unit adjacent to the ring nitrogen atom connected to R⁴;
the group -L^{1B}-L^{2B}-L^{3B} does not place an N, O or S atom adjacent to the oxime oxygen atom connected to R⁵, and
-L^{1C}-L^{2C}-L^{3C} does not place an -O-N- or -S-N- unit adjacent to the ring nitrogen atom connected to R⁶.

2. The compound of formula (I) or a pharmaceutically acceptable salt thereof according to claim 1, wherein X¹ is CR⁷, X² is CR⁸ and X³ is CR⁹.

3. The compound of formula (I) or a pharmaceutically acceptable salt thereof according to claim 1 or claim 2, wherein R¹ is cyano, C₁₋₂ alkyl or ethynyl.

4. The compound of formula (I) or a pharmaceutically acceptable salt thereof according to any one of claims 1 to 3, wherein R² and R³ together with the carbon atom to which they are attached form cyclopropyl or oxetanyl.

5. The compound of formula (I) or a pharmaceutically acceptable salt thereof according to any one of claims 1 to 4, wherein R⁹ is hydrogen, halogen, cyano, C₁₋₄ alkyl, C₁₋₄ haloalkyl, C₁₋₄ alkoxy, C₁₋₄ haloalkoxy, C₂₋₄alkynyl, C₃₋₆ cycloalkyl, phenyl, 5- to 6-membered heteroaryl containing 1, 2 or 3 heteroatoms as ring atoms independently selected from N, S and O, 5- to 7-membered monocyclic heterocyclyl containing 1, 2 or 3 heteroatoms as ring atoms independently selected from N, S and O, wherein the cycloalkyl, phenyl, heteroaryl and heterocyclyl are optionally substituted with R^{a}, R^{b} and/or R^{c}; or
R⁹ is spiro heterocyclyl, wherein the first ring connected to X³ is a 5- to 7-membered monocyclic heterocyclyl containing 1, 2 or 3 heteroatoms as ring atoms independently selected from N, S and O and the second ring is connected to the first ring with a common carbon atom and is a 3- to 6-membered monocyclic cycloalkyl or a 3- to 6-membered monocyclic heterocyclyl containing 1, 2 or 3 heteroatoms as ring atoms independently selected from N, S and O, wherein spiro heterocyclyl is optionally substituted by R^{a}, R^{b} and/or R^{c}.

6. The compound of formula (I) or a pharmaceutically acceptable salt thereof according to claim 5, wherein R⁹ is hydrogen, halogen, cyano, phenyl, 5- to 6-membered heteroaryl containing 1 or 2 heteroatoms as ring atoms selected from N, wherein the phenyl and 6-membered heteroaryl are optionally substituted at the para position with respect to the connection to the rest of the molecule with R^{a} and are additionally optionally substituted with R^{b}, and the 5-membered heteroaryl is optionally substituted at the 3 position distal to the connection to the rest of the molecule with R^{a} and is additionally optionally substituted with R^{b} or is the moiety (R^{9a}) or the moiety (R^{9b}): 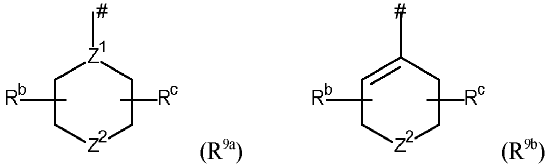 wherein Z¹ is N or CH, Z² is N(R^{a}), O, S, CH(R^{a}) or C(R^{x})(R^{y}), wherein R^{x} and R^{y} together form a 4- to 6-membered monocyclic heterocyclyl containing one heteroatom selected from N, O and S.

7. The compound of formula (I) or a pharmaceutically acceptable salt thereof according to any one of claims 1 to 6, wherein
R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k};
R^{b} is hydrogen, amino, -NH(C₁₋₄ alkyl), -N(C₁₋₄ alkyl)₂, C₁₋₄ alkyl, C₁₋₄ alkyl-NH₂, C₁₋₄ alkyl-NH(C₁₋₄ alkyl) or C₁₋₄ alkyl-N(C₁₋₄ alkyl)₂;
R^{c} is hydrogen or C₁₋₄ alkyl;
R^{d} is hydrogen, C₁₋₆ alkyl, C₁₋₆ haloalkyl, C₃₋₆ cycloalkyl, phenyl, heteroaryl or heterocyclyl, wherein the cycloalkyl, phenyl, heteroaryl and heterocyclyl are optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkyl-N(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy;
R^{f} is hydrogen or C₁₋₄ alkyl;
R^{g} is hydrogen or C₁₋₄ alkyl;
R^{j} is hydrogen or C₁₋₄ alkyl;
R^{k} is hydrogen or C₁₋₄ alkyl;
R^{m} is hydrogen or C₁₋₄ alkyl; and
Rⁿ is hydrogen or C₁₋₄ alkyl.

8. The compound of formula (I) or a pharmaceutically acceptable salt thereof according to any one of claims 1 to 7, wherein
R⁴ is the group -L^{1A}-L^{2A}-L^{3A},
L^{2A}, L^{2B} and L^{2C} are absent;
L^{3A} is cycloalkyl, phenyl, heterocyclyl or heteroaryl, each optionally substituted;
no more than two of L^{3A}, L^{3B}, and L^{3C} are cycloalkyl, phenyl, heterocyclyl or heteroaryl, each optionally substituted; and
L^{3C} is not cycloalkyl, phenyl, heterocyclyl or heteroaryl when R⁹ is cycloalkyl, phenyl, heteroaryl, heterocyclyl, fused heterocyclyl, spiro heterocyclyl or bridged heterocyclyl.

9. The compound of formula (I) or a pharmaceutically acceptable salt thereof according to any one of claims 1 to 8, wherein R⁴ is the group -L^{1A}-L^{2A}-L^{3A}, wherein L^{3A} is cycloalkyl, phenyl, heterocyclyl or heteroaryl, each optionally substituted; R⁵ is hydrogen or the group -L^{1B}-L^{2B}-L^{3B}, wherein L^{3B} is cycloalkyl, phenyl, heterocyclyl or heteroaryl, each optionally substituted, or -L^{1B}-L^{2B}-L^{3B} is C₁₋₆ alkyl; and R⁶ is hydrogen or C₁₋₆ alkyl.

10. The compound of formula (I) or a pharmaceutically acceptable salt thereof according to any one of claims 1 to 7, wherein
R⁴ is the group -L^{1A}-L^{2A}-L^{3A};
L^{1A} is C₁₋₃ alkylene;
L^{2A} is absent;
L^{3A} is phenyl or a 5- to 6-membered heteroaryl containing 1, 2 or 3 heteroatoms as ring atoms independently selected from N, O and S, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, -N(R^{d1})S(O)₂R^{c1} and -(CH₂)_{z}N(R^{d1})R^{c1};
R^{c1} is hydrogen or C₁₋₄ alkyl;
R^{d1} is hydrogen or C₁₋₄ alkyl;
y is 0, 1 or 2;
z is 1, 2 or 3;
R⁵ is hydrogen or the group -L^{1B}-L^{2B}-L^{3B};
L^{1B} is C₁₋₃alkylene;
L^{2B} is absent;
L^{3B} is phenyl or a 5- to 6-membered heteroaryl containing 1, 2 or 3 heteroatoms as ring atoms independently selected from N, O and S, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, -C(O)R^{c2}, -C(O)OR^{c2}, -OC(O)R^{c2}, -C(O)N(R^{d2})R^{c2}, -N(R^{d2})C(O)R^{c2}, -S(O)_{y'}R^{c2}, -S(O)₂N(R^{d2})R^{c2}, -N(R^{d2})S(O)₂R^{c2} and -(CH₂)_{z'}N(R^{d2})R^{c2};
R^{c2} is hydrogen or C₁₋₄ alkyl;
R^{d2} is hydrogen or C₁₋₄ alkyl;
y' is 0, 1 or 2;
z' is 1, 2 or 3;
or the group -L^{1B}-L^{2B}-L^{3B} is C₁₋₆ alkyl; and
R⁶ is hydrogen or the group -L^{1C}-L^{2C}-L^{3C}, wherein -L^{1C}-L^{2C}-L^{3C} is C₁₋₆ alkyl.

11. The compound of formula (I) or a pharmaceutically acceptable salt thereof according to claim 1, wherein
R¹ is cyano, C₁₋₂ alkyl or ethynyl;
R² and R³ together with the carbon atom to which they are attached form cyclopropyl or oxetanyl;
X¹ is CR⁷;
X² is CR⁸;
X³ is CR⁹;
R⁷ and R⁸ are hydrogen;
R⁹ is hydrogen, halogen, cyano, -N(R^{m})Rⁿ, C₁₋₄ alkyl, C₁₋₄ haloalkyl, C₁₋₄ alkoxy, C₁₋₄ haloalkoxy, C₂₋₄ alkynyl, C₃₋₆ cycloalkyl, phenyl, 5- to 6-membered heteroaryl containing 1, 2 or 3 heteroatoms as ring atoms independently selected from N, S and O, 5- to 6-membered monocyclic heterocyclyl containing 1, 2 or 3 heteroatoms as ring atoms independently selected from N, S and O, wherein the cycloalkyl, phenyl, heteroaryl and heterocyclyl are optionally substituted with R^{a}, R^{b} and/or R^{c};
or R⁹ is spiro heterocyclyl, wherein the first ring connected to X³ is a 5- to 7-membered monocyclic heterocyclyl containing 1, 2 or 3 heteroatoms as ring atoms independently selected from N, S and O and the second ring is connected to the first ring with a common carbon atom and is a 3- to 6-membered monocyclic cycloalkyl or a 3- to 6-membered monocyclic heterocyclyl containing 1, 2 or 3 heteroatoms as ring atoms independently selected from N, S and O, wherein spiro heterocyclyl is optionally substituted by R^{a}, R^{b} and/or R^{c};
R^{a} is hydrogen, -N(R^{m})Rⁿ, C₁₋₆ alkyl, oxo, -C(O)R^{d}, -C(O)N(R^{f})R^{g} or C₁₋₆ alkyl-N(R^{j})R^{k};
R^{b} is hydrogen -amino, -NH(C₁₋₄ alkyl), -N(C₁₋₄ alkyl)₂, C₁₋₄ alkyl, C₁₋₄ alkyl-NH₂, C₁₋₄ alkyl-NH(C₁₋₄ alkyl), C₁₋₄ alkyl-N(C₁₋₄ alkyl)₂;
R^{c} is hydrogen:
R^{d} is hydrogen, C₁₋₆ alkyl, C₁₋₆ haloalkyl, C₃₋₆ cycloalkyl, phenyl, heteroaryl or heterocyclyl, wherein the cycloalkyl, phenyl, heteroaryl and heterocyclyl are optionally substituted with 1, 2 or 3 substituents independently selected from C₁₋₆ alkyl, C₁₋₆ alkyl-NH₂, C₁₋₆ alkyl-NH(C₁₋₄ alkyl), C₁₋₆ alkylN(C₁₋₄ alkyl)₂, hydroxy, C₁₋₆ alkoxy, halogen, cyano, amino, -NH(C₁₋₄ alkyl), -N(C₁₋₄ alkyl)₂, C₁₋₆ haloalkyl and C₁₋₆ haloalkoxy;
R^{f} is hydrogen or C₁₋₄ alkyl;
R^{g} is hydrogen or C₁₋₄ alkyl;
R^{j} is hydrogen or C₁₋₄ alkyl;
R^{k} is hydrogen or C₁₋₄ alkyl;
each R^{m} is independently hydrogen or C₁₋₄ alkyl;
each Rⁿ is independently hydrogen or C₁₋₄ alkyl;
R⁴ is the group -L^{1A}-L^{2A}-L^{3A};
L^{1A} is C₁₋₃alkylene;
L^{1A} is absent;
L^{3A} is phenyl or 5- to 6-membered heteroaryl containing 1, 2 or 3 heteroatoms as ring atoms independently selected from N, S and O, wherein L^{3A} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c1})R^{d1}, -OR^{c1}, -C(O)R^{c1}, -C(O)OR^{c1}, -OC(O)R^{c1}, -C(O)N(R^{d1})R^{c1}, -N(R^{d1})C(O)R^{c1}, -S(O)_{y}R^{c1}, -S(O)₂N(R^{d1})R^{c1}, -N(R^{d1})S(O)₂R^{c1} and -(CH₂)_{z}N(R^{d1})R^{c1};
or the group -L^{1A}-L^{2A}-L^{3A}'is C₁₋₆ alkyl;
R^{c1} is hydrogen or C₁₋₄ alkyl;
R^{d1} is hydrogen or C₁₋₄ alkyl;
y is 0, 1 or 2;
z is 1, 2 or 3;
R⁵ is hydrogen or the group -L^{1B}-L^{2B}-L^{3B};
L^{1B} is C₁₋₃alkylene;
L^{2B} is absent;
L^{3B} is phenyl or a 5- to 6-membered heteroaryl containing 1, 2 or 3 heteroatoms as ring atoms independently selected from N, O and S, wherein L^{3B} is optionally substituted by 1, 2 or 3 substituents independently selected from halogen, trifluoromethyl, trifluoromethoxy, cyano, hydroxy, carboxy, carbamoyl, sulphamoyl, C₁₋₄ alkyl, -N(R^{c2})R^{d2}, -OR^{c2}, -C(O)R^{c2}, -C(O)OR^{c2}, -OC(O)R^{c2}, -C(O)N(R^{d2})R^{c2}, -N(R^{d2})C(O)R^{c2}, -S(O)_{y'}R^{c2}, -S(O)₂N(R^{d2})R^{c2}, -N(R^{d2})S(O)₂R^{c2} and -(CH₂)_{z'}N(R^{d2})R^{c2};
R^{c2} is hydrogen or C₁₋₄ alkyl;
R^{d2} is hydrogen or C₁₋₄ alkyl;
y' is 0, 1 or 2;
z' is 1, 2 or 3;
or the group -L^{1B}-L^{2B}-L^{3B} is C₁₋₆ alkyl;
R⁶ is hydrogen or the group -L^{1C}-L^{2C}-L^{3C}, wherein the group -L^{1C}-L^{2C}-L^{3C} is C₁₋₆ alkyl.

12. The compound of formula (I) or a pharmaceutically acceptable salt thereof according to claim 1, wherein the compound is selected from
2-Methoxyimino-N-(1-methylcyclopropyl)-3-[(2-methylthiazol-5-yl)methyl]-4-oxo-1*H*-quinazoline-6-sulfonamide;
2-methoxyimino-*N*-(3 -methyloxetan-3-yl)-3-[(1-methylpyrazol-4-yl)methyl]-4-oxo-1*H*-quinazoline-6-sulfonamide;
2-[(2,4-dimethylthiazol-5-yl)methoxyimino]-*N*-(1-methylcyclopropyl)-3-[(1-methylpyrazol-4-yl)methyl]-4-oxo-1*H*-quinazoline-6-sulfonamide;
8-chloro-2-methoxyimino-*N*-(1-methylcyclopropyl)-3-[(1-methylpyrazol-4-yl)methyl]-4-oxo-1*H-*quinazoline-6-sulfonamide;
4-[2-methoxyimino-6-[(1-methylcyclopropyl)sulfamoyl]-3-[(1-methylpyrazol-4-yl)methyl]-4-oxo-1*H-*quinazolin-8-yl]-*N*,*N*-dimethyl-benzamide;
2-methoxyimino-*N*-(1-methylcyclopropyl)-4-oxo-3-[[1-(trifluoromethyl)pyrazol-4-yl]methyl]-1H-quinazoline-6-sulfonamide;
2-methoxyimino-*N*-(1-methylcyclopropyl)-3-[(1-methylpyrazol-4-yl)methyl]-4-oxo-8-[(3*R*)-3-methylpiperazin-1-yl]-1*H*-quinazoline-6-sulfonamide;
2-methoxyimino-*N*-(1-methylcyclopropyl)-3-[(1-methylpyrazol-4-yl)methyl]-4-oxo-8-[(3*R*)-3-methyl-4-(1-methylcyclopropanecarbonyl)piperazin-1-yl]-1*H*-quinazoline-6-sulfonamide;
*N*-(1-cyanocyclopropyl)-2-methoxyimino-3-[(1-methylpyrazol-4-yl)methyl]-4-oxo-1*H*-quinazoline-6-sulfonamide;
2-ethoxyimino-*N*-(1-methylcyclopropyl)-3-[(1-methylpyrazol-4-yl)methyl]-4-oxo-1*H*-quinazoline-6-sulfonamide; and
2-isopropoxyimino-*N*-(1-methylcyclopropyl)-3-[(1-methylpyrazol-4-yl)methyl]-4-oxo-1*H*-quinazoline-6-sulfonamide.

13. A compound of formula (I) or a pharmaceutically acceptable salt thereof as defined in any one of claims 1 to 12 for use in the treatment of a neoplastic disease, preferably cancer, in a subject selected from a mammal, preferably a human.

14. A pharmaceutical composition comprising a compound of formula (I) or a pharmaceutically acceptable salt thereof as defined in any one of claims 1 to 12 and optionally one or more pharmaceutically acceptable excipients.

15. A compound of formula (Int-I) 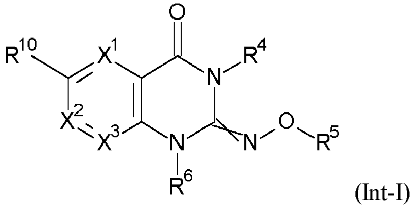 or a salt thereof, wherein
R¹⁰ is hydrogen, halogen (e.g. chloro, bromo, iodo), -B(OH)₂, -B(-O-C(CH₃)₂-C(CH₃)₂-O-), - S(O)₂OH, -S(O)₂Cl or -S-CH₂-phenyl; and
X¹, X², X³, R⁴, R⁵ and R⁶ are as defined for the compound of formula (I) in any one of claims 1 to 12;
and wherein the compound of (Int-I) is not
2-(Hydroxyamino)-6-iodo-3-phenyl-4(3*H*)-quinazolinone;
6-Iodo-2-(propoxyamino)-3-propyl-4(3*H*)-quinazolinone;
6-Bromo-2-(propoxyamino)-3-propyl-4(3*H*)-quinazolinone;
6-Iodo-2-[(2-methylpropoxy)amino]-3-propyl-4(3*H*)-quinazolinone;
6-Bromo-2-[(2-methylpropoxy)amino]-3-propyl-4(3*H*)-quinazolinone;
2-[[(3,4-Dihydro-6-iodo-4-oxo-3-phenyl-2-quinazolinyl)amino]oxy]acetic acid;
(3,4-Dihydro-6-iodo-4-oxo-3-phenyl-2-quinazolinyl)azanyl acetate;
2,4(*1H*,3*H*)-Quinazolinedione, 6-iodo-3-phenyl-, 2-[*O*-(ethoxycarbonyl)oxime];
(3,4-Dihydro-6-iodo-4-oxo-3-phenyl-2-quinazolinyl)azanyl 2-chloroacetate;
Ethyl 2-[[(3,4-dihydro-6-iodo-4-oxo-3-phenyl-2-quinazolinyl)amino]oxy]acetate;
(3,4-Dihydro-6-iodo-4-oxo-3-phenyl-2-quinazolinyl)azanyl benzoate;
(3,4-Dihydro-6-iodo-4-oxo-3-phenyl-2-quinazolinyl)azanyl benzeneacetate;
1-[(3,4-Dihydro-6-iodo-4-oxo-3-phenyl-2-quinazolinyl)azanyl] 2-ethyl ethanedioate; or
a compound of formula (Int-II) 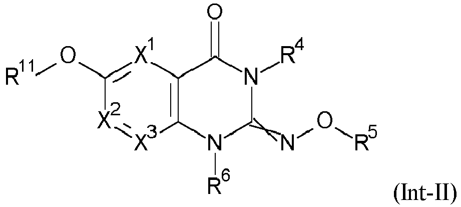 or a salt thereof, wherein
R¹¹ is hydrogen or C₁₋₈ alkyl; and
X¹, X², X³, R⁴, R⁵ and R⁶ are as defined for the compound of formula (I) in any one of claims 1 to 12.
